# EUROPEAN PATENT APPLICATION

(11) **EP 1 136 071 A2**
(43) Date of publication of application: **26.09.2001**
(21) Application number: 01301979.9
(22) Date of filing: 05.03.2001
(51) Int. Cl.: A61K 31/00, A61K 31/404, A61K 31/4439, A61K 31/496, A61K 31/422, A61K 31/5377, A61K 31/427, A61K 31/454, A61K 31/407, A61K 45/06, A61P 3/10

(54) **Use of glycogen phosphorylase inhibitors**

(30) Priority: 22.03.2000 US 191381 P
(71) Applicant: Pfizer Products Inc., Groton, CT 06340-5146 (US)
(72) Inventor: Treadway, Judith Lee, Groton, Connecticut 06340 (US)
(74) Representative: Hayles, James Richard

(57) **Abstract**

Individuals in whom Type 2 diabetes mellitus has not yet presented, but in whom there is an increased risk of developing such condition, can be treated prophylactically with a glycogen phosphorylase inhibitor; a glycogen phosphorylase inhibitor and a non-glycogen phosphorylase inhibiting anti-diabetic agent; or a glycogen phosphorylase inhibitor and an anti-obesity agent.

## Description

### Field of the Invention

The invention relates to methods of utilizing glycogen phosphorylase inhibitors in the prophylactic treatment of individuals who have not yet presented with Type 2 diabetes mellitus, but in whom there is an increased risk of developing such condition.

### Background of the Invention

The diabetic disease state is characterized by an impaired glucose metabolism that manifests itself in, *inter alia*, elevated blood glucose levels in patients suffering therefrom. Generally, diabetes is classified into two distinct subgroups:
(1) Type 1 diabetes, or insulin-demanding diabetes mellitus (IDDM), which arises when patients lack β-cells producing insulin in their pancreatic glands, and
(2) Type 2 diabetes, or non-insulin dependent diabetes mellitus (NIDDM), which occurs in patients with, *inter alia,* impaired β-cell function.

At present, Type 1 diabetic patients are treated with insulin, while the majority of Type 2 diabetic patients are treated with hypoglycemic agents, such as sulfonylureas that stimulate β-cell function, with other agents that enhance the tissue sensitivity of the patients towards insulin, or with insulin itself. Although hypoglycemic agents such as sulfonylureas have been employed widely in the treatment of NIDDM, this treatment is, in many instances, not completely satisfactory. In a large number of NIDDM patients, sulfonylureas have proven ineffective in normalizing blood sugar levels of patients, thereby leading to an increased risk of acquiring diabetic complications. Also, many patients gradually lose the ability to respond to treatment with sulfonylureas and are thus gradually forced into insulin treatment. This shift of patients from oral hypoglycemic agent therapy to insulin therapy is usually ascribed to exhaustion of the β-cells in NIDDM patients.

Type 2 diabetes is a heterogeneous disorder which appears to be polygenic in nature. The primary defect that leads to the clinically diagnosed state of Type 2 diabetes is not clearly identified at this time. It is suspected to be due to a defect in one or more of three primary loci - the liver, the beta cell (pancreatic islets), and/or peripheral insulin-responsive tissues (muscle and fat). There is great scientific debate about the primary importance in one loci over others in the etiology and progression of the disease from the non-diseased state, and at least one reference suggests that the primary defect could occur in liver, causing elevated hepatic glucose production, which in turn stimulates hyperinsulinemia by the beta-cell and peripheral insulin resistance (Am. J. Physiol., 264 (27), E18-E23, 1993).

This progression could then account for a primary defect in hepatic glucose production to produce secondary pancreatic and systemic dysfunction, or coupled with existing subthreshold defects at any of the three loci, to lead from a non-diseased state, to a state of insulin resistance, and/or impaired glucose tolerance without full presentation of Type 2 diabetes. This is called an insulin-resistant state, a Syndrome X state, or a metabolic syndrome state, or a prediabetic state, including, but not limited to, polycyctic ovary syndrome, pregnancy, growth hormone disorders, androgen disorders, and the like. Any of the above conditions could progress to worsen glycemic control to the extent that clinical presentation of Type 2 diabetes could result. For this reason, it is hypothesized here that treatment of individuals "at risk" for the onset or progression of phenotype to the state of Type 2 diabetes, before reaching the state at which Type 2 diabetes clinically presents, with a glycogen phosphorylase inhibitor to reduce hepatic glucose production and elicit the plethora of effects (both liver and otherwise) to provide efficacy and hence maintain glycemic control and insulin sensitivity, and hence prevent or slow the onset or progression to a clinically diagnosed Type 2 diabetic state, would be useful. Therefore, it is stated also here that glycogen phosphorylase inhibitors would be useful for reducing hepatic glucose production and/or insulin resistance in patients in whom impaired glucose tolerance or Type 2 diabetes has not presented but for whom are at increased risk of developing this disease, and/or preventing the disease in patients (people) "at risk" for Type 2 diabetes.

In both normal and diabetic individuals, the liver produces glucose in order to avoid hypoglycemia. This production of glucose is derived either from the release of glucose from stored glycogen or from gluconeogenesis, a *de novo* intracellular synthesis of glucose from a gluconeogenesis precursor, a process mediated by the enzyme glucose-6-phosphatase. In Type 2 diabetics, however, the regulation of hepatic glucose output is poorly controlled and/or increased, in some cases resulting in a doubling of glucose output following overnight fasting. Moreover, in these patients, there exists a strong correlation between the increased fasting plasma glucose levels and the rate of hepatic glucose production. See, for example, R. A. DeFronzo, Diabetes, 37, 667-687 (1988) and J. E. Gerich, Horm. Metab. Res., 26 18-21 (1992). Similarly, hepatic glucose production will be increased in Type 1 diabetes if the disease is not properly controlled by insulin treatment.

Since many existing forms of diabetes therapy have proven ineffective in achieving completely satisfactory glycemic control, there continues to be a great demand for novel therapeutic approaches. As the diabetic liver is known to have an abnormally augmented rate of glucose production, compounds targeting this abnormal activity are highly desirable. Recently, agents functioning as inhibitors of the hepatic enzyme glucose-6-phosphatase have been disclosed in, *inter alia,* International Application Publication WO 97/31901, and in commonly-assigned International Application Publication Nos. WO 96/39384 and WO 96/39385. The disclosures of the foregoing are all incorporated herein by reference. As such, gycogen phosphorylase inhibitors are known to be useful in the treatment of NIDDM by decreasing hepatic glucose production and lowering hypoglycemia. See T.L. Blundell, et al.; Diabetologia, 35, Suppl. 2, 569-576 (1992) and Martin, et al.; Biochemistry, 30, 10101 (1991).

Regarding the use of anti-diabetic agents for the prophylactic treatment of certain at-risk individuals, U.S. Pat. No. 5,874,454 discloses the use of certain thiazolidinedione derivatives in treating populations at risk for developing NIDDM and complications arising therefrom.

The methods of instant invention are directed to the use of glycogen phosphorylase inhibitors in treating prophylactically individuals in whom Type 2 diabetes mellitus has not yet presented, but in whom there is an increased risk of developing such condition.

### Summary of the Invention

The invention provides methods of treating prophylactically an individual in whom Type 2 diabetes mellitus has not yet presented, but in whom there is an increased risk of developing such condition, which methods comprise administering to an individual in need thereof an effective amount of a glycogen phosphorylase inhibitor.

The glycogen phosphorylase inhibitor, as employed according to the methods of the invention, preferably comprises a compound selected from the group consisting of:
(i) a compound of formula (I) 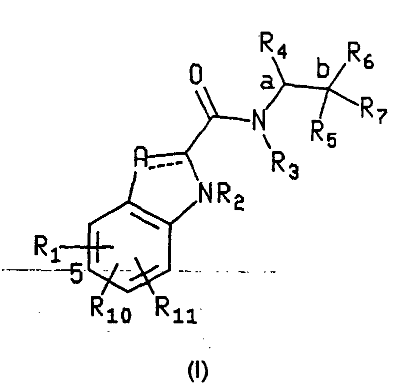 the stereoisomers and prodrugs thereof, and the pharmaceutically acceptable salts of the compounds, stereoisomers, and prodrugs, wherein A, R¹, R², R³, R⁴, R⁵, R⁶, R⁷, R¹⁰, and R¹¹, within the context of formula (I), are as defined hereinbelow;
(ii) a compound of formula (II) 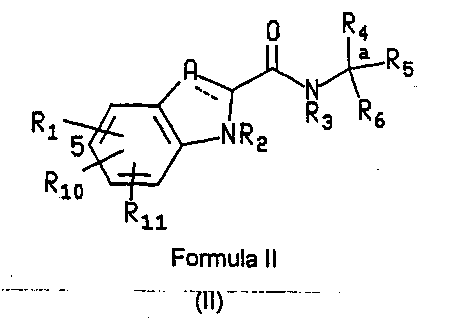 the stereoisomers and prodrugs thereof, and the pharmaceutically acceptable salts of the compounds, stereoisomers, and prodrugs, wherein A, R¹, R², R³, R⁴, R⁵, R⁶, R⁷, R¹⁰, and R¹¹, within the context of formula (II), are as defined hereinbelow;
(iii) a compound of formula (III) 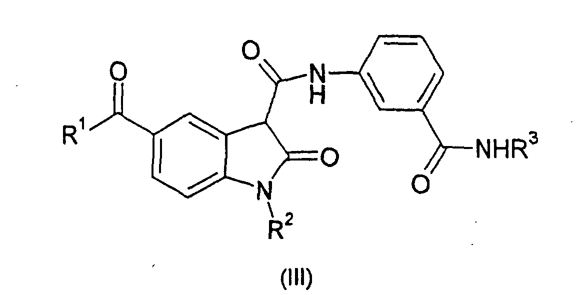 the stereoisomers and prodrugs thereof, and the pharmaceutically acceptable salts of the compounds, stereoisomers, and prodrugs wherein R¹, R², and R³, within the context of formula (III), are as defined hereinbelow; and
(iv) a compound of formula (IV) 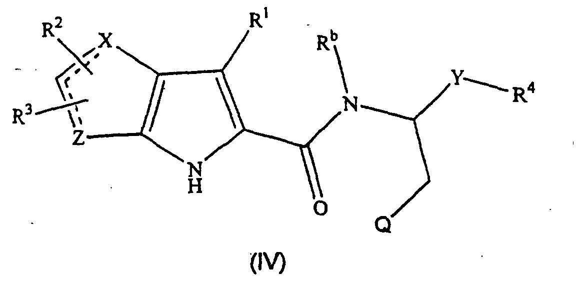 the stereoisomers and prodrugs thereof, and the pharmaceutically acceptable salts of the compounds, stereoisomers, and prodrugs, wherein Q, X, Y, Z, R⁰, R¹, R², and R³ and R⁴, within the context of formula (IV), are as defined hereinbelow.

The invention further provides methods of treating prophylactically an individual in whom Type 2 diabetes mellitus has not yet presented, but in whom there is an increased risk of developing such condition, which methods comprise administering to an individual in need thereof effective amounts of a glycogen phosphorylase inhibitor and a non-glycogen phosphorylase inhibiting anti-diabetic agent, or a glycogen phosphorylase inhibitor and an anti-obesity agent, preferably in the form of a pharmaceutical composition.

### Detailed Description of the Invention

The present invention provides methods of treating prophylactically an individual in whom Type 2 diabetes mellitus has not yet presented, but in whom there is an increased risk of developing such condition, which methods comprise administering to an individual in need thereof an effective amount of a glycogen phosphorylase inhibitor.

The invention further provides methods of treating prophylactically an individual in whom Type 2 diabetes mellitus has not yet presented, but in whom there is an increased risk of developing such condition, which methods comprise administering to an individual in need thereof effective amounts of a glycogen phosphorylase inhibitor and a non-glycogen phosphorylase inhibiting anti-diabetic agent, or a glycogen phosphorylase inhibitor and an anti-obesity agent, preferably in the form of a pharmaceutical composition.

Any individual representing a population having an increased risk of presenting with Type 2 diabetes mellitus may be prophylactically treated according to the methods of the instant invention. Accordingly, the methods of the invention are useful for preventing the transition to Type 2 diabetes mellitus of anydisease state or condition associated with risk factors having the potential to cause or induce such transition. Examples of such risk factors may include, but are not limited to:
(i) risk factors associated with classification as an individual having insulin resistance and/or hyperinsulinemia;
(ii) risk factors based on an environmental or genetic Type 2 diabetes pre-disposing disease state or condition such as a family history of diabetes, especially in parents or siblings;
(iii) risk factors predicated on race and/or ethnicity, especially individual membership in a population comprising African-Americans, Hispanics, Native Americans, Asians, Pacific Islanders, and the like;
(iv) risk factors based on genetic mutations affecting β-cell function including defects on chromosome 12, gene HNF-1α (MODY3); defects on chromosome 7, gene glucokinase (MODY2); defects on chromosome 20, gene HNF-4α (MODY1); defects in mitochondrial DNA, and the like;
(v) risk factors based on genetic defects in insulin action including genetic mutations leading to Type A insulin resistance, acanthosis nigricans, leprechaunism, Rabson-Mendenhall syndrome, lipoatrophic diabetes or condition, or otherwise having a genetic mutation or mutations in the insulin receptor, IRS proteins, glucose transporters, PC-1, glucokinase, UCP-1, β3 adrenergic receptor gene, and the like;
(vi) risk factors based on presence of excess adipose tissue or clinically diagnosed obesity (i.e. ≥ 20% excess of normal body weight, or BMI ≥ 27 kg/m²), especially central obesity;
(vii) risk factors identified through clinical chemistries or diagnostic testing signifying a pre-diabetic state including impaired glucose tolerance (currently defined as impaired glucose response 2 hours following oral glucose load, i.e. ≥ 140 mg/dl, but <200 mg/dl, with normal glucose fasting value), impaired fasting glucose (currently defined as fasting plasma glucose (FPG) ≥ 110 mg/dl, but < 126 mg/dl), or otherwise described as having hyperglycemia relative to normoglycemia;
(viii) risk factors related to physiologic and endocrine changes associated with growth, development, or aging such as classification as a menopausal, pubescent, or aged individual, especially an individual ≥ 45 years of age;
(ix) risk factors related to diet or eating behaviors, including consumption of high fat or high carbohydrate diets, experiencing prolonged fasting or starvation, or risk factors associated with eating disorders, including having anorexia nervosa or bulemia, and the like;
(x) risk factors based on abnormal cardiovascular or blood lipid parameters, such as hypertension, i.e. blood pressure ≥ 140/90 mmHg in adults, HDL cholesterol levels ≤ 35 mg/dl and/or TG levels ≥ 250 mg/dl, or classification as having metabolic syndrome, i.e. Syndrome X;
(xi) risk factors based on reproductive status, such as pregnancy, or a history of gestational diabetes or macrosomia, i.e. the delivery of offspring having a birthweight of >9 lbs.;
(xii) risk factors attributable to muscle wasting due to aging, starvation, exposure to anti-gravity environments, paralysis resulting from spinal cord injury, and the like;
(xiii) risk factors associated with polycystic ovary syndrome;
(xiv) risk factors due to organ disease or dysfunction including liver cirrhosis, or renal disease;
(xv) risk factors due to conditions resulting in metabolic disturbances;
(xvi) risk factors due to endocrine disorders or endocrinopathies, such as hyperandrogenism, thyrotoxicosis, hyperthyroidism, insulinoma, glucagonoma, somatostatinoma, aldosteroma, Cushing's Syndrome, pheochromocytoma, acromegaly, hypercortisolemia, and the like;
(xvii) risk factors due to pathophysiologic states including infection (especially congenital rubella, cytomegalovirus, and the like), toxemia, uremia, sepsis, or trauma;
(xviii) risk factors due to immune-mediated disease such as "stiff man" syndrome, production of anti-insulin receptor antibodies, and the like;
(xix) risk factors due to drug or chemical exposure, including being treated with insulin-resistance-inducing or hyperglycemia-inducing agents including, for example, glucocorticoids, cytokines, α-interferon, thyroid hormone, TNFα, thiazides, estrogen-containing products, β-blockers, nicotinic acid, olanzapine and other serotonin receptor-targeted antipsychotics or antidepressants, vacor, diazoxide, dilantin, HIV protease inhibitors, and the like;
(xx) risk factors associated with having a genetic syndrome associated with diabetes including Down's Syndrome, Klinefelter's Syndrome, Wolfram's Syndrome, Freidreich's Syndrome, Huntington's chorea, Laurence-Moon-Biedl Syndrome, myotonic dystrophy, porphyria, Prader-Willi Syndrome, Alzheimer's Disease, and the like; and
(xxi) risk factors associated with the long-term detrimental effects caused by the administration of prolonged, elevated doses of insulin and/or the presence of ketoacidosis.

Although any glycogen phosphorylase inhibitor may be employed in accordance with the methods of the instant invention, it is generally preferred that the inhibitor comprise a compound selected from the group consisting of:
(i) a compound of formula (I) 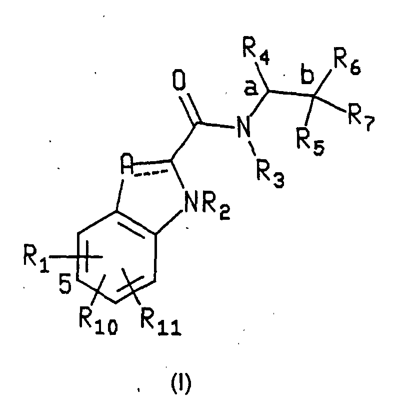 the stereoisomers and prodrugs thereof, and the pharmaceutically acceptable salts of the compounds, stereoisomers, and prodrugs, wherein
   the dotted line (---) is an optional bond;
      A is -C(H)=, -C((C₁-C₄)alkyl)= or -C(halo)= when the dotted line (-) is a bond, or A is methylene or -CH((C₁-C₄)alkyl)- when the dotted line (-) is not a bond;
      R₁, R₁₀ or R₁₁ are each independently H, halo, 4-, 6- or 7-nitro, cyano, (C₁-C₄)alkyl, (C₁-C₄)alkoxy, fluoromethyl, difluoromethyl or trifluoromethyl;
      R₂ is H;
      R₃ is H or (C₁-C₅)alkyl;
      R₄ is H, methyl, ethyl, n-propyl, hydroxy(C₁-C₃)alkyl, (C₁-C₃)alkoxy(C₁-C₃)alkyl, phenyl(C₁-C₄)alkyl, phenylhydroxy(C₁-C₄)alkyl, phenyl(C₁-C₄)alkoxy(C₁-C₄)alkyl, thien-2- or -3-yl(C₁-C₄)alkyl or fur-2- or -3-yl(C₁-C₄)alkyl wherein said R₄ rings are mono-, di- or tri-substituted independently on carbon with H, halo, (C₁-C₄)alkyl, (C₁-C₄)alkoxy, trifluoromethyl, hydroxy, amino or cyano; or
      R₄ is pyrid-2-, -3- or -4-yl(C₁-C₄)alkyl, thiazol-2-, -4- or -5-yl(C₁-C₄)alkyl, imidazol -1-,-2-, -4- or -5-yl(C₁-C₄)alkyl, pyrrol-2- or -3-yl(C₁-C₄)alkyl, oxazol-2-, -4- or - 5-yl-(C₁-C₄)alkyl, pyrazol-3-, -4- or -5-yl(C₁-C₄)alkyl, isoxazol-3-, -4- or -5-yl(C₁-C₄)alkyl, isothiazol-3-, -4- or -5-yl(C₁-C₄)alkyl, pyridazin-3- or -4-yl-(C₁-C₄)alkyl, pyrimidin-2-, -4-, -5- or -6-yl(C₁-C₄)alkyl, pyrazin-2- or -3-yl(C₁-C₄)alkyl or 1,3,5-triazin-2-yl(C₁-C₄)alkyl, wherein said preceding R₄ heterocycles are optionally mono- or disubstituted independently with halo, trifluoromethyl, (C₁-C₄)alkyl, (C₁-C₄)alkoxy, amino or hydroxy and said mono-or di-substituents are bonded to carbon;
      R₅ is H, hydroxy, fluoro, (C₁-C₅)alkyl, (C₁-C₅)alkoxy, (C₁-C₆)alkanoyl, amino(C₁-C₄)alkoxy, mono-N- or di-N,N-(C₁-C₄)alkylamino(C₁-C₄)alkoxy, carboxy(C₁-C₄)alkoxy, (C₁-C₅)alkoxy-carbonyl(C₁-C₄)alkoxy, benzyloxycarbonyl(C₁-C₄)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding R₅ rings are optionally mono-substituted with halo, (C₁-C₄)alkyl, (C₁-C₄)alkoxy, hydroxy, amino or trifluoromethyl and said mono-substituents are bonded to carbon;
      R₇ is H, fluoro or (C₁-C₅)alkyl; or
      R₅ and R₇ can be taken together to be oxo;
      R₆ is carboxy, (C₁-C₈)alkoxycarbonyl, C(O)NR₈R₉ or C(O)R₁₂,
         wherein
      R₈ is H, (C₁-C₃)alkyl, hydroxy or (C₁-C₃)alkoxy; and
      R₉ is H, (C₁-C₈)alkyl, hydroxy, (C₁-C₈)alkoxy, methylene-perfluorinated(C₁-C₈)alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding R₉ rings are carbon-nitrogen linked; or
      R₉ is mono-, di- or tri-substituted (C₁-C₅)alkyl, wherein said substituents are independently H, hydroxy, amino, mono-N- or di-N,N-(C₁-C₅)alkylamino; or
      R₉ is mono- or di-substituted (C₁-C₅)alkyl, wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl or 1,3,5-triazinyl
      wherein the nonaromatic nitrogen-containing R₉ rings are optionally mono-substituted on nitrogen with (C₁-C₆)alkyl, benzyl, benzoyl or (C₁-C₆)alkoxycarbonyl and wherein the R₉ rings are optionally mono-substituted on carbon with halo, (C₁-C₄)alkyl, (C₁-C₄)alkoxy, hydroxy, amino, or mono-N- and di-N,N (C₁-C₅)alkylamino provided that no quatemized nitrogen is included and there are no nitrogen-oxygen, nitrogennitrogen or nitrogen-halo bonds;
      R₁₂ is piperazin-1-yl, 4-(C₁-C₄)alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxo-thiazolidin-3-yl, 1,1-dioxo-thiazolidin-3-yl, 2-(C₁-C₆)alkoxycarbonylpyrrolidin-1-yl, oxazolidin-3-yl or 2(R)-hydroxymethylpyrrolidin-1-yl; or
      R₁₂ is 3- and/or 4-mono-or di-substituted oxazetidin-2-yl, 2-, 4-, and/or 5- mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di- substituted 1,1-dioxothiazolidin-3-yl, 3- and/or 4-, mono- or disubstituted pyrrolidin-1-yl, 3-, 4- and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4- and/or 5-, mono- and/or di-substituted isothiazolidin-2-yl wherein said R₁₂ substituents are independently H, halo, (C₁-C₅)-alkyl, hydroxy, amino, mono-N- or di-N,N-(C₁-C₅)alkylamino, formyl, oxo, hydroxyimino, (C₁-C₅)alkoxy, carboxy, carbamoyl, mono- N-or di-N,N-(C₁-C₄)alkylcarbamoyl, (C₁-C₄)alkoxyimino, (C₁-C₄)alkoxymethoxy, (C₁-C₆)alkoxycarbonyl, carboxy(C₁-C₅)alkyl or hydroxy(C₁-C₅)alkyl;
(ii) a compound of formula (II) 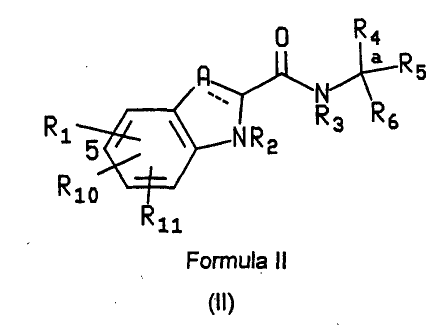 the stereoisomers and prodrugs thereof, and the pharmaceutically acceptable salts of the compounds, stereoisomers, and prodrugs,
   wherein
   the dotted line (---) is an optional bond;
      A is -C(H)=, -C((C₁-C₄)alkyl)=, -C(halo)= or -N=, when the dotted line (-) is a bond, or A is methylene or -CH((C₁-C₄)alkyl)-, when the dotted line (-) is not a bond;
      R₁, R₁₀ or R₁₁ are each independently H, halo, cyano, 4-, 6-, or 7-nitro, (C₁-C₄)alkyl, (C₁-C₄)alkoxy, fluoromethyl, difluoromethyl or trifluoromethyl;
      R₂ is H;
      R₃ is H or (C₁-C₅)alkyl;
      R₄ is H, methyl, ethyl, n-propyl, hydroxy(C₁-C₃)alkyl, (C₁-C₃)alkoxy(C₁-C₃)alkyl, phenyl(C₁-C₄)alkyl, phenylhydroxy(C₁-C₄)alkyl, (phenyl)((C₁-C₄)-alkoxy)(C₁-C₄)alkyl, thien-2- or -3-yl(C₁-C₄)alkyl or fur-2- or -3-yl(C₁-C₄)alkyl wherein said R₄ rings are mono-, di- or tri-substituted independently on carbon with H, halo, (C₁-C₄)alkyl, (C₁-C₄)alkoxy, trifluoromethyl, hydroxy, amino, cyano or 4,5-dihydro-1H-imidazol-2-yl; or
      R₄ is pyrid-2-, -3- or -4-yl(C₁-C₄)alkyl, thiazol-2-, -4- or -5-yl(C₁-C₄)alkyl, imidazol-2-, -4- or -5-yl(C₁-C₄)alkyl, pyrrol-2- or -3-yl(C₁-C₄)alkyl, oxazol-2-, -4- or -5- yl(C₁-C₄)alkyl, pyrazol-3-, -4- or -5-yl(C₁-C₄)alkyl, isoxazol-3-, -4- or -5-yl(C₁-C₄)alkyl, isothiazol-3-, -4- or -5-yl(C₁-C₄)alkyl, pyridazin-3- or -4-yl(C₁-C₄)alkyl, pyrimidin-2-, -4-, -5- or -6-yl(C₁-C₄)alkyl, pyrazin-2- or -3-yl(C₁-C₄)alkyl, 1,3,5-triazin-2-yl(C₁-C₄)alkyl or indol-2-(C₁-C₄)alkyl, wherein said preceding R₄ heterocycles are optionally mono- or di-substituted independently with halo, trifluoromethyl, (C₁-C₄)alkyl, (C₁-C₄)alkoxy, amino, hydroxy or cyano and said substituents are bonded to carbon; or
      R₄ is R₁₅-carbonyloxymethyl, wherein said R₁₅ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding R₁₅ rings are optionally mono- or di-substituted independently with halo, amino, hydroxy, (C₁-C₄)alkyl, (C₁-C₄)alkoxy or trifluoromethyl and said mono- or di-substituents are bonded to carbon;
      R₅ is H, methyl, ethyl, n-propyl, hydroxymethyl or hydroxyethyl
      R₆ is carboxy, (C₁-C₈)alkoxycarbonyl, benzyloxycarbonyl, C(O)NR₈R₉ or C(O)R₁₂
         wherein
      R₈ is H, (C₁-C₆)alkyl, cyclo(C₃-C₆)alkyl, cyclo(C₃-C₆)alkyl(C₁-C₅)alkyl, hydroxy or (C₁-C₈)alkoxy; and
      R₉ is H, cyclo(C₃-C₈)alkyl, cyclo(C₃-C₈)alkyl(C₁-C₅)alkyl, cyclo(C₄-C₇)alkenyl, cyclo(C₃-C₇)alkyl(C₁-C₅)alkoxy, cyclo(C₃-C₇)alkyloxy, hydroxy, methylene-perfluorinated(C₁-C₈)alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or
      R₉ is (C₁-C₆)alkyl or (C₁-C₈)alkoxy wherein said (C₁-C₆)alkyl or (C₁-C₈)alkoxy is optionally monosubstituted with cyclo(C₄-C₇)alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, thiomorpholinyl, 1-oxothiomorpholinyl, 1,1-dioxothiomorpholinyl, pyridazinyl, pyrimidinyl, pyrazinyl, piperazinyl, 1,3,5-triazinyl or indolyl and wherein said (C₁-C₆)alkyl or (C₁-C₈)alkoxy are optionally additionally independently mono- or di-substituted with halo, hydroxy, (C₁-C₅)alkoxy, amino, mono-N- or di-N,N-(C₁-C₅)alkylamino, cyano, carboxy, or (C₁-C₄)alkoxycarbonyl; and
      wherein the R₉ rings are optionally mono- or di-substituted independently on carbon with halo, (C₁-C₄)alkyl, (C₁-C₄)alkoxy, hydroxy, hydroxy(C₁-C₄)alkyl, amino(C₁-C₄)alkyl, mono-N- or di-N,N-(C₁-C₄)alkylamino(C₁-C₄)alkyl, (C₁-C₄)alkoxy(C₁-C₄)alkyl, amino, mono-N- or di-N,N-(C₁-C₄)alkylamino, cyano, carboxy, (C₁-C₅)alkoxycarbonyl, carbamoyl, formyl or trifluoromethyl and said R₉ rings may optionally be additionally mono- or di-substituted independently with (C₁-C₅)alkyl or halo;
      R₁₂ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydrobenzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,
      wherein said R₁₂ rings are optionally mono-, di- or tri-substituted independently with halo, (C₁-C₅)alkyl, (C₁-C₅)alkoxy, hydroxy, amino, mono-N- or di-N,N-(C₁-C₅)alkylamino, formyl, carboxy, carbamoyl, mono-N- or di-N,N-(C₁-C₅)alkylcarbamoyl, (C₁-C₆)alkoxy(C₁-C₃)alkoxy, (C₁-C₅)alkoxycarbonyl, benzyloxycarbonyl, (C₁-C₅)alkoxycarbonyl(C₁-C₅)alkyl, (C₁-C₄)alkoxycarbonylamino, carboxy(C₁-C₅)alkyl, carbamoyl(C₁-C₅)alkyl, mono-N- or di-N,N-(C₁-C₅)alkylcarbamoyl(C₁-C₅)alkyl, hydroxy(C₁-C₅)alkyl, (C₁-C₄)alkoxy(C₁-C₄)alkyl, amino(C₁-C₄)alkyl, mono-N- or di-N,N-(C₁-C₄)alkylamino(C₁-C₄)alkyl, oxo, hydroxyimino or (C₁-C₆)alkoxyimino and wherein no more than two substituents are selected from oxo, hydroxyimino or (C₁-C₆)alkoxyimino and oxo, hydroxyimino or (C₁-C₆)alkoxyimino are on nonaromatic carbon; and
      wherein said R₁₂ rings are optionally additionally mono- or di-substituted independently with (C₁-C₅)alkyl or halo;
(iii) a compound of formula (III) 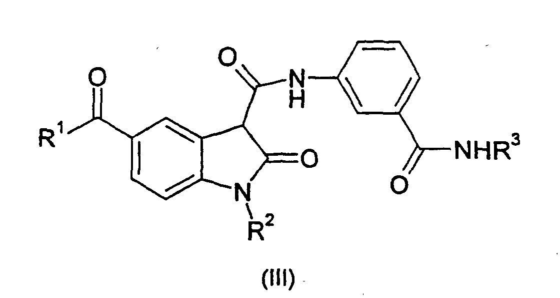 the stereoisomers and prodrugs thereof, and the pharmaceutically acceptable salts of the compounds, stereoisomers, and prodrugs,
   wherein
   R¹ is (C₁-C₄)alkyl, (C₃-C₇)cycloalkyl, phenyl or phenyl independently substituted with up to three (C₁-C₄)alkyl, (C₁-C₄)alkoxy or halogen;
   R² is (C₁-C₄)alkyl optionally substituted with up to three fluoro atoms; and
   R³ is (C₃-C₇)cycloalkyl; phenyl; phenyl substituted at the para position with (C₁-C₄)alkyl, halo or trifluoromethyl; phenyl substituted at the meta position with fluoro; or phenyl substituted at the ortho position with fluoro; and
(iv) a compound of formula (IV) 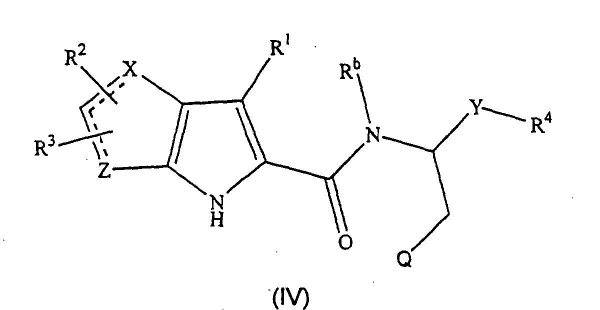 the stereoisomers and prodrugs thereof, and the pharmaceutically acceptable salts of the compounds, stereoisomers, and prodrugs,
   wherein
   Q is aryl, substitued aryl, heteroaryl, or substitued heteroaryl
   each Z and X are independently (C, CH or CH₂), N, O or S;
   X¹ is NR^{a}, -CH₂-, O or S;
   each - - - - is independently a bond or is absent, provided that both - - - - are not simultaneously bonds;
   R¹ is hydrogen, halogen, -OC₁-C₈alkyl, -SC₁₋-C₈alkyl,
   -C₁-C₈alkyl, -CF₃, -NH₂, -NHC₁-C₈alkyl, -N(C₁-C₈alkyl)₂, -NO₂, -CN,
   -CO₂H, -CO₂C₁-C₈alkyl, -C₂-C₈alkenyl, or -C₂-C₈alkynyl;
   each R^{a} and R^{b} is independently hydrogen or -C₁-C₈alkyl;
   Y is 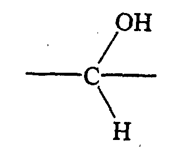 or absent;
   R² and R³ are independently hydrogen, halogen, -C₁-C₈alkyl, -CN, -C≡C-Si(CH₃)₃, -OC₁-C₈alkyl, -SC₁-C₈alkyl, -CF₃, -NH₂, -NHC₁-C₈alkyl, -N(C₁-C₈alkyl)₂, -NO₂, -CO₂H, -CO₂C₁-C₈alkyl, -C₂-C₈alkenyl, or -C₂-C₈alkynyl, or R² and R³ together with the atoms on the ring to which they are attached form a five or six membered ring containing from 0 to 3 heteroatoms and from 0 to 2 double bonds;
   R⁴ is -C(=O)-A;
   A is -NR^{d}R^{d}, -NR^{a}CH₂CH₂OR^{a}, 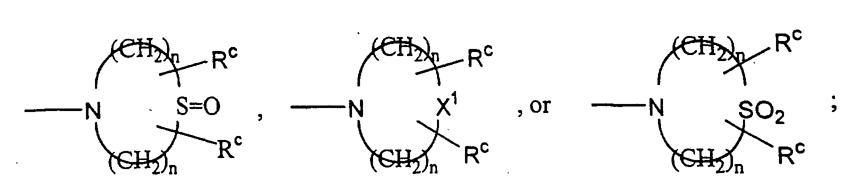
   each R^{d} is independently hydrogen, C₁-C₈alkyl, C₁-C₈alkoxy, aryl, substituted aryl, heteroaryl, or substituted heteroaryl;
   each R^{c} is independently hydrogen, -C(=O)OR^{a}, -CR^{a}, -SR^{a}, or -MR^{a}R^{a;} and
   each n is independently 1-3.

The compounds of formula (I), the stereoisomers and prodrugs thereof, and the pharmaceutically acceptable salts of the compounds, stereoisomers, and prodrugs, may be prepared as described in the aforementioned International Application Publication No. WO 96/39385.

A particularly preferred subgroup of formula (I) compounds are those compounds selected from the group consisting of:
5-chloro-1H-indole-2-carboxylic acid-[(1S)-((R)-hydroxy-dimethylcarbamoylmethyl)-2-phenyl-ethyl]-amide;
5,6-dichloro-1H-indole-2-carboxylic acid-{(1S)-[(R)-hydroxy-(methoxy-methylcarbamoyl)-methyl]-2-phenyl-ethyl}-amide;
5-chloro-1H-indole-2-carboxylic acid-{(1S)-[(R)-hydroxy-(methoxy-methylcarbamoyl)-methyl]-2-phenyl-ethyl}-amide;
5-chloro-1H-indole-2-carboxylic acid-((1S)-{(R)-hydroxy-[(2-hydroxy-ethyl)methyl-carbamoyl]-methy}-2-phenyl-ethyl}-amide;
5-chloro-1H-indole-2-carboxylic acid-((1S)-{(R)-hydroxy-[methyl-(2-pyridin-2-yl-ethyl)-carbamoyl]-methyl}-2-phenyl-ethyl)-amide;
5-chloro-1H-indole-2-carboxylic acid-[(1S)-benzyl-(2R)-hydroxy-3-(4-methylpiperazin-1-yl)-3-oxo-propyl]-amide;
5-chloro-1H-indole-2-carboxylic acid-[(1S)-benzyl-(2R)-hydroxy-3-(3-hydroxyazetidin-1-yl)-3-oxo-propyl]-amide;
5-chloro-1H-indole-2-carboxylic acid-((1S)-benzyl-(2R)-hydroxy-3-isoxazolidin-2-yl-3-oxo-propyl)-amide;
5-chloro-1H-indole-2-carboxylic acid-((1S)-benzyl-(2R)-hydroxy-3-[1,2]oxazinan-2-yl-3-oxo-propyl)-amide;
5-chloro-1H-indole-2-carboxylic acid-[(1S)-benzyl-(2R)-hydroxy-3-((3S)-hyd roxy-pyrrolidin-1-yl)-3-oxo-propyl]-amide;
5-chloro-1H-indole-2-carboxylic acid-[(1S)-benzyl-3-((3S,4S)-dihydroxypyrrolidin-1-yl)-(2R)-hydroxy-3-oxo-propyl]-amide;
5-chloro-1H-indole-2-carboxylic acid-[(1S)-benzyl-3-((3R,4S)-dihydroxypyrrolidin-1-yl)-(2R)-hydroxy-3-oxo-propyl]-amide; and
5-chloro-1H-indole-2-carboxylic acid-((1S)-benzyl-(2R)-hydroxy-3-morpholin-4-yl-3-oxo-propyl)-amide; the stereoisomers and prodrugs thereof, and the pharmaceutically acceptable salts of the compounds, stereoisomers, and prodrugs.

The compounds of formula (II), the stereoisomers and prodrugs thereof, and the pharmaceutically acceptable salts of the compounds, stereoisomers, and prodrugs, may be prepared as described in the aforementioned International Application Publication No. WO 96/39384.

A particularly preferred subgroup of formula (II) compounds are those compounds selected from the group consisting of:
5-chloro-1H-indole-2-carboxylic acid-[(1S)-benzyl-2-(3-hydroxyiminopyrrolidin-1-yl)-2-oxo-ethyl]-amide;
5-chloro-1H-indole-2-carboxylic acid-[2-(cis-3,4-dihydroxy-pyrrolidin-1-yl)-2-oxo-ethyl]-amide;
5-chloro-1H-indole-2-carboxylic acid-[(1S)-benzyl-2-(cis-3,4-dihydroxypyrrolidin-1-yl)-2-oxo-ethyl]-amide;
5-chloro-1H-indole-2-carboxylic acid-[2-(1,1-dioxo-thiazolidin-3-yl)-2-oxoethyl]-amide;
5-chloro-1H-indole-2-carboxylic acid-(2-oxo-2-thiazolidin-3-yl-ethyl)-amide;
5-chloro-1H-indole-2-carboxylic acid-(1S)-(4-fluoro-benzyl)-2-(4-hydroxypiperidin-1-yl)-2-oxo-ethyl]-amide;
5-chloro-1H-indole-2-carboxylic acid-[(1S)-benzyl-2-((3RS)-hydroxy-piperidin-1-yl)-2-oxo-ethyl]-amide;
5-chloro-1H-indole-2-carboxylic acid-2-oxo-2-((1RS)-oxo-1-thiazolidin-3-yl)ethyl]-amide;
5-chloro-1H-indole-2-carboxylic acid-[(1S)-(2-fluoro-benzyl)-2-(4-hydroxypiperidin-1-yl)-2-oxo-ethyl]-amide;
5-chloro-1H-indole-2-carboxylic acid-(1S)-benzyl-2-(3-hydroxy-azetidin-1-yl)2-oxo-ethyl]-amide;
5-chloro-1H-indole-2-carboxylic acid-[(1S)-benzyl-2-(3-hydroxyimino-azetidin-1-yl)-2-oxo-ethyl]-amide; and
5-chloro-1H-indole-2-carboxylic acid-[(1S)-benzyl-2-(4-hydroxyimino-piperidin-1-yl)-2-oxo-ethyl]-amide; the stereoisomers and prodrugs thereof, and the pharmaceutically acceptable salts of the compounds, stereoisomers, and prodrugs.

The compounds of formula (III), the stereoisomers and prodrugs thereof, and the pharmaceutically acceptable salts of the compounds, stereoisomers, and prodrugs, may be prepared according to the following synthetic methodologies. The following definitions are applicable with respect to the compounds of formula (III).

By "halo" is meant chloro, bromo, iodo, or fluoro.

By "alkyl" is meant straight chain or branched saturated hydrocarbon Exemplary of such alkyl groups (assuming the designated length encompasses the particular example) are methyl, ethyl, propyl, isopropyl, butyl, sec-butyl, *tert*-butyl, pentyl, isopentyl, hexyl, isohexyl, and so forth.

By "alkoxy" is meant straight chain or branched saturated alkyl bonded through an oxy. Exemplary of such alkoxy groups (assuming the designated length encompasses the particular example) are methoxy, ethoxy, propoxy, isopropoxy, butoxy, isobutoxy, *tert*-butoxy, pentoxy, isopentoxy, hexoxy and isohexoxy.

The expression "prodrug" refers to compounds that are drug precursors, which, following administration, release the drug *in vivo* via a chemical or physiological process (e.g., a prodrug on being brought to the physiological pH is converted to the desired drug form).

As used herein, the expressions "reaction-inert solvent" and "inert solvent" refers to a solvent or mixture of solvents which does not interact with starting materials, reagents, intermediates or products in a manner which adversely affects the yield of the desired product.

The chemist of ordinary skill will recognize that certain compounds of formula (III) may contain one or more atoms which may be in a particular stereochemical or geometric configuration, giving rise to stereoisomers and configurational isomers. All such isomers and mixtures thereof are included in this invention.

In general, the compounds of formula (III) can be made by processes including those known in the chemical arts, particularly in light of the description contained herein. Certain processes for the manufacture of formula (III) compounds are illustrated hereinbelow in the following reaction schemes.

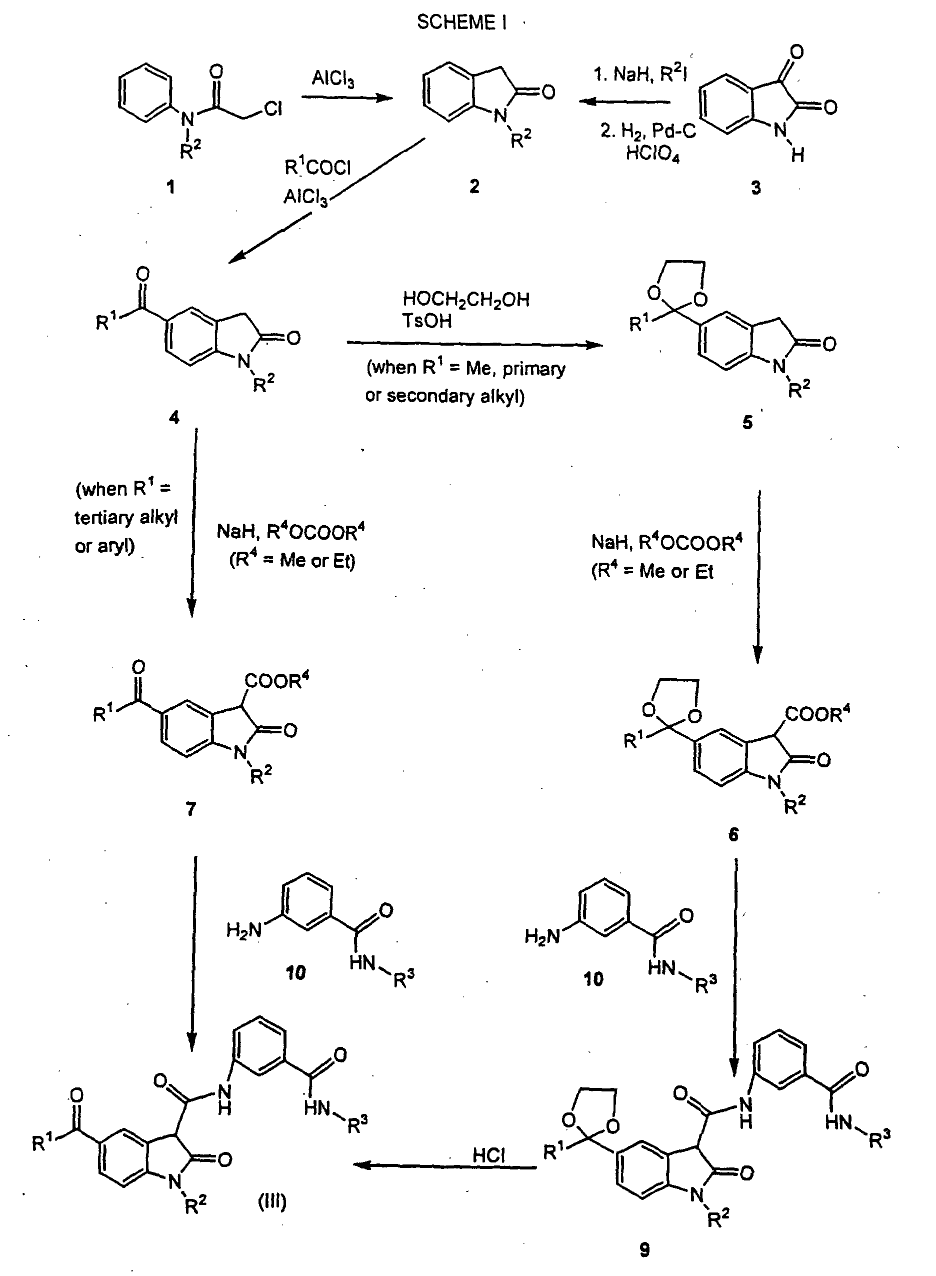

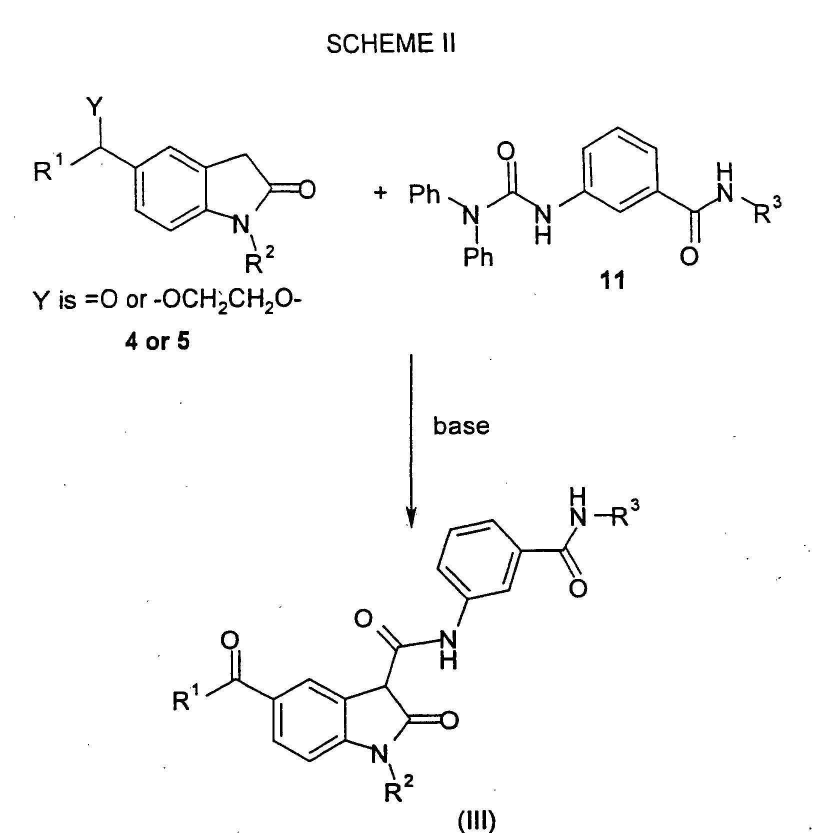

According to Scheme I, the compounds of formula (III), wherein R¹, R² and R³ are as defined hereinabove, may be prepared by either of two general procedures which involve coupling a carboxylic acid or carboxylic acid ester derivative of formula 6 or 7 with an appropriate 3-substituted aniline derivative of formula 10. When the coupling is performed using a compound of formula 7, the immediate product is a compound of formula (III). When the coupling is performed using a compound of formula 6, which compound contains a protected ketone moiety, the intermediate result is the formation of a compound of formula 9 which may then be converted into a compound of formula (III) through subsequent deprotection.

Typically, the deprotection of the formula 9 compounds may be effected using methods well known to one of ordinary skill in the art, for example, the methods described in "Protecting Groups in Organic Synthesis," Second Edition, T.W. Greene and P.G.M. Wuts, John Wiley and Sons, Inc., 1991. Generally, the compound of formula 9 is dissolved in a reaction-inert solvent such as tetrahydrofuran (THF) and strong aqueous acid is added. The temperature of the reaction may be varied from 0°C to 50°C. Generally, however, the reaction is performed at room temperature. The reaction is stirred until all or most of the starting material has reacted as determined by thin layer chromatography or other analytical technique well known to those skilled in the art. Ordinarily, the reaction is stirred for about fifteen minutes to about twenty-four hours, and preferably for about one hour. The resulting compound of formula (III) is then isolated according to methods well known to one of ordinary skill in the art.

The coupling reaction referred to hereinabove is used to generate the compounds of formula (III) directly from the compounds of formula 7, or to generate the compounds of formula 9 from the compounds of formula 6. The coupling reaction is most readily accomplished by reacting a carboxylic acid ester derivative of formula 6 or formula 7 with the appropriate aniline derivative of formula 10. Typically, a compound of formula 6 or formula 7 is dissolved in a reaction-inert solvent and a compound of formula 10 is added. Molecular sieves (4Å) are then added and the reaction mixture is generally heated at the reflux temperature of the chosen solvent until the starting materials are no longer present as determined by thin layer chromatography or other analytical techniques that will be well known to one of ordinary skill in the art. The coupled product of formula 9 or formula (III) is then isolated according to methods well known to those skilled in the art.

Alternatively, the compounds of formula (III) may be prepared according to the procedure set forth in Scheme II. In this procedure, a compound of formula 5, wherein Y is as set forth in Scheme II, is reacted with an activated amide such as a NN-diphenylureido derivative in the presence of a base to form the compound of formula (III). Typically, the compound of formula 5, wherein Y is =O (when R¹ is tertiary alkyl or aryl) or -OCH₂CH₂O- (when R¹ is primary or secondary alkyl) and R¹ and R² are as described hereinabove, is dissolved in a suitable solvent and treated with a base which is strong enough to deprotonate the carbon atom alpha to the carbonyl group. The anion thus formed is treated with the activated amide compound and the reaction mixture is stirred for about 16 hours to about 7 days. Typically, the reaction is complete after stirring for about three days. The reaction mixture is then acidifed to provide the compound of formula (III).

The compounds of formulae 6 and 7 in Scheme I may be prepared by standard acylation chemistry well known to one of ordinary skill in the art. For example, the compounds of formula 4 are acylated directly, when R¹ is tertiary alkyl or aryl, by reacting the compound of formula 4 under standard acylation conditions, e.g.; base and acylating agent, to obtain the compound of formula 6. When R is primary or secondary alkyl, the ketone moiety attached to the 5-position of the oxindole ring must be protected using standard ketone protecting groups as set forth in Greene and Wuts, *supra.* The protected compound of formula 5 is then acylated in the same manner as the compound of formula 4 to obtain the compound of formula 7. The acylation reaction described in this paragraph is readily carried out using procedures well known to those skilled in the art or by using methods analogous to those set forth in U.S. Pat. No. 4,686,224, the teachings of which are incorporated herein by reference. Deprotection, if required, is performed using methods analogous to those described in Greene and Wuts, *supra*.

The compounds of formulae 1, 2, 3, 4 and 10 are prepared according to methods well known to those skilled in the art. Further, the starting materials and reagents for the above described reaction schemes are also readily available from commercial sources or can be readily synthesized by those skilled in the art using conventional methods of organic synthesis.

The compounds of formula (III) may have an asymmetric carbon atom and therefore are racemic mixtures of enantiomers when prepared from nonoptically active intermediates and reagents. Enantiomers can be separated by reacting the enantiomeric mixture with an appropriate optically active compound (e.g,amine) to form a mixture of diastereomeric salts of the compound of formula (III) and separating the diastereomers by crystallization or other method well known to those skilled in the art. It will be recognized by those skilled in the art that racemization of the optically active center may occur upon removal of the ammonium counterion. Therefore, when resolving the compounds of formula (III) using optically active amine compounds, it is particularly advantageous to use pharmaceutically acceptable optically active amines such as naturally occurring amino acids protected as carboxylic acid esters or other pharmaceutically acceptable protected amino acids. Other physical resolution techniques such as chromatography are well known to those skilled in the art and these techiques may also be used to resolve the enantiomers of formula (III). All such diastereomers and enantiomers and mixtures thereof are intended to be included within the scope of the general formula (III).

It will be further recognized that the compounds of formub (III) are acidic and they may form a salt with a pharmaceutically acceptable cation. All such salts are within the scope of the general formula (III) and can be prepared by conventional methods well known to one of ordinary skill in the art. Typical bases used to form such cationic salts are sodium hydroxide, sodium methoxide, sodium ethoxide, sodium hydride, potassium methoxide, magnesium hydroxide, calcium hydroxide, benzathine, choline, diethanolamine, piperazine and tromethamine. For example, the cationic salts can be prepared simply by contacting the acidic and basic entities, usually in a stoichiometric ratio, in either an aqueous, non-aqueous or partially aqueous medium, as deemed appropriate. The salts may then be recovered either by filtration, by precipitation with a non-solvent followed by filtration, by evaporation of the solvent, or, in the case of aqueous solutions, by lyophilization, as deemed appropriate.

In addition, when the compounds of general formula (III) form hydrates or solvates they are also intended to be included within the scope of the invention.

NMR spectra were recorded on a Varian XL-300 (Varian Co., Palo Alto, California) or Bruker AM-300 spectrometer (Bruker Co., Billerica, Massachusetts) at about 23°C at 300 MHz for proton and 75.4 mHz for carbon nuclei. Chemical shifts are expressed in parts per million downfield from trimethylsilane.

Column chromatography was performed with Amicon silica gel (30 uM, 60A pore size) (Amicon D Vision, W.R. Grace & Co., Beverly, Mass.) in glass columns under low nitrogen pressure. Unless otherwise specified, reagents were used as obtained from commercial sources. Dimethylformamide, 2-propanol, tetrahydrofuran, and dichloromethane were used as reaction solvents were the anhydrous grade supplied by Aldrich Chemical Company (Milwaukee, Wisconsin). Microanalyses were performed by Schwarzkopf Microanalytical Laboratory, Woodside, NY. The terms "concentrated" and "coevaporated" refer to removal of solvent at water aspirator pressure on a rotary evaporator with a bath temperature of < 45°C.

### Example 1

### 5-Acetyl-1-ethyl-2-oxo-2,3-dihydro-1H-indole-3-carboxylic acid (3-phenylcarbamoylphenyl)-amide

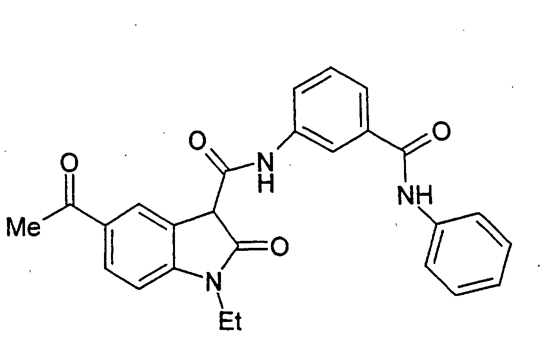

Step A. 1-Ethyl-5-(2-methyl-[1,3]dioxolan-2-yl)-2-oxo-2,3-dihydro-1H-indole-3-carboxylic acid (3-phenylcarbamoyl-phenyl)-amide. 1-Ethyl-5-(2-methyl-[1,3]dioxolan-2-yl)-2-oxo-2,3-dihydro-1H-indole-3-carboxylic acid methyl ester (3.6 g, 11.8 mmol) and 3-amino-N-phenyl-benzamide (5.0 g, 24 mmol) were combined in benzene (160 mL) in a flask fitted with a Soxhlet containing activated 4A molecular sieves. The mixture was heated to reflux for 30 min, then cooled, washed with 0.1 N HCI (2 X 30 mL), water and brine, dried over magnesium sulfate and concentrated in vacuo to a dark orange foam which was purified by flash-chromatography (chloroform/ methanol, 20:1) to give a slightly orange, foamy solid (3.75 g, 66%)

Step B. 5-Acetyl-1-ethyl-2-oxo-2,3-dihydro-1H-indole-3-carboxylic acid (3-phenylcarbamoyl-phenyl)-amide. To a solution of 1-ethyl-5-(2-methyl-[1,3]dioxolan-2-yl)-2-oxo-2,3-dihydro-1H-indole-3-carboxylic acid (3-phenylcarbamoyl-phenyl)-amide (the title compound of Example 1, Step A, 3.75 g, 7.8 mmol) in THF (70 mL) was added 2 N HCI (20 mL). The mixture was stirred at room temperature for 1 hour then poured into water (200 mL) and extracted with ethyl acetate. The extracts were washed with water and brine, dried over magnesium sulfate and concentrated in vacuo to afford an orange foam. The foam was dissolved in a small amount of ethyl acetate and precipitated with hexane to yield a tan solid, which was dried on high vacuum (mp 173-175 °C, 2.5 g, 78%). Calculated for C₂₆H₂₃N₃O₄: C 70.74; H 5.25; N 9.52; found C70.91; H 5.56; N 9.25.

### Examples 2-15

Examples 2 to 15 were prepared from the appropriate starting materials in a manner analogous to the method of Example 1, with variations in reaction time, temperature, and reagents as noted.

### Example 2

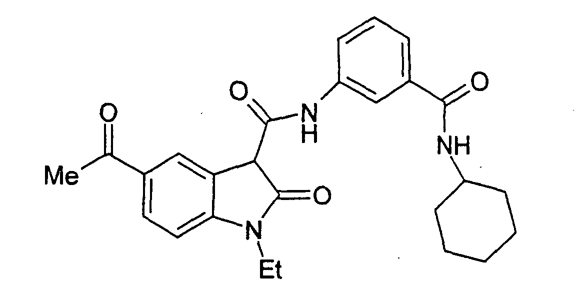

5-Acetyl-1-ethyl-2-oxo-2,3-dihydro-1H-indole-3-carboxylic acid (3-cyclohexylcarbamoyl-phenyl)-amide. Prepared as in Example 1 from 1-ethyl-5-(2-methyl-[1,3]dioxolan-2-yl)-2-oxo-2,3-dihydro-1H-indole-3-carboxylic acid methyl ester and 3-amino-N-cyclohexyl-benzamide. Purified by trituration in ethyl acetate. mp 180°C dec.

### Example 3

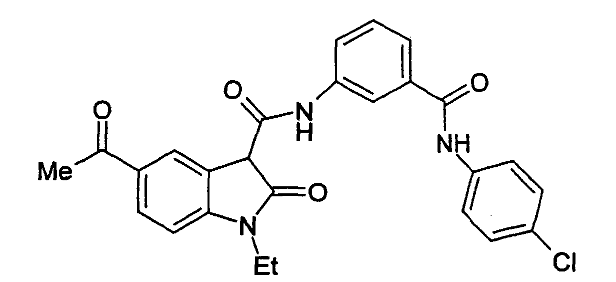

5-Acetyl-1-ethyl-2-oxo-2,3-dihydro-1H-indole-3-carboxylic acid [3-(4-chlorophenylcarbamoyl)-phenyl]-amide. Prepared as in Example 1 from 1-ethyl-5-(2-methyl[1,3]dioxolan-2-yl)-2-oxo-2,3-dihydro-1H-indole-3-carboxylic acid methyl ester and 3-amino-N-(4-chloro-phenyl)-benzamide. Recrystallized from ethyl acetate - hexanes. mp 119-121 °C.

### Example 4

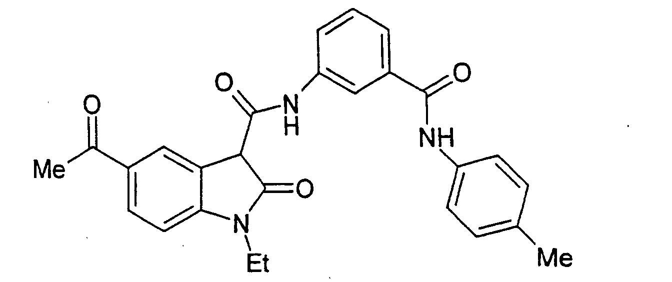

5-Acetyl-1-ethyl-2-oxo-2,3-dihydro-1H-indole-3-carboxylic acid (3-p-tolylcarbamoylphenyl)-amide. Prepared as in Example 1 from 1-ethyl-5-(2-methyl-[1,3]dioxolan-2-yl)-2-oxo-2,3-dihydro-1H-indole-3-carboxylic acid methyl ester and 3-amino-N-(p-tolyl)-benzamide. Purified by trituration in ethyl acetate / hexanes.mp 168 dec.

### Example 5

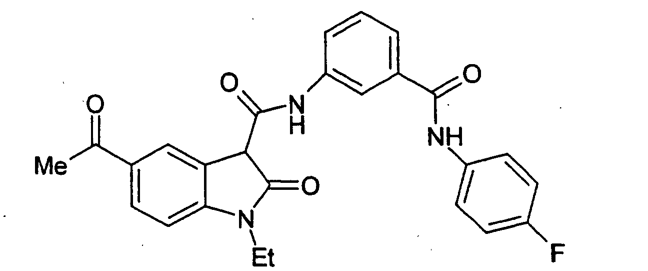

5-Acetyl-1-ethyl-2-oxo-2,3-dihydro-1H-indole-3-carboxylic acid [3-(4-fluorophenylcarbamoyl)-phenyl]-amide. Prepared as in Example 1 from 1-ethyl-5-(2-methyl-[1,3]dioxolan-2-yl)-2-oxo-2,3-dihydro-1H-indole-3-carboxylic acid methyl ester and 3-amino-N-(4-fluoro-phenyl)-benzamide. Recrystallized from ethyl acetate - hexanes - benzene. Calculated for C₂₆H₂₂FN₃O₄: C 67.97; H 4.83; N 9.15; found C 68.34; H 5.31; N 8.80.

### Example 6

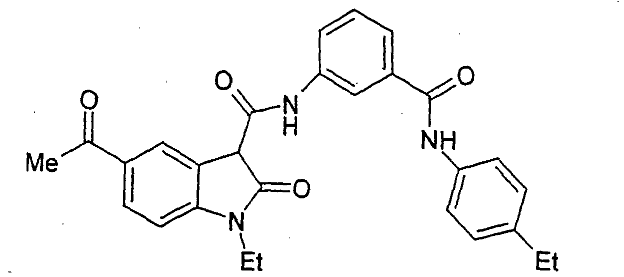

5-Acetyl-1-ethyl-2-oxo-2,3-dihydro-1H-indole-3-carboxylic acid [3-(4-ethylphenylcarbamoyl)-phenyl]-amide. Prepared as in Example 1 from 1-ethyl-5-(2-methyl-[1,3]dioxolan-2-yl)-2-oxo-2,3-dihydro-1H-indole-3-carboxylic acid methyl ester and 3-amino-N-(4-ethyl-phenyl)-benzamide. Purified by chromatography (chloroform / methanol, 10:1). ¹H NMR (DMSO-d6) δ 1.15 (m, 6 H), 2.5 (q, 2 H), 3.85 (q, 2 H), 6.85-8.3 (m, 11 H), 10.0 (s, 1 H), 11.1 (s, 1 H).

### Example 7

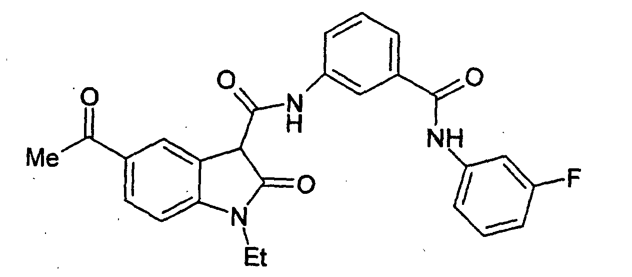

5-Acetyl-1-ethyl-2-oxo-2,3-dihydro-1H-indole-3-carboxylic acid [3-(3-fluorophenylcarbamoyl)-phenyl]-amide. Prepared as in Example 1 from 1-ethyl-5-(2-methyl-[1,3]dioxolan-2-yl)-2-oxo-2,3-dihydro-1H-indole-3-carboxylic acid methyl ester and 3-amino-N-(3-fluoro-phenyl)-benzamide. ¹H NMR (CDCl₃) δ 1.25 (t, 3 H), 2.6 (s, 3 H), 3.85 (q, 2 H), 4.45 (s, 1 H), 6.85-8.05 (m, 11 H), 8.3 (s, 1 H), 9.65 (s, 1 H).

### Example 8

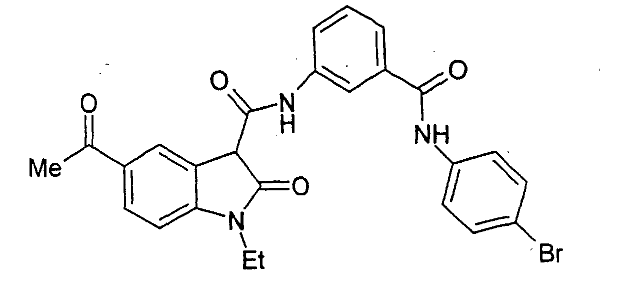

5-Acetyl-1-ethyl-2-oxo-2,3-dihydro-1H-indole-3-carboxylic acid [3-(4-bromophenylcarbamoyl)-phenyl]-amide. Prepared as in Example 1 from 1-ethyl-5-(2-methyl-[1,3]dioxolan-2-yl)-2-oxo-2,3-dihydro-1H-indole-3-carboxylic acid methyl ester and 3-amino-N-(4-bromo-phenyl)-benzamide. mp 117-120°C.

### Example 9

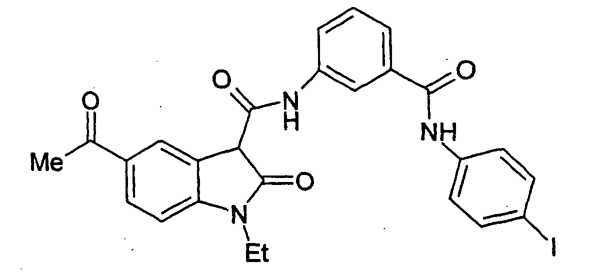

5-Acetyl-1-ethyl-2-oxo-2,3-dihydro-1H-indole-3-carboxylic acid [3-(4-iodophenylcarbamoyl)-phenyl]-amide. Prepared as in Example 1 from 1-ethyl-5-(2-methyl-[1,3]dioxolan-2-yl)-2-oxo-2,3-dihydro-1H-indole-3-carboxylic acid methyl ester and 3-amino-N-(4-iodo-phenyl)-benzamide. mp 205-210 °C. Calculated for C₂₆H₂₂IN₃O₄: C 55.04; H 3.91; N 7.41; found C 55.13; H 4.04; N 7.12.

### Example 10

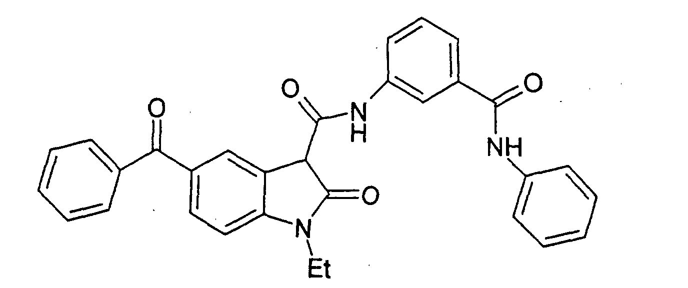

5-Benzoyl-1-ethyl-2-oxo-2,3-dihydro-1H-indole-3-carboxylic acid (3-phenylcarbamoylphenyl)-amide. Prepared as in Example 1, Step A, from 5-benzoyl-1-ethyl-2-oxo-2,3-dihydro-1H-indole-3-carboxylic acid ethyl ester (US 4,686,224) and 3-amino-N-phenyl-benzamide. Purified by trituration in ethyl acetate / hexanes.mp 129-135°C.

### Example 11

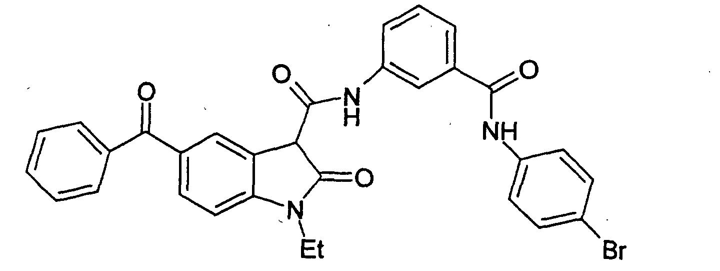

5-Benzoyl-1-ethyl-2-oxo-2,3-dihydro-1H-indole-3-carboxylic acid [3-(4-bromophenylcarbamoyl)-phenyl]-amide. Prepared as in Example 1, Step A, from 5-benzoyl-1-ethyl-2-oxo-2,3-dihydro-1H-indole-3-carboxylic acid ethyl ester (US 4,686,224) and 3-amino-N-(4-bromo-phenyl)-benzamide. Purified by flash-chromatography (hexanes / acetone, 1:1) followed by trituration in ethyl acetate */* hexanes.mp 139-142°C.

### Example 12

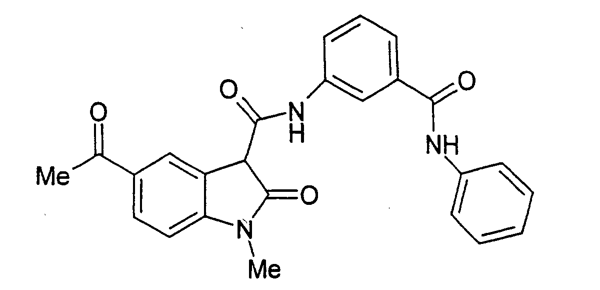

5-Acetyl-1-methyl-2-oxo-2,3-dihydro-1H-indole-3-carboxylic acid (3-phenylcarbamoylphenyl)-amide. Prepared as in Example 1 from 1-methyl-5(2-methyl-[1,3]dioxolan-2-yl)-2-oxo-2,3-dihydro-1H-indole-3-carboxylic acid methyl ester and 3-amino-N-phenyl-benzamide. Purified by trituration in hexanes/ethyl acetate. mp 178-179 °C. Calculated for C₂₅H₂₁N₃O₄: C 70.25; H 4.95; N 9.83; found C 69.89; H 4.79; N 9.69.

### Example 13

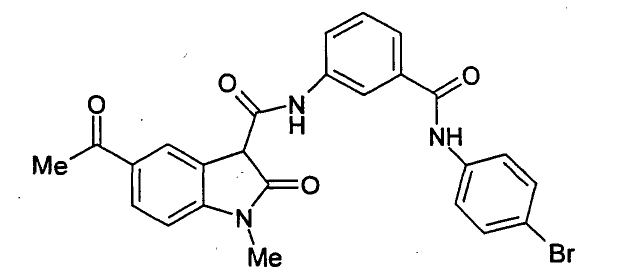

5-Acetyl-1-methyl-2-oxo-2,3-dihydro-1H-indole-3-carboxylic acid [3-(4-bromophenylcarbamoyl)-phenyl]-amide. Prepared as in Example 1 from 1-methyl-5-(2-methyl-[1,3]dioxolan-2-yl)-2-oxo-2,3-dihydro-1H-indole-3-carboxylic acid methyl ester and 3-amino-N-(4-bromo-phenyl)-benzamide. Purified by flash-chromatography (hexanes / acetone, 1:1).
mp 234-236 °C. Calculated for C₂₅H₂₀BrN₃N₃O₄: C 59.30; H 3.98; N 8.30; found C 59.12; H 4.15; N 8.08.

### Example 14

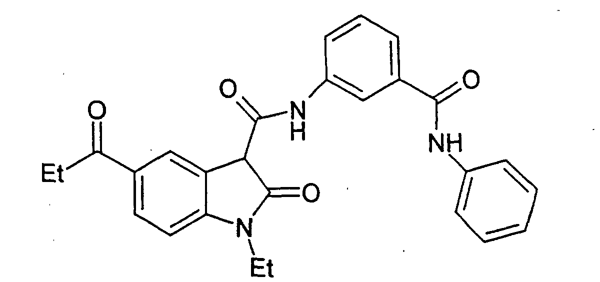

1-Ethyl-2-oxo-5-propionyl-2,3-dihydro-1H-indole-3-carboxylic acid (3-phenylcarbamoyl-phenyl)-amide. Prepared as in Example 1 from 1-ethyl-5-(2-ethyl-[1 ,3]dioxolan-2-yl)-2-oxo-2,3-dihydro-1H-indole-3-carboxylic acid methyl ester and 3-amino-N-phenyl-benzamide. Purified by flash-chromatography (chloroform/methanol, 20:1) followed by trituration in hexanes / ethyl acetate.mp 179-180 °C. Calculated for C₂₇ H₂₅ N₃ O₄: C 71.19; H 5.53; N 9.22; found C 70.79; H 5.73; N 8.79.

### Example 15

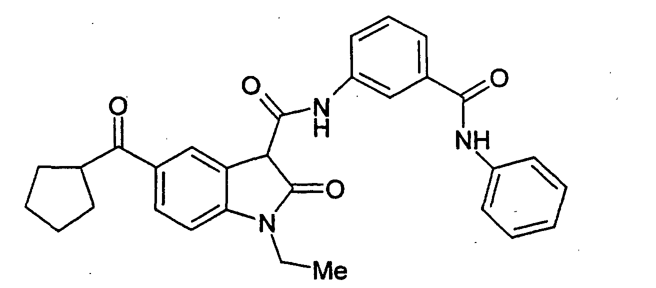

5-Cyclopentanecarbonyl-1-ethyl-2-oxo-2,3-dihydro-1H-indole-3-carboxylic acid (3-phenylcarbamoyl-phenyl)-amide. Prepared as in Example 1 from 5-(2-cyclopentyl-[1,3]dioxolan-2-yl)-1-ethyl-2-oxo-2,3-dihydro-1H-indole-3-carboxylic acid ethyl ester and 3-amino-N-phenyl-benzamide. Purified by flash-chromatography (hexanes / acetone, 1:1) followed by trituration in ethyl acetate.mp 208-213 °C.

### Example 16

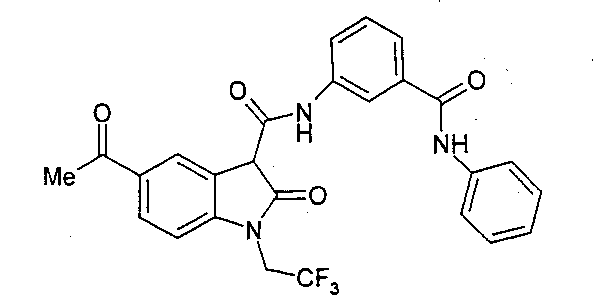

5-Acetyl-2-oxo-1-(2,2,2-trifluoro-ethyl)-2,3-dihydro-1H-indole-3-carboxylic acid (3-phenylcarbamoyl-phenyl)-amide. Sodium hydride (0.13 g of a 60% suspension, 3.3 mmol) was added to a solution of 5-(2-methyl-[1,3]dioxolan-2-yl)-1-(2,2,2-trifluoroethyl)-1,3-dihydro-indol-2-one (0.5 g, 1.66 mmol) in HMPA (6 mL) and after 10 min 3-(3,3-diphenyl-ureido)-N-phenyl-benzamide (0.74 g, 1.8 mmol) was added. The reaction mixture was stirred for three days, acidified with 1 N HCI, poured into water and extracted with ethyl acetate. The combined extracts were washed with water and brine, dried over magnesium sulfate and concentrated in vacuo to a dark foam. Trituration in ethyl acetate / hexanes gave the title compound as a colorless solid (124 mg, 15%, mp 197-203°C). Calculated for C₂₆ H₂₀ F₃ N₃ O₄: C 63.03; H 4.07; N 8.48; found C 62.83; H 4.32; N 8.47.

### Preparation 1

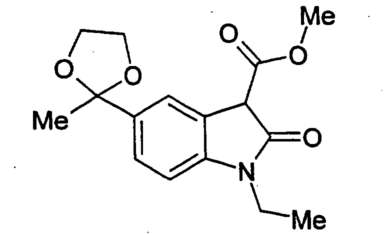

1-Ethyl-5-(2-methyl-[1,3]dioxolan-2-yl)-2-oxo-2,3-dihydro-1H-indole-3-carboxylic acid methyl ester. Sodium (2.68 9, 112 mmol) was added to methanol (110 mL) in a 3-neck flask fitted with a reflux condenser, while controlling the temperature with an icebath. After dissolution, dimethyl carbonate (9.4 mL, 112 mmol) was added followed by 1-ethyl-5-(2-methyl-[1,3]dioxolan-2-yl)-1,3-dihydro-indol-2-one (U.S. Pat. No. 4,686,224, 9.2 g, 37 mmol). The mixture was heated to reflux for 70 hours, then cooled, concentrated to about 25 mL, diluted with water, acidified to pH 7 with acetic acid and extracted twice with ethyl acetate. The combined extracts were washed with brine, dried over magnesium sulfate and concentrated in vacuo to an oily solid which was triturated in isopropyl ether, collected and dried to furnish 7.9 g (70%) of product.

### Preparations 2-4

The compounds of Preparations 2 to 4 were prepared from the appropriate starting materials in a manner analogous to the method of Preparation 1.

### Preparation 2

1-Methyl-5-(2-methyl-[1,3]dioxolan-2-yl)-2-oxo-2,3-dihydro-1H-indole-3-carboxylic acid methyl ester. Prepared from 1-methyl-5-(2-methyl-[1,3]dioxolan-2-yl)-2,3-dihydro-indol-2-one, which was prepared as disclosed in U.S. Pat. No. 4,686,224.

### Preparation 3

1-Ethyl-5-(2-ethyl-[1,3]dioxolan-2-yl)-2-oxo-2,3-dihydro-1H-indole-3-carboxylic acid methyl ester. Prepared from 1-ethyl-5-(2-ethyl-[1,3]dioxolan-2-yl)-2,3-dihydro-indol-2-one, the title compound of Preparation 22.

### Preparation 4

5-(2-Cyclopentyl-[1,3]dioxolan-2-yl)-1-ethyl-2-oxo-2,3-dihydro-1H-indole-3-carboxylic acid ethyl ester. Prepared from 5-(2-cyclopentyl-[1,3]dioxolan-2-yl)-1-ethyl-2,3-dihydro-indol-2-one, the title compound of Preparation 24.

### Preparation 5

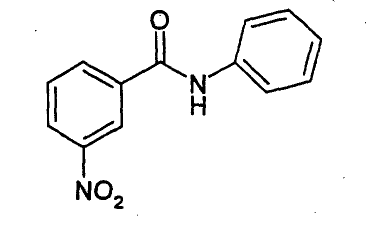

3-Nitro-N-phenyl-benzamide. To a cooled (0°C) solution of aniline (17 g, 180 mmol) and triethylamine (27 mL, 190 mmol) in dichloromethane (100 mL) was added a solution of 3-nitrobenzoyl chloride (30 g, 160 mmol) in dichloromethane (60 mL). The mixture was stirred for 15 min at 0°C then overnight at room temperature. It was then poured into saturated sodium bicarbonate (1 L) and stirred vigorously for 15 min. The precipitate was collected, washed with water and dried (40 g, 100%).

### Preparation 6

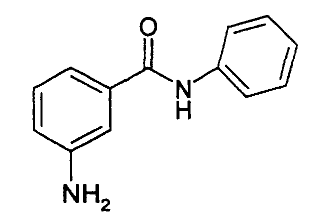

3-Amino-N-phenyl-benzamide. 10% Palladium on carbon (2.0 g) was added to a solution of 3-nitro-N-phenyl-benzamide (20 g, 83 mmol) in ethanol (225 mL) and the mixture was hydrogenated at 45 psi for 2 hours. The mixture was filtered through diatomaceous earth and concentrated to give a colorless solid (mp 118-120°C, 16 g, 90%).

### Preparations 7-15

The compounds of Preparations 7 to 15 were prepared from the appropriate commercially available starting materials in a manner analogous to the methods of Preparations 5 and 6 performed sequentially.

### Preparation 7

3-Amino-N-cyclohexyl-benzamide.

### Preparation 8

3-Amino-N-(4-chloro-phenyl)-benzamide.

### Preparation 9

3-Amino-N-(p-tolyl)-benzamide.

### Preparation 10

3-Amino-N-(4-fluoro-phenyl)-benzamide.

### Preparation 11

3-Amino-N-(4-ethyl-phenyl)-benzamide.

### Preparation 12

3-Amino-N-(3-fluoro-phenyl)-benzamide.

### Preparation 13

3-Amino-N-(4-bromo-phenyl)-benzamide.

### Preparation 14

3-Amino-N-(4-iodo-phenyl)-benzamide.

### Preparation 15

3-Amino-N-(3-methyl-phenyl)-benzamide.

### Preparation 16

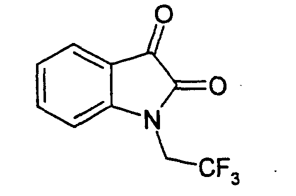

1-(2,2,2-Trifluoro-ethyl)-1H-indole-2,3-dione. Sodium hydride (8.65 g of a 60 % oil dispersion, 0.22 mol) was washed with hexane and suspended in hexamethylphosphoramide (200 mL). Isatin (29.4 g, 0.20 mol)) was added carefully. After the gas evolution subsided, 2,2,2-trifluoroethyl iodide (46.2 g, 0.22 mol) was added and the mixture was heated to 55 °C for 4 hours. The solution was cooled, diluted with water (1 L), and the precipitate was collected. The filtrate was acidified with 6 N HCI and a new precipitate formed and was collected. The combined solids were recrystallized from 95% ethanol. Yield 22.5 (49%).mp 161-163 °C:

### Preparation 17

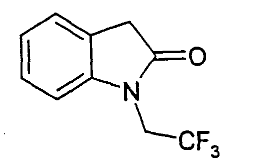

1-(2,2,2-Trifluoro-ethyl)-1,3-dihydro-indol-2-one. A mixture of 1-(2,2,2-trifluoro-ethyl)1H-indole-2,3-dione (9.2 g, 40 mmol) and 10% palladium on carbon (2.4 g) in acetic acid (100 mL) and 70% perchloric acid (6.4 mL, 80 mmol) was hydrogenated in a Parr apparatus for 22 hours. The mixture was filtered through diatomaceous earth, diluted with water (1.5 L) and the precipitate was collected and dried. mp 155-162 °C. Yield 7.5 g (87%).

### Preparation 18

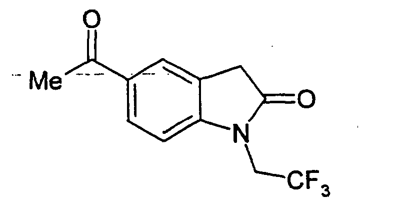

5-Acetyl-1-(2,2,2-trifluoro-ethyl)-1,3-dihydro-indol-2-one. To a solution of 1-(2,2,2-trifluoro-ethyl)-1,3-dihydro-indol-2-one (2.0 g, 9.3 mmol) and acetyl chloride (0.86 mL, 12 mmol) in carbon disulfide (40 mL) was added aluminum trichloride (7.4 g, 56 mmol) by portions. The mixture was heated to reflux for 3 hours, then cooled. The liquid phase was decanted and the residue was quenched carefully with ice, then water. The solids were filtered, washed with water, dried and recrystallized from acetone/hexanes to give a pale pink solid, mp 170-171°C. Yield 1.23 g (51%).

### Preparation 19

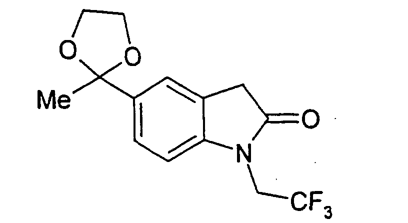

5-(2-Methyl-[1,3]dioxolan-2-yl)-1-(2,2,2-trifluoro-ethyl)-1,3-dihydro-indol-2-one. A solution of 5-acetyl-1-(2,2,2-trifluoro-ethyl)-1,3-dihydro-indol-2-one (1.04 g, 4.0 mmol), ethylene glycol (1.37 mL, 24 mmol) and p-toluenesulfonic acid (10 mg) in benzene (25 mL) was heated to reflux for 4 hours in a flask fitted with a Dean-Stark trap. The solution was diluted with ethyl acetate, washed with saturated sodium bicarbonate, water and brine, dried over magnesium sulfate and concentrated in vacuo to an oil which solidified upon standing (1.19 g, 98%, mp 87-89°C).

### Preparation 20

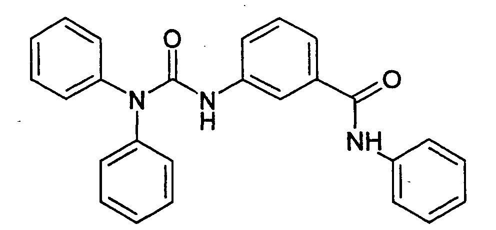

3-(3,3-Diphenyl-ureido)-N-phenyl-benzamide. A mixture of 3-amino-N-phenyl-benzamide (2.0 g, 9.4 mmol), diphenylcarbamoyl chloride (2.2 g, 9.4 mmol) and triethylamine (2.6 mL, 19 mmol) in ethanol (10 mL) was heated to reflux for 4.5 hours. The mixture was cooled and concentrated, water was added, the slurry was acidified with 1 N HCI and the solid was collected, washed with water, dried and recrystallized from acetone / hexanes, to give a colorless solid (1.63 g, 42%).

### Preparation 21

1-Ethyl-5-propionyl-2,3-dihydro-indol-2-one. Prepared from readily available starting materials (N-ethyloxindole and propionyl chloride) in a manner analogous to that set forth in Preparation 18.

### Preparation 22

1-Ethyl-5-(2-ethyl-[1,3]dioxolan-2-yl)-2,3-dihydro-indol-2-one. Prepared from the title compound of Preparation 21 in a manner analogous to that set forth in Preparation 19.

### Preparation 23

5-Cyclopentanecarbonyl-1-ethyl-2,3-dihydro-indol-2-one. Prepared from readily available starting materials (N-ethyloxindole and cyclopentanecarbonyl chloride) in a manner analogous to that set forth in Preparation 18.

### Preparation 24

5-(2-cyclopentyl-[1,3]dioxolan-2-yl)-1-ethyl-2,3-dihydro-indol-2-one. Prepared from the title compound of Preparation 23 in a manner analogous to that set forth in Preparation 19.

A particularly preferred subgroup of formula (III) compounds are those compounds selected from the group consisting of:
5-acetyl-1-ethyl-2-oxo-2,3-dihydro-1H-indole-3-carboxylic acid (3-p-tolylcarbamoyl-phenyl)-amide;
5-acetyl-1-ethyl-2-oxo-2,3-dihydro-1H-indole-3-carboxylic acid (3-(4-bromophenylcarbamoyl-phenyl)-amide; and
5-acetyl-1-ethyl-2-oxo-2,3-dihydro-1H-indole-3-carboxylic acid (3-phenylcarbamoyl-phenyl)-amide; the stereoisomers and prodrugs thereof, and the pharmaceutically acceptable salts of the compounds, stereoisomers, and prodrugs.

The compounds of formula (IV), the stereoisomers and prodrugs thereof, and the pharmaceutically acceptable salts of the compounds, stereoisomers, and the prodrugs, may be prepared according to the following synthetic methodologies.

Exemplary processes for the manufacture of the compounds of the general formula (IV) are provided below and are illustrated by reaction schemes. These processes may be carried out in sequential or convergent synthetic routes. Purification procedures include crystallization and normal phase or reverse phase chromatography.

As a general note, the preparation of the compounds of formula (IV) may also require protection of remote functionality (e.g., primary amine, secondary amine, carboxyl). The need for such protection will vary depending on the nature of the remote functionality and the conditions of the preparation methods. The need for such protection is readily determined by one skilled in the art. The use of such protection/deprotection methods is also within the skill in the art. For a general description of protecting groups and their use, see T.W. Greene and P.G.M. Wuts, *Protective Groups in Organic Synthesis,* John Wiley & Sons, New York, 1991.

The following abbreviations are used herein.
- Et: ethyl
- DMF: dimethylformamide
- BOC: *tert*-butyloxycarbonyl
- CBz: benzyloxycarbonyl
- Ph: phenyl
- h: hours
- d: days
- min: minutes
- equiv: equivalent(s)
- DMSO: dimethlysulfoxide
- dec: decomposition
- mp: melting point
- CIMS: chemical ionization mass spectrometry

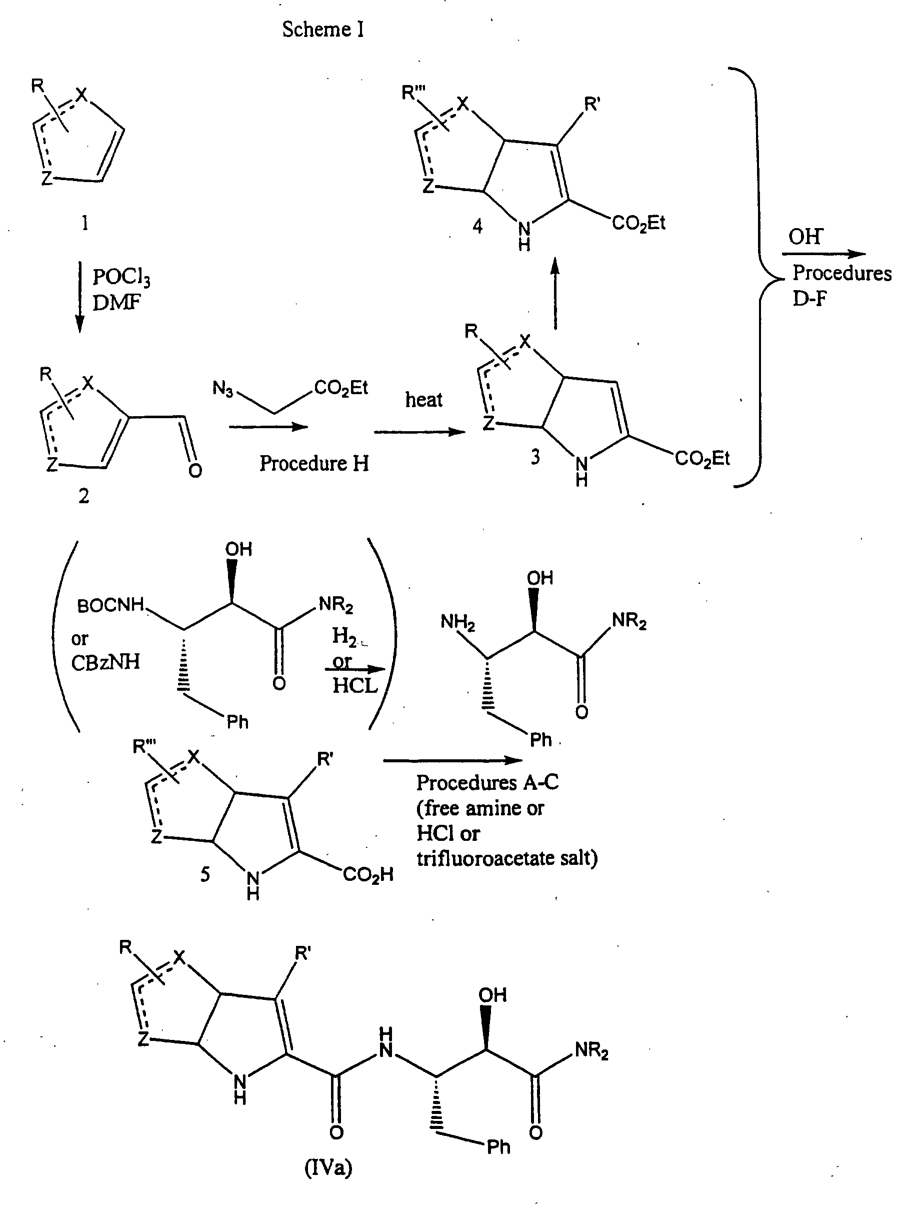

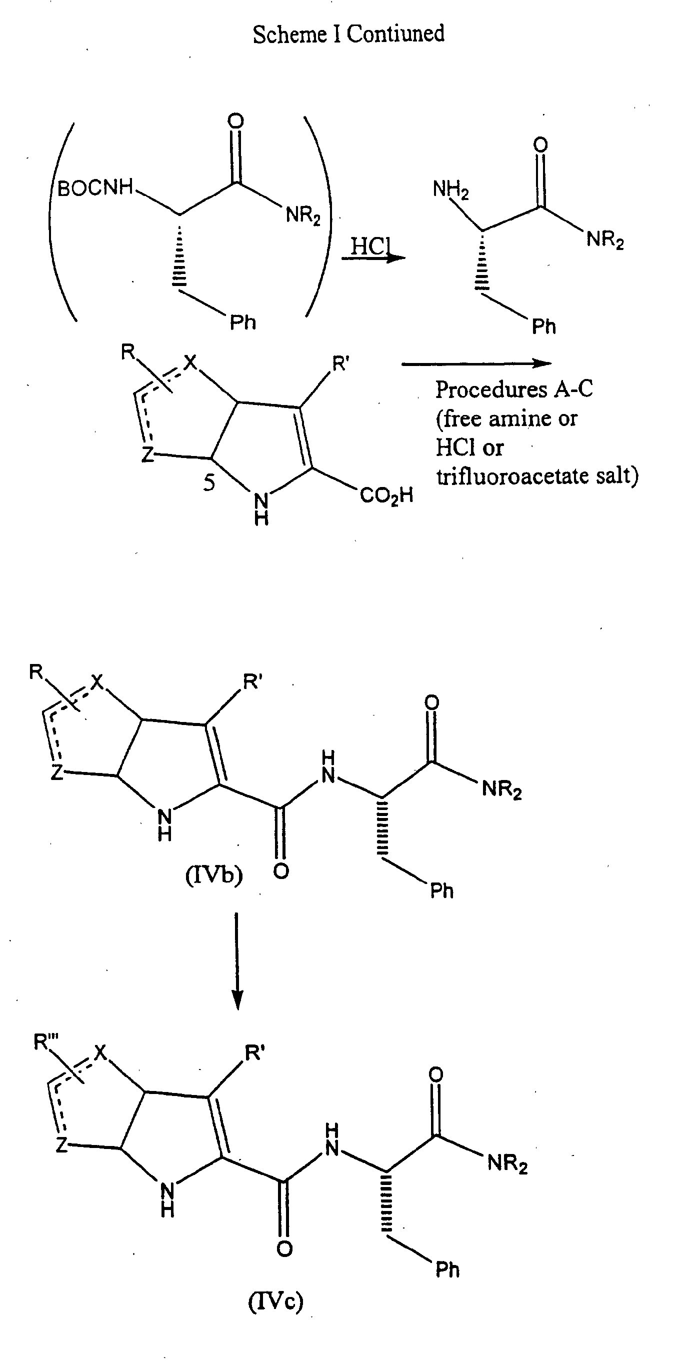

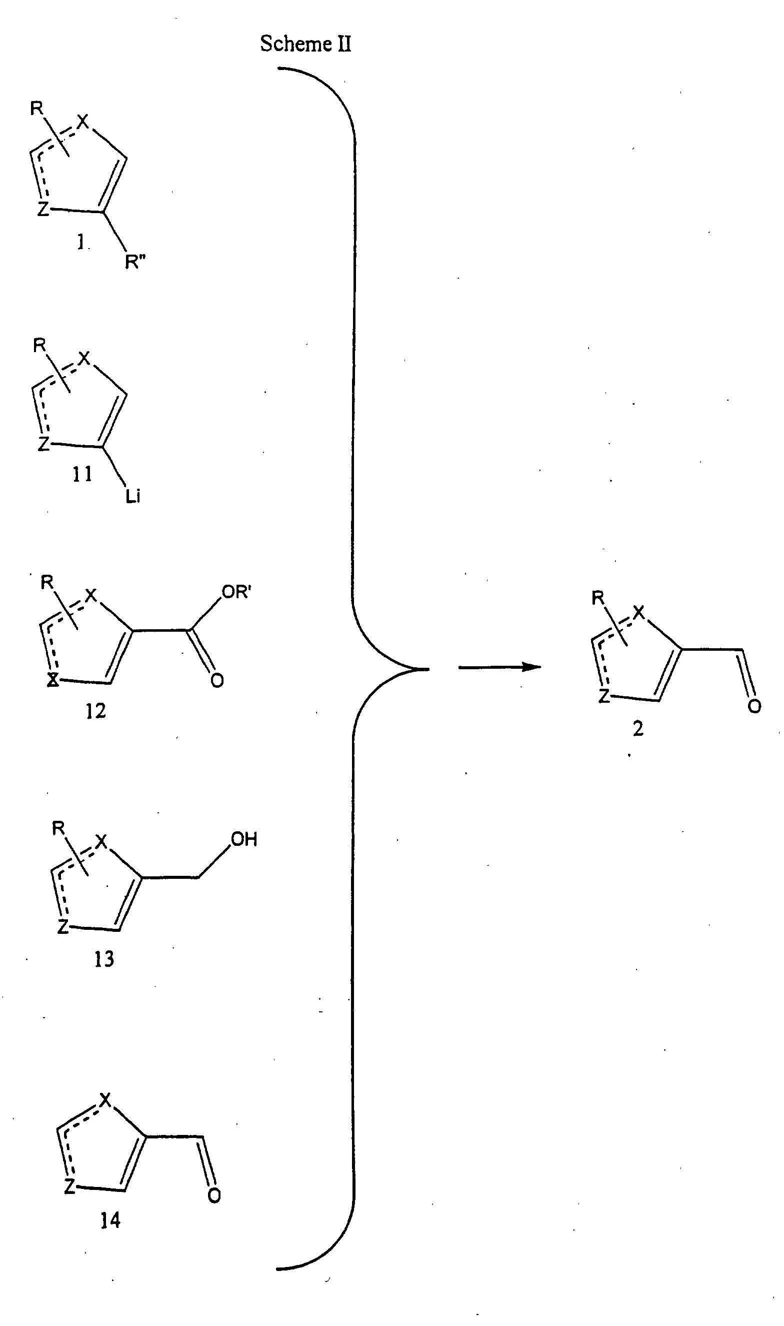

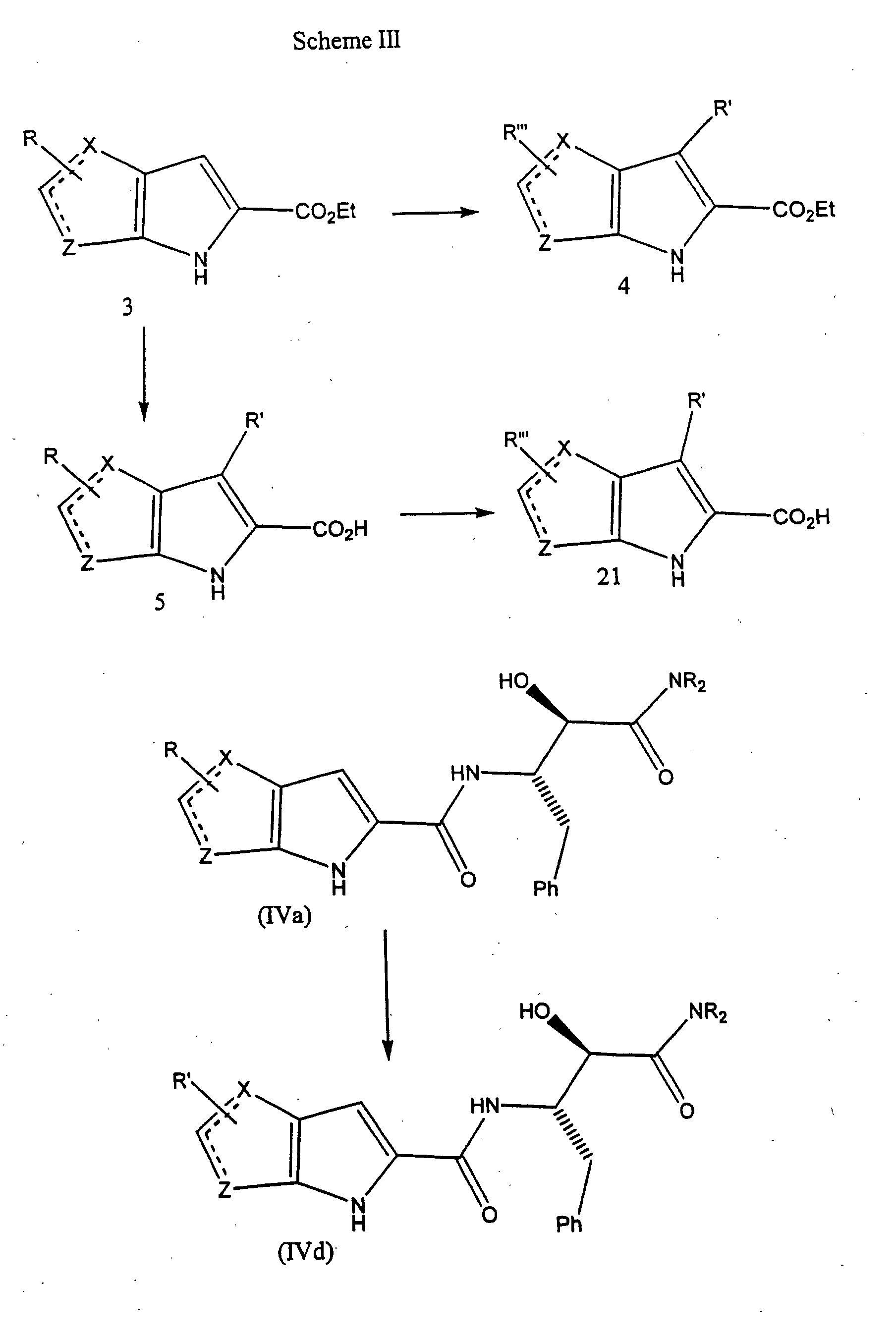

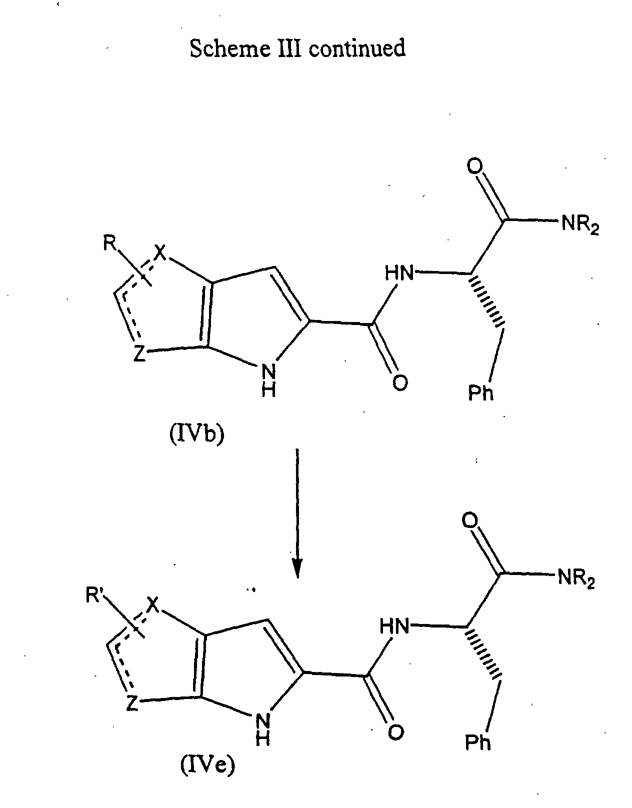

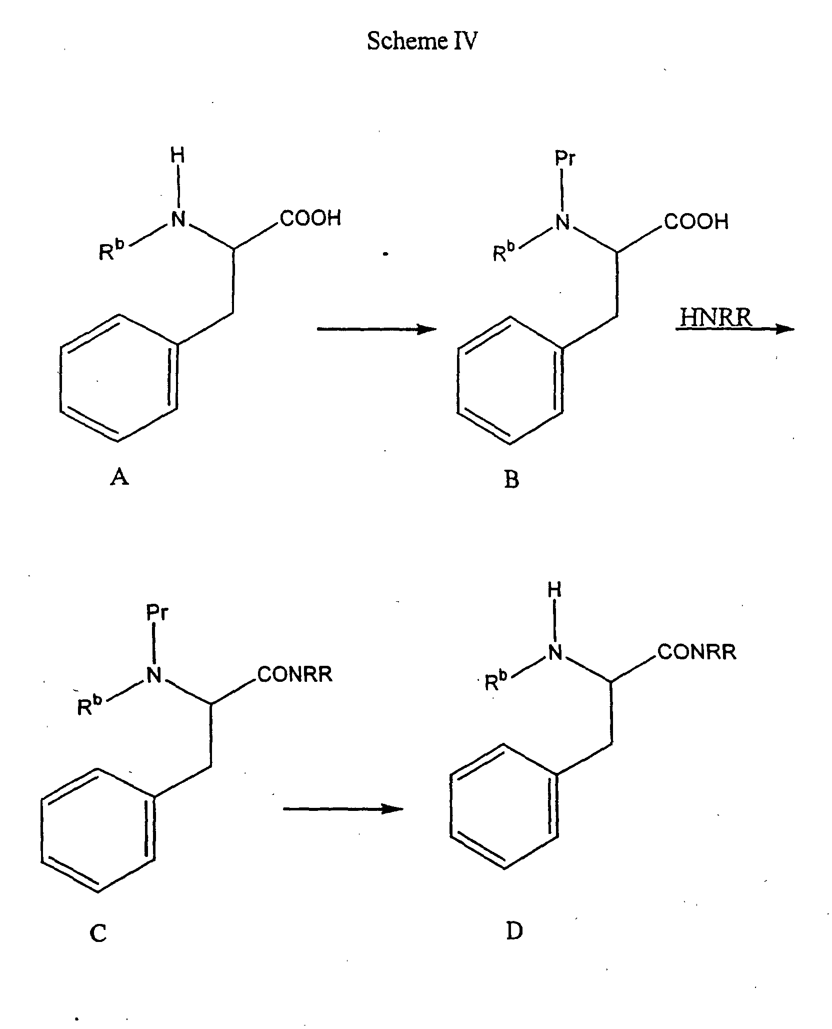

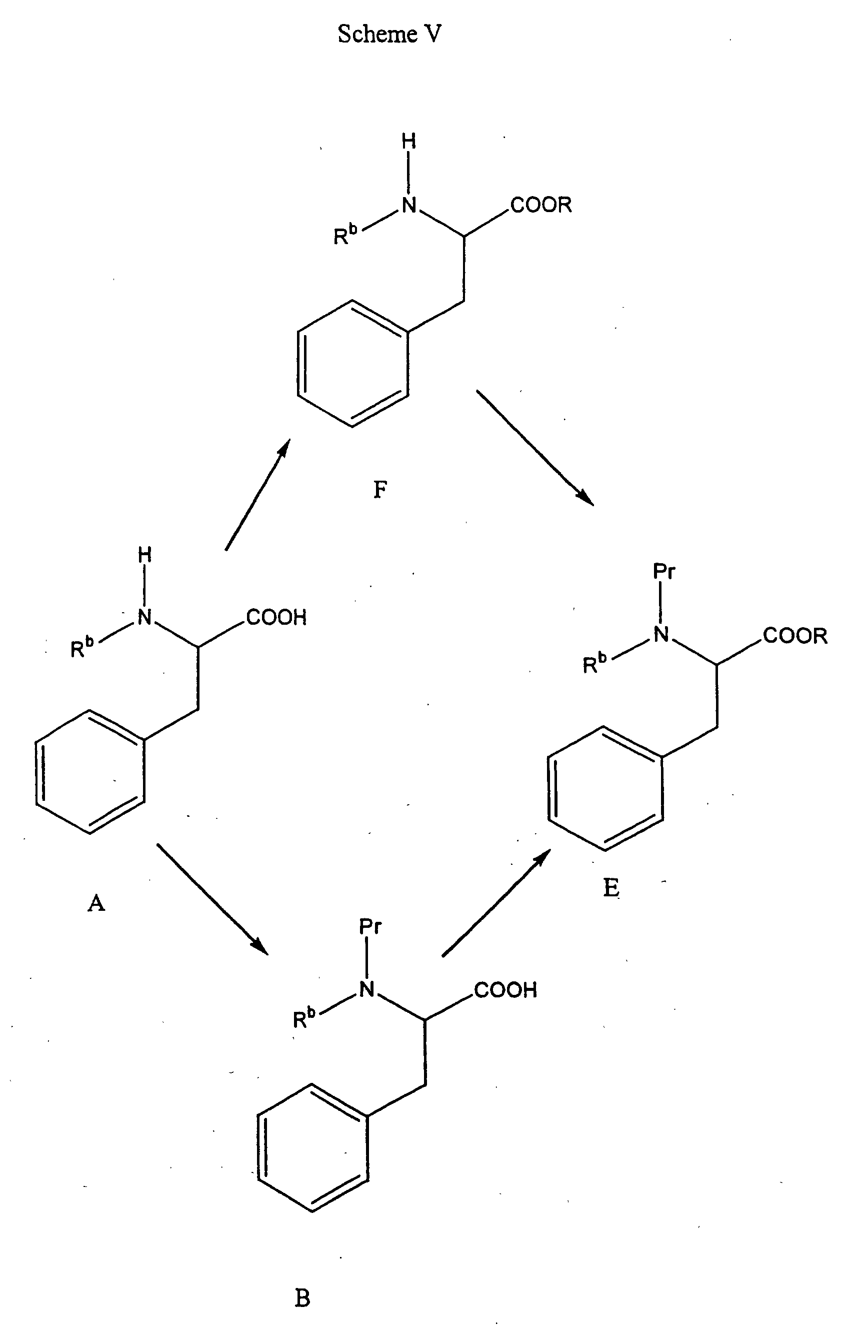

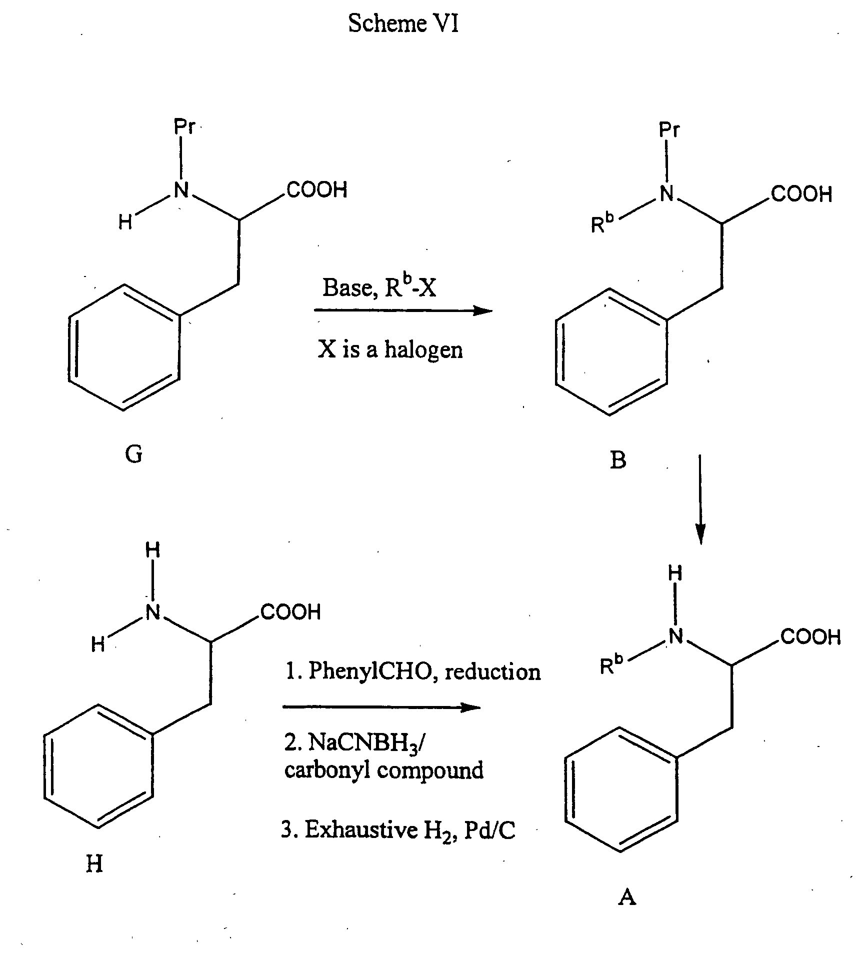

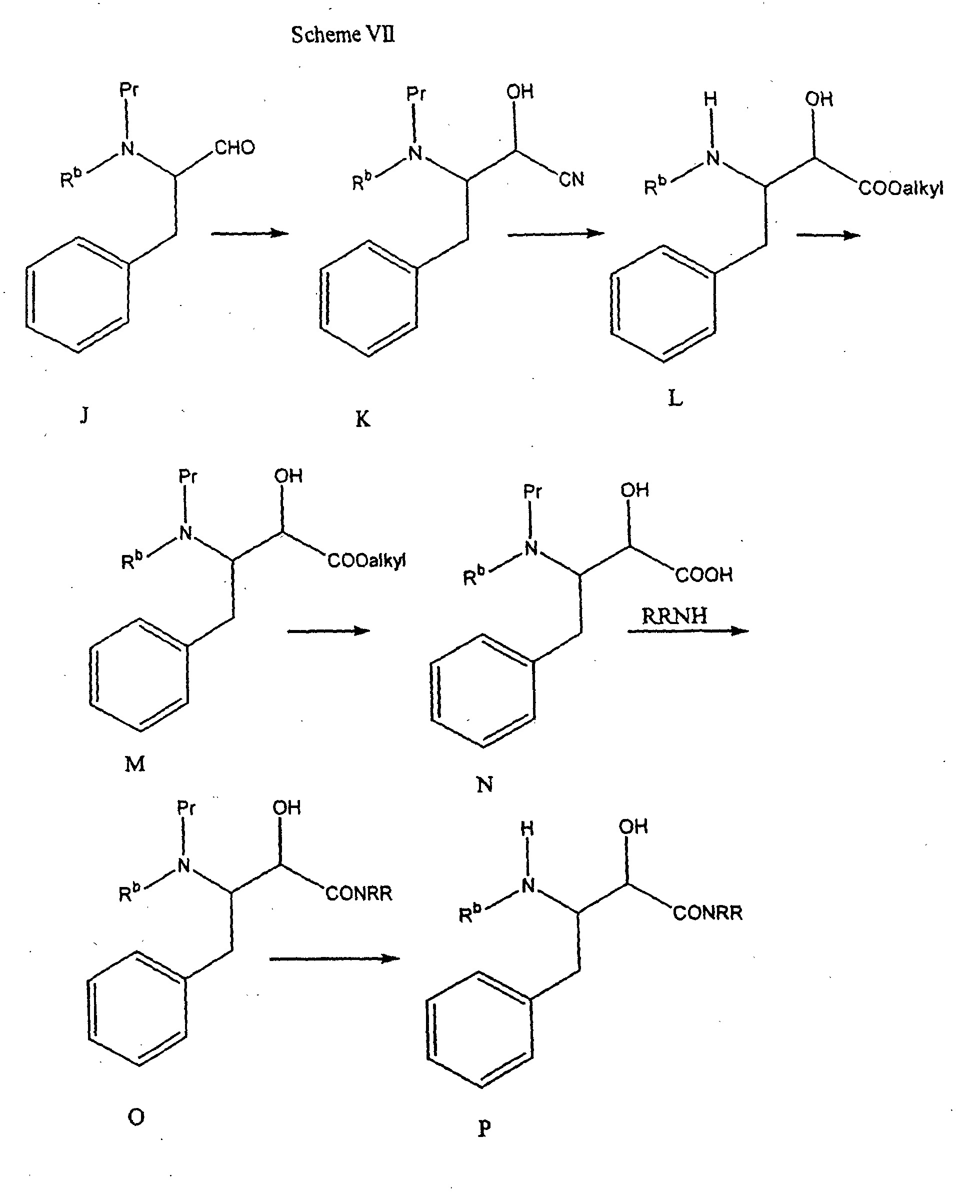

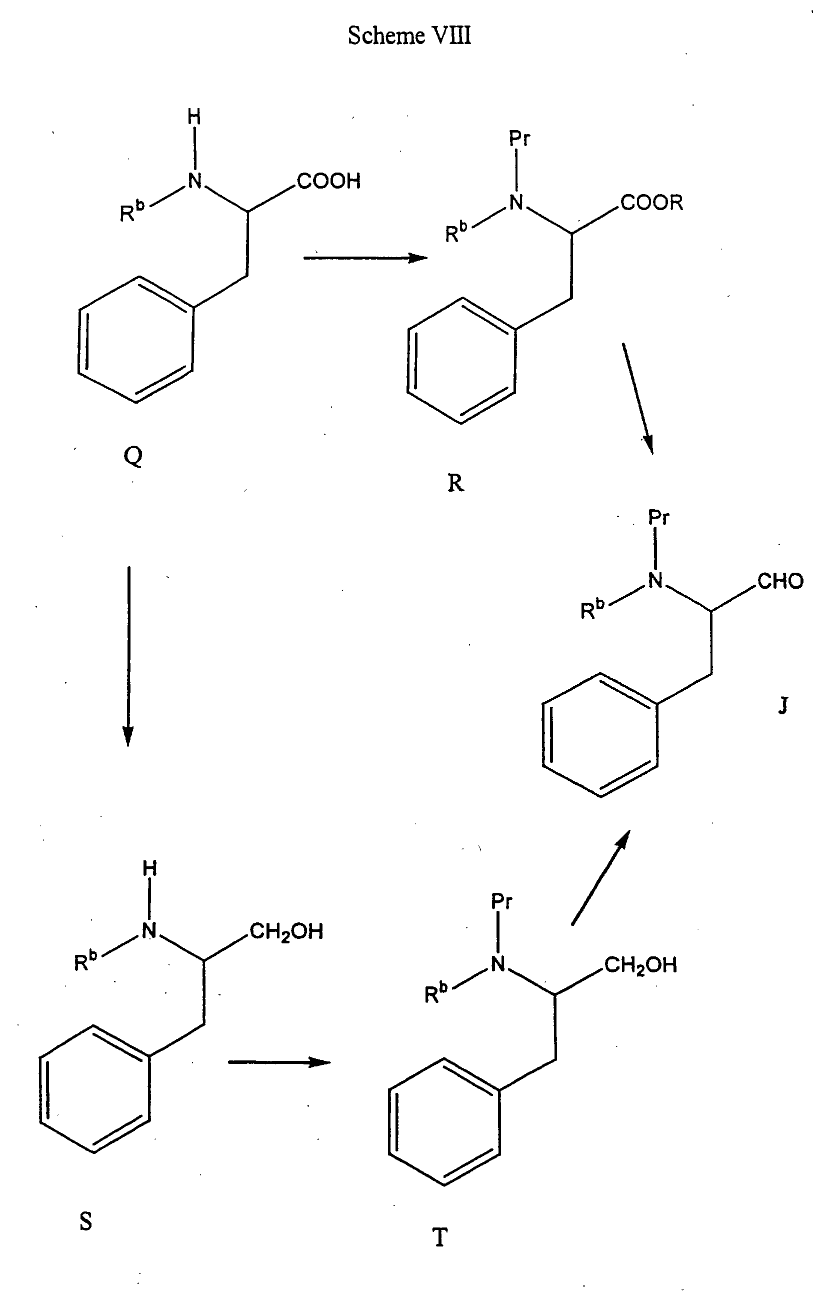

The bicyclic pyrrolyl acids of Formula 5 can be made by several synthetic methods. With regard to Scheme I, a preferred method (Hemetsberger, H. et al., Monatshefte fur Chemie, 103: 194-204 (1972)), begins with condensation of an azido-acetic acid alkyl ester with an aldehyde of Formula 2 in an alcoholic solvent in the presence of an alkoxide. Preferably, the alcohol and alkoxide are derived from the corresponding alkyl ester to avoid transesterification problems. The reaction is performed at a temperature of about -20°C to about 25°C for about 1-24 hours, generally employing 3-8 equivalents of the alkoxide and an equimolar quantity of the azido-acetic acid alkyl ester. The resultant azides are then heated at reflux in an inert solvent such as xylenes to afford the heterocyclopyrrole esters of Formula 3. An example of a suitable preparation is shown by Procedure H below.

The aldehydes of Formula 2 can be made by conventional methods known to those skilled in the art, or methods for their preparation can readily be determined from the chemical literature (See, for example, Ortiz, J. A. et al., Eur. J. Med. Chem., 23: 477-482 (1988)). With regard to Scheme II, exemplary preparations include Villsmeyer-Haack formylation of heterocycles (R" = H) of Formula 1 (See, O. Meth-Cohn and S. P. Stanforth in Comprehensive Organic Synthesis: Selectivity, Strategy & Efficiency in Modem Organic Chemistry Vol. 2, Pergamon, New York, 1991, C. H. Heathcock, Ed., p 777), metal-halogen exchange of bromo- or iodoheterocycles (R" = Br,I) of Formula 1 or lithiation of heterocycles of Formula 1 (R" = H) followed by treatment of heteroaryl lithiums of Formula 11 with a formylating agent such as dimethylformamide (Ortiz, J. A. et al., Eur. J. Med. Chem.,23: 477-482 (1988)) or N-methyl formanilide.(See, D. Comins & S. P. Joseph in Encyclopedia of Reagents for Organic Synthesis Vol. 5, Wiley, New York, 1995, L. A. Paquette, Ed., p 3503), reduction of heterocyclic esters of Formula 12 (R = alkyl) or acids (R = H) to Formula 13 alcohols or aldehydes of Formula 2 (Nicolaou, K. C. et al., Angew. Chem. Int. Ed. Engl., 36: 166-7 (1997)) with reducing agents (Comprehensive Organic Synthesis: Selectivity, Strategy & Efficiency in Modem Organic Chemistry Vol 8, I. Fleming, Ed., Pergamon, 1991, New York) such as lithium aluminum hydride, diisobutylaluminum hydride, or borane and subsequent oxidation of alcohols of Formula 13 to aldehydes of Formula 2 using oxidizing agents (Comprehensive Organic Synthesis: Selectivity, Strategy & Efficiency in Modem Organic Chemistry Vol 7, S. V. Ley, Ed., Pergamon, 1991, New York) such as pyridinium chlorochromate, manganese dioxide, Swem reagent, and barium oxide, or halogenation of the aldehydes of Formula 14 using electrophilic halide sources such as *N*-halosuccinimide (R. M. Kellogg et al., J. Org. Chem., 33: 2902-2909 (1968)), *N-*fluoropyridinium salts (Umemoto, T. et al., J. Am. Chem. Soc., 112: 8563-75 (1990)), or elemental halogen (Ortiz, J. A. et al., Eur. J. Med. Chem., 23: 477-482 1988)).

Alternatively, substitution of the heterocyclopyrroles of Formula 3 can be accomplished by analogous conventional methods known to those skilled in the art or substitution methods can readily be determined from the literature. For example, with regard to Scheme III, mono- and bis-halide substitution can be accomplished by treatment with an electrophilic halide source such as the *N*-halosuccinimide, *N*-fluoropyridinium salts, or elemental halogen (Gale, W. W. et al., J. Org. Chem.,29: 2160-2165 (1964)) to produce heterocyclopyrroles of Formula 4 (R',R'" = H and/or halide). Methyl substitution can be accomplished by Villsmeyer-Haack formylation to aldehydes of Formula 4 (R'" =·CHO) followed by complete reduction of the formyl group under various reducing conditions such as sodium cyanoborohydride in the presence of zinc iodide in dichloroethane (C. K. Lau et. al., J. Org. Chem.,51: 3038-3043 (1964)). Methyl and other alkyl substitution can also be accomplished by coupling Formula 3 bromo- or iodoheterocyclopyrroles (R = Br, I) with alkyl metals such as alkyl copper reagents (Corey, E. J. et al., J. Am. Chem. Soc.89: 3911-12 (1967)). Alkenes and alkynes, in the presence of copper salts such as copper iodide (J. M. Tour et al., J. Org. Chem. 61: 6906-6921 (1996); G. M. Whitesides et al., J. Org. Chem., 53: 2489-2496 (1988)), and alkenyl and alkynyl stannanes (Stille, J. K., Angew. Chem. Int. Ed. Engl., 25: 508-524 (1986)) can also be coupled to the bromo- or iodoheterocyclopyrroles of Formula 3 (R = Br, I) in the presence of a catalyst such as palladium. Palladium catalysts include, but are not limited to, palladium chloride, dichlorobis(triphenylphosphine)palladium (II), tetrakis(triphenylphosphine)palladium (0), and palladium acetate. Other exemplary conditions useful for forming carbon bonds to aromatic rings are described by K. Tamao, D. W. Knight, and K. Sonogashira in Comprehensive Organic Synthesis: Selectivity, Strategy & Efficiency in Modern Organic Chemistry Vol 3 (Pergamon, New York, 1991, G. Pattenden, Ed., pp 435-551). Condensation of hydroxylamine with formylated esters of Formula 4 (R'"=CHO) or acids of Formula 5 (R = CHO) can either directly (Ford, R. E. et al., J. Med. Chem.,29, 538-549 (1986)) or after a second dehydration step (Malicorne, G. et al., Eur. J. Med. Chem. Chim.Ther. 26: 3-11 (1991) afford nitrites. Alternatively, nitrile substitution can be accomplished by coupling cuprous cyanide to the bromo- or iodoheterocyclopyrroles of Formula 3 (R = Br, I) in dimethylformamide (Klemm, L. H. et al., J. Heterocyclic Chem.,21: 785-9 (1984)). Other exemplary conditions useful for forming nitriles are described by R. Grashey in Comprehensive Organic Synthesis: Selectivity, Strategy & Efficiency in Modern Organic Chemistry Vol 6 (Pergamon, New York, 1991, E. Winterfeldt, Ed., p 225). An example of a suitable nitrile preparation is Procedure G below.

Alternatively the aformentioned methods of substituting the heterocyclopyrroles of Formula 3 can also be applied to the amides of Formulae (IVa) and (IVb).

The coupling of an acid of Formula 5 (Scheme I) with an amine of Formula A (Scheme IV) or P (Scheme VII) to furnish compounds of the general formula (IV) can be effected in several ways, which are analogous to those well known to one of ordinary skill in the art.

In a typical coupling procedure, the acid and amine are combined with a suitable coupling agent. A suitable coupling agent is an agent that transforms the carboxylic acid group into a reactive species such that an amide linkage is formed between the carboxylic acid and the amine.

The coupling agent can provide for the coupling in a one-pot process or several steps may be required to achieve the coupling. Examples of suitable coupling agents include 1-(3-dimethylaminopropyl)-3-ethylcarbodiimide hydrochloride/hydroxybenzotriazole (DEC/HBT), carbonyldiimidazole, dicyclohexylcarbodiimide/ hydroxybenzotriazole, 2-ethoxy-1-ethoxycarbonyl-1,2-dihydroquinoline (EEDQ), carbonyldiimidazole/ HBT, propanephosphonic anhydride (propanephosphonic acid anhydride, PAA) and diethylphosphorylcyanide.

The coupling reaction is generally performed in an inert solvent, preferably an aprotic solvent at a temperature of about -20°C to about 50°C for about 1 to about 48 hours, optionally in the presence of a tertiary amine such as triethylamine. Suitable solvents include acetonitrile, dichloromethane, ethyl acetate, dimethylformamide and chloroform, or mixtures thereof.

In an exemplary multistep coupling process, the carboxylic acid group is reacted with the coupling agent to form an activated intermediate, which can be isolated in the first step of the process. In a second step, the activated intermediate is then reacted with the amine to form the amide. Examples of coupling agents that convert an acid to an activated intermediate include thionyl chloride, oxalyl chloride, which form acid chlorides, cyanuric fluoride, which forms acid flourides, or an alkyl chloroformate such as isobutyl or isopropenyl chloroformate (with a tertiary amine base), which forms a mixed anhydride of the carboxylic acid. If the coupling agent is oxalyl chloride, it is advantageous to employ a small amount of dimethylformamide as a cosolvent with another solvent such as dichloromethane to catalyze the formation of the acid chloride. The acid chloride may be coupled with the amine in an appropriate solvent and a suitable base. Acceptable solvent/base combinations include dichloromethane, dimethylforamide or acetonitirile, or mixture thereof in the presence of a tertiary amine base such as triethylamine. Other appropriate solvent/base combinations include water or a C₁-C₅ alcohol, or mixtures thereof, together with a cosolvent such as dichloromethane, tetrahydrofuran or dioxane, and a base such as sodium or potassium carbonate, sodium, potassium or lithium hydroxide, or sodium bicarbonate in sufficient quantity to consume the acid liberated in the reaction. Use of a phase transfer catalyst (typically 1 to 10 mole %) such as a quaternary ammonium halide (e.g., tetrabutylammonium bromide or methyl trioctylammonium chloride) is advantageous when a mixture of only partially miscible cosolvents is employed (e.g. dichloromethane-water or dichloromethane-methanol). Use of these coupling agents and appropriate selection of solvents and temperatures are known to those skilled in the art and can be readily determined from the literature. These and other exemplary conditions useful for coupling carboxylic acids with amines are described in Houben-Weyl, Vol. XV, part II, E. Wunsch, Ed., G. Thieme Verlag, 1974, Stuttgart, and M. Bodansky, *Principles of Peptide Synthesis,* Springer-Verlag Berlin 1984, and *The Peptides: Analysis*, *Synthesis and Biology* (ed. E. Gross and J. Meienhofer), Vols 1-5 (Academic Press, NY 1979-1983).

The amines that are reacted with the carboxylic acid function group to make an amide of the present invention can be synthesized in a number of ways. With regard to Scheme IV, an alpha amino acid of Formula A can be protected on the amine nitrogen with an appropriate protecting group (Pr) to form a protected amino acid of Formula B. The ability to readily select an appropriate amine protecting group is within the purview of one of ordinary skill in the art. For example, two common employed protecting groups are BOC, which is introduced by treating the amino acid with di-*tert*-butyldicarbonate, preferably in a protic solvent or a solvent mixture at high pH, and CBZ, which is introduced by treating the amino acid with benzylchloroformate, preferably in a protic solvent or a solvent mixture, and a base. The amine-protected amino acid intermediate of Formula B is then coupled with an appropriate amine of the formula HNRR, where the R groups are consistent with the compounds of the general formula (IV), in a procedure analogous to the coupling reaction set forth above to form a protected amide compound of Formula C. The protected amide of Formula C can then be deprotected to form an amide of Formula D. If the protecting group is BOC, the deprotection is typically performed by treating the protected compound with an acid in an aprotic solvent. Suitable acids include HCI, CH₃SO₃H, and trifluoroacetic acid.

It may also be desired to make esters of the compounds of Formula A or B. With regard to Scheme V, the esters of compound A and B can be made by reacting the compound with an appropriate alcohol and an acid catalyst such as concentrated sulfuric acid or by treatment with an alkyl halide such as methyl idodide and a base such as potassium carbonate. Compounds of Formula E can also be made by protecting a compound of Formula A, and then forming the ester. Alternatively, compounds of Formula E can be made starting with a compound of Formula A, forming an ester, and then protecting the amine group. Analogous procedures for the formation and cleavage of esters and the protection of amine groups are well known to those skilled in the art.

According to reaction Scheme VI, the compounds of Formula A when R^{b} is not hydrogen can be prepared as follows. The Formula B amino acid can be prepared by N-alkylation of a compound of Formula G, which is an amine protected alpha amino acid. N-alkylation is well known in the art and can be accomplished using an appropriate alkylating agent and a suitable base. Specific procedures for alkylation are described in Benoiton, Can. J. Chem, 55: 906-910 (1985), and Hansen, J. Org. Chem., 50: 945-950 (1977). For example, when R^{b} is methyl, and Pr is BOC, sodium hydride and methyl iodide in tetrahydrofuran can be used. Deprotection of the compound of Formula B furnishes a compound of Formula A.

Alternatively, a compound of Formula H can be N-alkylated by a three step sequence involving reductive benzylation, such as with benzaldehyde followed by Pd/C-catalyzed hydrogenation to give the mono-N-benzyl derivative, and reductive amination with an appropriate carbonyl compound, for example formaldehyde and sodium cyanoborohydride to introduce R^{b} as methyl, to give the N-benzyl, substituted amino acid. The N-benzyl protecting group is conveniently removed, for example, by hydrogenation with an appropriate catalyst, to yield a compound of Formula A. Specific conditions for the three step alkylation procedure are described by Reinhold et al., J. Med. Chem., 11: 258-260 (1968).

While many of the alpha amino acid starting materials are known, they can be synthesized by a number of procedures that are well known in the art. For example, the Strecker synthesis or variations thereof can be used. Accordingly, an aldehyde, sodium or potassium cyanide and ammonium chloride react to form an aminonitrile. It is noted that the aldehyde selected is determined by the desired amino acid. The aminonitirle is then hydrolyzed with a mineral acid to form the desired amino acid. Alternatively, the Bucherer-Berg method may be used where a hydantoin is formed by heating an aldehyde with ammonium carbonate and potassium cyanide followed by hydrolysis, for example, with barium hydroxide in refluxing dioxane, with acid or base to form the desired compounds.

Suitable methods for the synthesis and/or resolution of compounds of Formula H (Scheme VI) (alpha amino acids) are found in reviews by Duthaler, Tetrahedron, 50: 1539-1650 (1994), or by Williams, Synthesis of Optically Active Amino Acids, Pergamon, Oxford, U.K. 1989. An alternative method is disclosed in Corey and Link, J. Am. Chem. Soc., 114: 1906-1908 (1992).

The synthesis of the compounds of formula (IV) where Y is 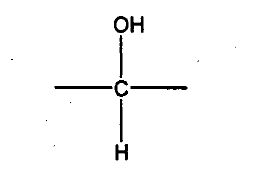 may be accomplished by the coupling of an amide compound of Formula P (Scheme VII) with a bicylic pyrrolyl carboxylic acid of Formula 5. The procedure for the coupling can be carried out as described above. The synthesis of the amides of Formula P is illustrated by Scheme VII. Initially, a nitrogen-protected amino aldehyde of Formula J is treated with potassium or sodium cyanide in aqueous solution with a co-solvent such as dioxane or ethyl acetate at a temperature of about 0°C to about 50°C to provide a compound of Formula K, which is cyanohydrin. The cyanohydrin of Formula K is then reacted with an alcohol such as methanol and a strong acid catalyst such as HCI at a temperature of about 0°C to about 50°C, followed by the addition of water, if necessary. The protecting group is then removed, if still present, by an appropriate deprotection method yielding a compound of Formula L. For example, if the protecting group is BOC, the Formula L compound is directly formed from the Formula K compound, and addition of water is not necessary. The Formula L compound can be protected on the nitrogen to form a compound of Formula M followed by hydrolysis of the ester with aqueous alkali at a temperature of about 0°C to about 50°C in a reaction-inert solvent resulting in the corresponding hydroxy acid of Formula N. The hydroxy acid of formula N is coupled to a suitable amine to form the protected amino amide of Formula O, which is then deptrotected to form a compound of Formula P. An analogous example of the conversion of a Formula K compound to the corresponding Formula L compound is provided in PCT Application Publication No. WO/9325574, Example 1a. Other analogous examples where a cyanohydrin is converted to a Formula M compound can be found in U.S. Pat. No. 4,814,342 and EPO Application Publication No. 0 438 233.

It may be desirable to have a certain stereochemistry at the alpha and beta positions of the compounds of Formula P. (The alpha position is the carbon atom containing the hydroxyl group.) The desired stereochemistry can be obtained by the use of a single stereoisomeric aldehyde of Formula J. The Formula K cyanohydrin can be prepared from the stereochemically pure aldehyde by treatment with sodium or potassium cyanide as described above while maintaining the stereochemistry of the chiral carbon of the aldehyde, resulting in a mixture of stereoisomers, which can be separated, as is well known to those skilled in the art by crystallization. See, for example, Biochemistry, 31: 8125-8141 (1992). Alternatively, isomer separation can be effected by chromatography or recrystallization techniques after conversion of a compound of Formula K to a compound of Formula L, M, N, O, or P by the procedures described herein and analogous to those well known in the art.

With reference to Scheme VIII, the aminoaldehydes of Formula J can be made from the corresponding alpha amino acid of Formula Q. In one method, the alpha amino acid of Formula Q is protected on nitrogen and esterified to form a compound of Formula R. The compound of Formula R is reduced, for example, with diisobutylaluminum hydride in hexane or toluene, or a mixture thereof, at a temperature of about -78°C to about -50°C followed by quenching with methanol at - 78°C as described in J. Med. Chem., 28: 1779-1790 (1985) to form the Formula J aldehyde.

Alternatively, the Formula J aldehydes can be made by oxidation of Formula T alcohols, for example, with pyridine-SO₃ at a temperature of about -10°C to about 40°C in a reaction-inert solvent, preferably dimethylsulfoxide. The protected amino alcohols of Formula T, if not commercially available, can be made by the protection of aminoalcohols of Formula S. The Formula S aminoalcohols are prepared by the reduction of amino acids of formula Q. The reduction can be accomplished by treating Formula Q amino acids with lithium aluminum hydride according to the procedure described by Dickman et al., Organic Synthesis, Wiley: New York, 1990; Collect. Vol. VIII, p. 530. or with sulfuric acid-sodium borohydride by the procedure of Abiko and Masamune, Tetrahedron Lett, 333: 5517-5518 (1992) or with sodium borohydride-iodine according to the procedure of McKennon and Myers, J.Org. Chem., 58: 3568-3571 (1993), where other suitable procedures are also reviewed. The preparation of the alpha amino acid and N-alkylated alpha amino acids has been described above.

In addition, the aforementioned PCT Application Publication Nos. WO 96/3985 and WO 96/39384 contain further details and exemplifications of the processes of synthesizing aspects of the present compounds.

### Equipment and General Procedures

NMR spectra were recorded on a Bruker AM300 or Varian XL-400 spectrometer at about 23°C at 300 or 400 MHz, respectively, for proton nuclei. Unless otherwise specified, NMR spectral data is reported for a 400 MHz spectrometer. Routine mass spectral data were obtained using a VG/Fisons Instruments Platform II spectrometer operating with an Atmospheric Pressure Chemical Ionization (APCI) source. Melting points are uncorrected and were determined on a Thomas Hoover capillary melting point apparatus. Unless otherwise specified, reagents were used as obtained from commercial sources. The term "concentrated" refers to removal of solvent on a rotary evaporator. Exceptions in the use of the Procedures A-H are noted individually in parentheses, following mention of the procedure.

### GENERAL SYNTHETIC PROCEDURES

### Procedure A (Amide Formation Using 1-Hydroxybenzotriazole Hydrate and 1-(3-Dimethylamino-propyl)-3-ethylcarbodiimide Hydrochloride)

A 0 °C 0.1-0.7 M mixture the primary amine (1 equiv, or a primary amine salt and 1 equiv of triethylamine per equiv HCl), 1 equiv of the specified carboxylic acid, and 1 equiv of 1-hydroxybenzotriazole hydrate (1 equiv relative to the carboxylic acid), in 3:1 dichloromethane:dimethylformamide is treated with 1 equiv 1-(3-dimethylaminopropyl)-3-ethylcarbodiimide hydrochloride. The mixture is allowed to warm to room temperature over several hours, stirred overnight, concentrated to remove the dichloromethane, and partitioned between ethyl acetate and 1-2 N HCI. The aqueous phase is extracted with ethyl acetate. The combined organic phases are washed with saturated aqueous NaHCO₃, dried over MgSO₄, and concentrated giving crude product which is purified by chromatography on silica gel and/or recrystallization.

### Procedure B (Amide Formation Using 1-Hydroxy-7-azabenzotriazole Hydrate and 1-(3-Dimethylamino-propyl)-3-ethylcarbodiimide Hydrochloride)

A 0°C 0.1-0.3 M mixture of the primary amine or primary amine salt (1 equiv), 1 equiv of triethylamine, 1 equiv of the specified carboxylic acid, and 1 equiv of 1-hydroxy-7-azabenzotriazole (1 equiv. relative to the carboxylic acid), in dimethylformamide is treated with 1 equiv (corresponding in mol ratio to the carboxylic acid) 1-(3-dimethylamino-propyl)-3-ethylcarbodiimide hydrochloride. The mixture is allowed to warm to room temperature over several hours, stirred overnight, and partitioned between ethyl acetate and 1-2 N HCI. The organic phase is washed with saturated aqueous NaHCO₃, dried over MgSO₄, and concentrated giving crude product which is purified by chromatography on silica gel.

### Procedure C (Amide Formation Using 1-Hydroxy-7-azabenzotriazole Hydrate and 1-(3-Dimethylamino-propyl)-3-ethylcarbodiimide Methiodide)

A 0.3 M mixture of the primary amine hydrochloride (1 equiv), 1.2 equiv of triethylamine, 1 equiv of the specified carboxylic acid, and 1.2 equiv of 1-hydroxybenzotriazole hydrate in dimethylformamide is treated with 1.2 equiv 1-(3-dimethylamino-propyl)-3-ethylcarbodiimide methiodide. The mixture is stirred overnight and partitioned between ethyl acetate and 1 N NaOH. The organic phase is washed sequentially with 1 N HCI and water, dried over MgSO₄, and concentrated giving crude product.

### Procedure D (Hydrolysis of Ethyl Ester with Potassium Hydroxide)

A 0.1-0.8 M suspension of the ethyl ester (1 equiv) and KOH (2 equiv) in water is heated at reflux for 1-7 h, allowed to cool to room temperature, stirred overnight, and extracted with ethyl acetate. The aqueous phase is acidified with 2 N HCI and extracted with ethyl acetate. The combined organic phases are dried over MgSO₄, and concentrated giving crude product which is purified by chromatography and/or washing with solvent.

### Procedure E (Hydrolysis of Ethyl Ester with Sodium Hydroxide)

A 0.1-0.8 M suspension of the ethyl ester (1 equiv) and 2 N NaOH (10 equiv) in methanol is heated at 65°C for 2 h, allowed to cool to room temperature, concentrated to remove the methanol, diluted with water, and extracted with ethyl acetate. The aqueous phase is acidified with 2 N HCI and extracted with ethyl acetate. The combined organic phases are dried over MgSO₄, and concentrated giving crude product which is purified by recrystallization.

### Procedure F (Hydrolysis of Ethyl Ester with Lithium Hydroxide)

A 0.1-0.3 M solution of the ethyl ester (1 equiv) and LiOH-H₂O (4-6 equiv) in 3:2:1 tetrahydrofuran:methanol:water is heated at 60-65°C overnight, allowed to cool to room temperature, concentrated to remove the tetrahydrofuran and methanol, and acidified with 1-2 N HCI. The resultant precipitate is filtered, washed with water, and dried *in vacuo* giving product.

### Procedure G (Nitrile Formation with Hydroxylamine Hydrochloride)

A 0.1-0.2 M mixture of the aldehyde (1 equiv) and hydroxylamine hydrochloride (2.2-4 equiv) in dimethylformamide is heated at 125°C overnight, allowed to cool to room temperature, and partitioned between ethyl acetate and water. The aqueous phase is extracted with ethyl acetate. The combined organic phases are washed with water, dried over MgSO₄, and concentrated giving crude product which is purified by chromatography on silica gel.

### Procedure H (Annulation with Azido-acetic Acid Ethyl Ester)

A 0°C 0.6-1.2 M solution of sodium (3-4 equiv) in ethanol is treated with a mixture of the aldehyde (1 equiv) and azido-acetic acid ethyl ester (1 equiv relative to sodium) dropwise such that the reaction temperature was maintained at 5-10°C. The reaction mixture is stirred for 1-2 h, quenched with cold saturated aqueous NH₄Cl, and extracted with ether. The combined organic phases are dried over MgSO₄ and concentrated. The residue is purified by chromatography on silica gel. A 0.1-0.2 M solution of the resultant acrylate in xylenes is heated at reflux for 20-60 min and allowed to cool to room temperature. The reaction solution is either cooled further to induce crystallization of the product or concentrated giving crude product which is purified by washing with hexanes and/or chromatography on silica gel.

### Example 1

### 6H-Thieno[2,3-b]pyrrole-5-carboxylic acid [(1S)-benzyl-3-((3R,4S)-dihydroxypyrrolidin-1-yl)-(2R)-hydroxy-3-oxo-propyl]-amide

6H-Thieno[2,3-b]pyrrole-5-carboxylic acid (Soth, S. et al., Bull. Soc. Chim. Fr., 2511-2515 (1975)) and (3S)-amino-1-((3R,4S)-dihydroxy-pyrrolidin-1-yl)-(2R)-hydroxy-4-phenyl-butan-1-one were coupled according to Procedure A (4-(dimethylamino)pyridine (0.1 equiv) also added to the reaction mixture); mp 137-145°C; CIMS m/e 430.2 (MH⁺); ¹H NMR (DMSO-*d*₆) δ 11.67 (br s, 1H), 7.74 (d, J = 8.9 Hz, 1H), 7.21 (m, 4H), 7.11 (m, 1H), 6.96 (s, 3H), 5.03 (dd, J = 2.9, 7.5 Hz, 0.5H), 4.93 (m, 1H), 4.87 (m, 0.5H), 4.80 (dd, J = 2.9, 7.5 Hz, 0.5H), 4.74 (br s, 0.5H), 4.40 (br s, 1H), 4.19 (m, 1H), 4.06 (dq, J = 3.2, 5.3 Hz, 0.5H), 3.99-3.87 (m, 1.5H), 3.54. (m, 1H), 3.38 (m, 0.5H), 3.25-3.06 (m, 2.5H), 2.94-2.81 (m, 2H).

### Example 1a

### [(1S)-Benzyl-3-((3R,4S)-dihydroxy-pyrrolidin-1-yl)-(2R)-hydroxy-3-oxopropyl]-carbamic acid benzyl ester

(2R,3S)-3-Benzyloxycarbonylamino-2-hydroxy-4-phenyl-butyric acid (Takita, T. et al., J. Med. Chem., 20: 510-515 (1977)) and pyrrolidine-(3R,4S)-diol hydrochloride were coupled according to Procedure A (dimethylformamide reaction solvent concentrated to ½ volume before work-up).
CIMS m/e 415.2 (MH⁺); ¹H NMR (DMSO-*d*₆) δ 7.28-7.15 (m, 10H), 7.07-7.01 (m, 1H), 4.94-4.75 (m, 4.5H), 4.65 (d, J = 7.7 Hz, 0.5H), 4.09-3.88 (m, 4H), 3.51-3.38 (m, 1H), 3.27-3.07 (m, 3H), 2.83-2.63 (m, 2H).

### Example 1b

### (3S)-Amino-1-((3R,4S)-dihydroxy-pyrrolidin-1 -yl)-(2R)-hydroxy-4-phenyl-butan-1-one

According to a procedure by Takita, T. et al. (J. Med. Chem., 20: 510-515 (1977)) a mixture of [(1S)-benzyl-3-((3R,4S)-dihydroxy-pyrrolidin-1-yl)-(2R)-hydroxy-3-oxo-propyl]-carbamic acid benzyl ester (1.2 g, 2.9 mmol) and 10% palladium on carbon (120 mg) in methanol (20 mL) was shaken under a hydrogen atmosphere (40-45 psi) on a Parr apparatus overnight, filtered through Celite® , and concentrated. The product was obtained as a sticky solid (1.0 g, 100%).
CIMS m/e 281.2 (MH⁺); ¹H NMR (DMSO-*d*₆) δ 7.27-7.13 (m, 5H), 4.95-4.80 (m, 3H), 3.93 (br s, 2H), 3.83 (dd, J = 3.3, 9.1 Hz, 1H), 3.45-3.05 (m, 6H), 2.99 (dq, J = 3.5, 6.3 Hz, 1H), 2.65 (m, 1H), 2.50 (m, 1H).

### Example 2

### 2-Bromo-6H-thieno[2,3-b]pyrrole-5-carboxylic acid [(1S)-benzyl-3-((3R,4S)-dihydroxypyrrolidin-1-yl)-(2R)-hydroxy-3-oxo-propyl]-amide

2-Bromo-6H-thieno[2,3-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure A.
mp 143-145°C; CIMS mle 508.0/510.0 (MH⁺); ¹H NMR (DMSO-*d*₆) δ 11.72 (br s, 1H), 7.84 (d, J = 9.1 Hz, 1H), 7,22 (m, 5H), 7.11 (m, 1H), 6.99 (s, 1H), 5.04 (d, J = 7.3 Hz, 0.5H), 4.95 (m, 1H), 4.89 (d, J = 5.0 Hz, 0.5H), 4.80 (d, J = 7.7 Hz, 0.5H), 4.75 (d, J = 4.4 Hz, 0.5H), 4.40 (m, 1H), 4.19 (m, 1H), 4.00-3.85 (m, 2H), 3.54 (m, 1H), 3.39 (dd, J = 4.9, 12.6 Hz, 0.5H), 3.22 (m, 1.5H), 3.16-3.06 (m, 1H), 2.94-2.81 (m, 2H).

### Example 2a

### 2-Bromo-6H-thieno[2,3-b]pyrrole-5-carboxylic acid

2-Bromo-6H-thieno[2,3-b]pyrrole-5-carboxylic acid ethyl ester (Eras, J.; Galvez, C.; Garcia, F., J. Heterocycl. Chem., 21: 215-217 (1984)) was hydrolyzed according to Procedure F.
CIMS m/e 244.0/246.0 ((M-H)⁺); ¹H NMR (DMSO-*d*₆) δ 12.66 (br s, 1H), 12.10 (br s, 1H), 7.22 (s, 1H), 6.87 (d, J = 2.1 Hz, 1H).

### Example 3

### 2-Methyl-6H-thieno[2,3-b]pyrrole-5-carboxylic acid [(1S)-benzyl-3-((3R,4S)-dihydroxypyrrolidin-1-yl)-(2R)-hydroxy-3-oxo-propyl]-amide

2-Methyl-6H-thieno[2,3-b]pyrrole-5-carboxylic acid and (3S)-amino-1-((3R,4S)-dihydroxy-pyrrolidin-1-yl)-(2R)-hydroxy-4-phenyl-butan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 15:1 dichloromethane:dimethylformamide; combined organic phases washed with water prior to saturated aqueous NaHCO₃); mp 154-157°C; CIMS m/e 442.2 ((M-H)⁺); ¹H NMR (DMSO-*d*₆) δ 11.58 (m, 1H), 7.66 (d, J = 8.5 Hz, 1H), 7.22-7.10 (m, 5H), 6.86 (s, 1H), 6.64 (s, 1H), 5.03-4.73 (m, 3H), 4.38 (br s, 1H), 4.18 (m, 1H), 3.98-3.88 (m, 2H), 3.53 (m, 1H), 3.39-3.05 (m, 3H), 2.90-2.83 (m, 2H), 2.38 (s, 3H).

### Example 3a

### 2-Methyl-6H-thieno[2,3-b]pyrrole-5-carboxylic acid ethyl ester

Using a procedure by C. K. Lau et. al. (J. Org. Chem., 51: 3038-3043 (1986)), a mixture of 2-formyl-6H-thieno[2,3-b]pyrrole-5-carboxylic acid ethyl ester (Soth, S. et al., Bull. Soc. Chim. Fr., 2511-2515 (1975); 500 mg, 2.24 mmol), ZnI₂ (1.08 g, 3.36 mmol), and NaBH₃CN (1.06 g, 16.8 mmol) in dichloroethane (25 mL) was stirred for 7 days and quenched with saturated aqueous NH₄Cl (25 mL). The resultant biphasic mixture was stirred for an additional 30 min, extracted with ethyl acetate, dried over Na₂SO₄, and concentrated. The product was purified by Chromatotron-chromatography (3:2 hexanes:ether) and obtained as a white foam (233 mg, 50%); mp 107-109°C; CIMS m/e 208.3 (MH⁺); ¹H NMR (CDCl₃) δ 9.13 (br s, 1H), 6.94 (s, 1H), 6.61 (s, 1H), 4.33 (q, J = 7.1 Hz, 2H), 2.48 (s, 3H), 1.36 (t, J = 7.1 Hz, 3H).

### Example 3b

### 2-Methyl-6H-thieno[2,3-b]pyrrole-5-carboxylic acid

2-Methyl-6H-thieno[2,3-b]pyrrole-5-carboxylic acid ethyl ester was hydrolyzed according to Procedure E.
mp 180-182°C dec.; CIMS m/e 180.1 ((M-H)⁺); ¹H NMR(DMSO-*d*₆) δ 12.36 (s, 1H), 11.93 (s, 1H), 6.77 (s, 1H), 6.66 (s, 1H), 2.40 (s, 3H), 1.36 (t, J = 7.1 Hz, 3H).

### Example 3c

### [(1S)-Benzyl-3-((3R,4S)-dihydroxy-pyrrolidin-1-yl)-(2R)-hydroxy-3-oxo-propyl]carbamic acid tert-butyl ester

(2R,3S)-3-*tert*-Butoxycarbonylamino-2-hydroxy-4-phenyl-butyric acid and pyrrolidine-(3R,4S)-diol hydrochloride were coupled according to Procedure A (1.05 equiv triethylamine, 1.1 equiv carboxylic acid; 1.5 equiv 1-hydroxybenzotriazole hydrate; 1.1 equiv 1-(3-dimethylamino-propyl)-3-ethylcarbodiimide hydrochloride; after dichloromethane removal, residue partitioned between ethyl acetate and 2 N NaOH; combined organic phases washed sequentially with 2 N HCI and saturated NaCl).
CIMS m/e 381 (MH⁺).

### Example 3d

### (3S)-Amino-1-((3R,4S)-dihydroxy-pyrrolidin-1-yl)-(2R)-hydroxy-4-phenyl-butan-1-one hydrochloride

To a 0°C solution of [(1S)-benzyl-3-((3R,4S)-dihydroxy-pyrrolidin-1-yl)-(2R)-hydroxy-3-oxo-propyl]-carbamic acid *tert*-butyl ester (1.1 g, 2.9 mmol) in methanol (4 mL) was added 4 N HCI in dioxane (7.2 mL, 28.9 mmol). The solution was allowed to slowly warm to room temperature and stirred overnight. The reaction mixture was concentrated and the residue was washed with methanol and dried *in vacuo.* The product was obtained as a white solid (1.03 g, 113%).
CIMS m/e 281.2 (MH⁺).

### Example 4

### (±)-2-Methyl-6H-thieno[2,3-b]pyrrole-5-carboxylic acid [1-benzyl-2-((3R,4S)-dihydroxy-pyrrolidin-1-yl)-2-oxo-ethyl]-amide

2-Methyl-6H-thieno[2,3-b]pyrrole-5-carboxylic acid and (±)-2-amino-1-((3R,4S)-dihydroxy-pyrrolidin-1-yl)-3-phenyl-propan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 15:1 dichloromethane:dimethylformamide; combined organic phases washed with water prior to saturated aqueous NaHCO₃); mp 134-136°C; CIMS m/e 412.0 ((M-H)⁺); ¹H NMR (DMSO-*d*₆) δ 11.60 (s, 1H), 8.37 (m, 1H), 7.27-6.98 (m, 6H), 6.65 (s, 1H), 4.99-4.73 (m, 3H), 4.05-3.82 (m, 2.5H), 3.40-2.87 (m, 5.5H), 2.38 (s, 3H).

### Example 4a

### (±)-[1-Benzyl-2-((3R,4S)-dihydroxy-pyrrolidin-1-yl)-2-oxo-ethyl]-carbamic acid tert-butyl ester

Boc-DL-Phenylalanine and pyrrolidine-(3R,4S)-diol hydrochloride were coupled according to Procedure A (1.5 equiv 1-hydroxybenzotriazole hydrate, 1.1 equiv 1-(3-dimethylamino-propyl)-3-ethylcarbodiimide hydrochloride, dichloromethane; 3 d reaction time).
CIMS m/e 351.2 (MH⁺); ¹H NMR (CDCl₃) δ 7.28-7.19 (m, 5H), 5.35 (m, 1H), 4.52 (m, 1H), 4.14-3.99 (m, 1.5H), 3.78-3.63 (m, 1.5H), 3.46-3.34 (m, 2H), 3.00-2.65 (m, 3H), 1.40 (s, 9H).

### Example 4b

### (±)-2-Amino-1 -((3R,4S)-dihydroxy-pyrrolidin-1 -yl)-3-phenyl-propan-1-one hydrochloride

To a 0°C solution of (±)-[1-benzyl-2-((3R,4S)-dihydroxy-pyrrolidin-1-yl)-2-oxo-ethyl]-carbamic acid *tert*-butyl ester (6.5 g, 20 mmol) in methanol (8 mL) was added 4 N HCI in dioxane (50 mL, 200 mmol). The solution was allowed to slowly warm to room temperature and stirred overnight. The resultant white reaction mixture was diluted with ether and the precipitate was filtered, washed with ether, and dried *in vacuo* The product was obtained as a white solid (5 g, 87%).
CIMS m/e 251.2 (MH⁺); ¹H NMR (300 MHz, DMSO-*d*₆) δ 8.28 (br s, 3H), 7.38-7.21 (m, 5H), 5.11-4.93 (m, 2H), 4.34-4.22 (m, 1H), 3.96 (m, 1H), 3.81-3.70 (m, 1H), 3.89 (m, 0.5H), 3.47 (m, 0.5H), 3.33-2.85 (m, 4H), 2.63 (m, 1H).

### Example 5

### 2-Bromo-6H-thieno[2,3-b]pyrrole-5-carboxylic acid [(1S)-benzyl-2-((3R,4S)-dihydroxypyrrolidin-1-yl)-2-oxo-ethyl]-amide

2-Bromo-6H-thieno[2,3-b]pyrrole-5-carboxylic acid and (2S)-amino-1-((3R,4S)-dihydroxy-pyrrolidin-1-yl)-3-phenyl-propan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 25:1 dichloromethane:dimethylformamide; combined organic phases washed with water prior to saturated aqueous NaHCO₃); mp 140-142°C; CIMS m/e 477.9/479.9 (MH⁺); ¹H NMR (DMSO-*d*₆) δ 11.73 (s, 1H), 8.55 (d, J = 8.1 Hz, 1H), 7.26-7.09 (m, 7H), 5.00 (br s, 0.5H), 4.91-4.85 (m, 1.5H), 4.77 (m, 1H), 4.07-3.93 (m, 1.5H), 3.83 (m, 1.5H), 3.41-3.25 (m, 1H), 3.13 (m, 2H), 3.00-2.87 (m, 2H).

### Example 5a

### [(1S)-Benzyl-2-((3R,4S)-dihydroxy-pyrrolidin-1-yl)-2-oxo-ethyl]-carbamic acid tert-butyl ester

Boc-L-Phenylalanine and pyrrolidine-(3R,4S)-diol hydrochloride were coupled according to Procedure A (1.5 equiv 1-hydroxybenzotriazole hydrate, dichloromethane; reaction mixture diluted with ethyl acetate and washed sequentially with 1 N NaOH, 1 N HCI, and saturated sodium chloride prior to drying).
CIMS m/e 351.2 (MH⁺); ¹H NMR (DMSO-*d*₆) δ 7.25-7.13 (m, 5H), 7.06 (dd, J = 8.4, 13.6 Hz, 1H), 4.98 (d, J = 5.4 Hz, 0.5H), 4.91 (d, J = 5.0 Hz, 0.5H), 4.84 (m, 1H), 4.25 (dd, J = 8.5, 14.3 Hz, 1H), 4.02 (m, 0.5H), 3.94 (m, 0.5H), 3.79 (m, 1H), 3.68 (dd, J = 5.9, 10.1 Hz, 0.5H), 3.38 (dd, J = 5.3, 12.2 Hz, 0.5H), 3.27-3.10 (m, 3H), 2.83-2.67 (m, 2H), 1.27 (s, 5H), 1.25 (s, 4H).

### Example 5b

### (2S)-Amino-1-((3R,4S)-dihydroxy-pyrrolidin-1-yl)-3-phenyl-propan-1-one hydrochloride

To 4 N HCI in dioxane (120 mL, 480 mmol) was added [(1S)-benzyl-2-((3R,4S)-dihydroxy-pyrrolidin-1-yl)-2-oxo-ethyl]-carbamic acid *tert*-butyl ester (27 g, 77 mmol). The solution was stirred 2.5 h and concentrated. The product was obtained as a white solid (21.5 g, 98%).
CIMS m/e 251.0 (MH⁺); ¹H NMR (DMSO-*d*₆) δ 8.33 (br s, 3H), 7.32-7.16 (m, 5H), 5.10-4.86 (m, 2H), 4.25-4.13 (m, 1H), 3.93 (m, 1H), 3.73-3.66 (m, 1H), 3.54 (m, 0.5H), 3.46-3.23 (m, 1.5H), 3.18-3.05 (m, 2H), 3.00 (m, 0.5H), 2.91-2.79 (m, 1H), 2.57 (dd, J = 5.6, 10.0 Hz, 0.5H).

### Example 6

### 2-Chloro-6H-thieno[2,3-b]pyrrole-5-carboxylic acid [(1S)-benzyl-3-((3R,4S)-dihydroxy-pyrrolidin-1-yl)-(2R)-hydroxy-3-oxo-propyl]-amide

2-Chloro-6H-thieno[2,3-b]pyrrole-5-carboxylic acid and (3S)-amino-1-((3R,4S)-dihydroxy-pyrrolidin-1-yl)-(2R)-hydroxy-4-phenyl-butan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 25:1 dichloromethane:dimethylformamide; combined organic phases washed with water prior to saturated aqueous NaHCO₃); mp 148-152°C; CIMS m/e 464.0/465.9 (MH⁺); ¹H NMR (DMSO-*d*₆) δ 11.71 (m, 1H), 7.84 (d, J = 8.9 Hz, 1H), 7.23-6.98 (m, 7H), 5.05-4.74 (m, 3H), 4.39 (m, 1H), 4.20 (m, 1H), 4.02-3.88 (m, 2H), 3.54 (m, 0.5H), 3.41-3.06 (m, 3.5H), 2.94-2.83 (m, 2H).

### Example 6a

### 2-Chloro-6H-thieno[2,3-b]pyrrole-5-carboxylic acid ethyl ester

Using a modified procedure by R. M. Kellogg et al. (J. Org. Chem., 33: 2902-290 (1968)), to a 0°C solution of 6H-thieno[2,3-b]pyrrole-5-carboxylic acid ethyl ester (Eras, J.; Galvez, C.; Garcia, F., J. Heterocycl *Che*m., 21: 215-217 (1984); 1.45 g, 7.44 mmol) in acetic acid (15 mL) and CHCl₃ (15 mL) was added *N*-chlorosuccinimide (1.04 g, 7.81 mmol) over 2 h. The reaction mixture was slowly allowed to warm to room temperature over several hours, stirred overnight, concentrated to remove the chloroform, diluted with water, basified with 5 N NaOH, and extracted with ethyl acetate. The combined organic phases were washed with saturated aqueous NaHCO₃, dried over MgSO₄, and concentrated. The product was purified by chromatron chromatography (radial) using 90:10 hexanes/diethyl ether and then then then the product obtained was recrystallized using hexanes/diethyl ether (90:10). Last, the product of the recrystallization was further purified by flash column chromatography using 90:10 petroleum ether/isopropyl ether. The resulting product was obtained as a white solid (824 mg, 48%).
CIMS m/e 228.2/230.2 ((M-H)⁺); ¹H NMR (CDCl₃) δ 9.28 (br s, 1H), 6.98 (d, J = 1.9 Hz, 1H), 6.88 (s, 1H), 4.33 (q, J = 7.2 Hz, 2H), 1.35 (t, J = 7.2 Hz, 3H).

### Example 6b

### 2-Chloro-6H-thieno[2,3-b]pyrrole-5-carboxylic acid

2-Chloro-6H-thieno[2,3-b]pyrrole-5-carboxylic acid ethyl ester was hydrolyzed according to Procedure D (reaction heated at 85 °C).
CIMS m/e 200.1/202.1 ((M-H)⁺); ¹H NMR (DMSO-*d*₆) δ 12.66 (br s, 1H), 12.08 (s, 1H), 7.11 (d, J = 1.9Hz, 1H), 6.86 (t, J = 2.1 Hz, 1H).

### Example 7

### 2-Chloro-6H-thieno[2,3-b]pyrrole-5-carboxylic acid [(1S)-benzyl-2-((3R,4S)-dihydroxy-pyrrolidin-1-yl)-2-oxo-ethyl]-amide

2-Chloro-6H-thieno[2,3-b]pyrrole-5-carboxylic acid and (25)-amino-1-((3R,4S)-dihydroxy-pyrrolidin-1-yl)-3-phenyl-propan-1-one hydrochloride were coupled according to Procedure A (1.5 equiv. 1-hydroxybenzotriazole hydrate, 1.1 equiv. 1-(3-dimethylamino-propyl)-3-ethylcarbodiimide hydrochloride, 25:1 dichloromethane:dimethylformamide; combined organic phases washed with water prior to saturated aqueous NaHCO₃); mp 142-145°C; CIMS m/e 432.1/434.2 ((M-H)⁺); ¹H NMR (DMSO-*d*₆) δ 11.72 (m, 1H), 8.55 (d, J = 8.5 Hz, 1H), 7.28-7.10 (m, 7H), 5.00 (d, J = 5.2 Hz, 0.5H), 4.99-4.70 (m, 2.5H), 4.09-3.76 (m, 2.5H), 3.41-3.24 (m, 2H), 3.13 (m, 1.5H), 3.02-2.87 (m, 2H).

### Example 8

### 2,4-Dichloro-6H-thieno[2,3-b]pyrrole-5-carboxylic acid [(1S)-benzyl-3-((3R,4S)-dihydroxy-pyrrolidin-1-yl)-(2R)-hydroxy-3-oxo-propyl]-amide

2,4-Dichloro-6H-thieno[2,3-b]pyrrole-5-carboxylic acid and (3S)-amino-1-((3R,4S)-dihydroxy-pyrrolidin-1-yl)-(2R)-hydroxy-4-phenyl-butan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 25:1 dichloromethane:dimethylformamide, 0.06 M); mp 130-134°C (dec.); CIMS m/e 496.2/498.2 ((M-H)⁺); ¹H NMR (DMSO-*d*₆) δ 12.13 (br s, 1H), 7.40-7.15 (m, 7H), 5.51 (d, J = 5.8 Hz, 0.5H), 5.38 (d, J = 6.3 Hz, 0.5H), 4.99 (d, J = 4.9 Hz, 1H), 4.92 (d, J = 4.8 Hz, 0.5H), 4.85 (d, J = 4.1 Hz, 0.5H), 4.55-4.40 (m, 1H), 4.26 (m, 1H), 4.08-3.90 (m, 2H), 3.56-2.89 (m, 6H).

### Example 8a

### 2,4-Dichloro-6H-thieno[2,3-b]pyrrole-5-carboxylic acid ethyl ester

To a 0°C solution of 6H-thieno[2,3-b]pyrrole-5-carboxylic acid ethyl ester (180 mg, 0.92 mmol) in acetic acid (2 mL) and CHCl₃ (2 mL) was added *N*-chlorosuccinimide (294 mg, 2.2 mmol) over 30 min. The reaction mixture was slowly allowed to warm to room temperature over several hours, stirred overnight, concentrated to remove the chloroform, diluted with water, basified with 5 N NaOH, and extracted with ethyl acetate. The combined organic phases were washed with saturated aqueous NaHCO₃, dried over MgSO₄, and concentrated. The product was purified by Chromatron-chromatography (4:1 hexanes:ether) and obtained as a white solid (180 mg, 74%); mp 166-167°C; CIMS m/e 262.1/264.1 ((M-H)⁺); ¹H NMR (300 MHz, CDCl₃) δ 9.21 (br s, 1H), 6.90 (s, 1H), 4.37 (q, J = 7.2 Hz, 2H), 1.39 (t, J = 7.2 Hz, 3H).

### Example 8b

### 2,4-Dichloro-6H-thieno[2,3-b]pyrrole-5-carboxylic acid

2,4-Dichloro-6H-thieno[2,3-b]pyrrole-5-carboxylic acid ethyl ester was hydrolyzed according to Procedure E (reflux for 12 h before allowing to cool to room temperature; acidification with concentrated HCI; no purification).
CIMS m/e 234.0/236.0 ((M-H)⁺); ¹H NMR (DMSO-*d*₆) δ 12.28 (br s, 1H), 7.17 (s, 1H).

### Example 9

### (±)-4H-Thieno[3,2-b]pyrrole-5-carboxylic acid [1-benzyl-2-((3R,4S)-dihydroxypyrrolidin-1-yl)-2-oxo-ethyl]-amide

4H-Thieno[3,2-b]pyrrole-5-carboxylic acid (Soth, S.; Farnier, M.; Paulmier, C., Can. J. Chem. 56, 1429-34 (1978)) and (±)-2-amino-1-((3R,4S)-dihydroxy-pyrrolidin-1-yl)-3-phenyl-propan-1-one hydrochloride were coupled according to Procedure A (9:1 dichloromethane:dimethylformamide, 0.06 M; combined organic phases washed with 2 N NaOH, dried over Na₂SO₄); mp 212°C; CIMS m/e 400.1 (MH⁺); ¹H NMR (DMSO-*d*₆) δ 11.59 (m, 1H), 8.51 (d, J = 8.5 Hz, 1H), 7.36 (d, J = 5.2 Hz, 1H), 7.30-7.11 (m, 5H), 6.92 (m, 1H), 5.01 (d, J = 5.0 Hz, 0.5H), 4.92 (d, J = 4.8 Hz, 0.5H), 4.87-4.76 (m, 2H), 4.04-3.93 (m, 1H), 3.83 (m, 1.5H), 3.43-3.25 (m, 2.5), 3.13 (m, 1H), 3.03-2.86 (m, 2H).

### Example 10

### 2-Bromo-4H-thieno[3,2-b]pyrrole-5-carboxylic acid [(1S)-benzyl-3-((3R,4S)-dihydroxypyrrolidin-1-yl)-(2R)-hydroxy-3-oxo-propyl]-amide

2-Bromo-4H-thieno[3,2-b]pyrrole-5-carboxylic acid and (3S)-amino-1-((3R,4S)-dihydroxy-pyrrolidin-1-yl)-(2R)-hydroxy-4-phenyl-butan-1-one hydrochloride were coupled according to Procedure B (1.5 equiv 1-hydroxy-7-azabenzotriazole hydrate, 1.1 equiv 1-(3-dimethylamino-propyl)-3-ethylcarbodiimide hydrochloride, 25:1 dichloromethane:dimethylformamide; reaction mixture stirred for 5 d, concentrated to remove dichloromethane before work-up; combined organic phases washed with water prior to saturated aqueous NaHCO₃); mp 138-143°C; CIMS m/e 508.0/510.0 (MH⁺); ¹H NMR (DMSO-*d*₆) δ 11.68 (m, 1H), 7.86 (d, J = 9.1 Hz, 1H), 7.25-7.18 (m, 4H), 7.12-7.08 (m, 2H), 7.02 (s, 1H), 5.05 (d, J = 7.5 Hz, 0.5H), 4.95 (m, 1H), 4.88 (d, J = 5.0 Hz, 0.5H), 4.81 (d, J = 7.5 Hz, 0.5H), 4.75 (d, J = 3.5 Hz, 0.5H), 4.46-4.37 (m, 1H), 4.20 (m, 1H), 4.08-3.85 (m, 2H), 3.54 (m, 1H), 3.40-3.05 (m, 3H), 2.95-2.81 (m, 2H).

### Example 10a

### 2-Bromo-4H-thieno[3,2-b]pyrrole-5-carboxylic acid

2-Bromo-4H-thieno[3,2-b]pyrrole-5-carboxylic acid ethyl ester (Eras, J.; Galvez, C.; Garcia, F. J., Heterocycl. Chem., 21: 215-217 (1984)) was hydrolyzed according to Procedure D (after cooling to room temperature, acidification with 2 N HCI; resultant precipitate filtered, suspended in toluene, concentrated; no purification).
CIMS m/e 244.0/246.0 ((M-H)⁺); ¹H NMR (DMSO-*d*₆) δ 12.63 (s, 1H), 12.04 (s, 1H), 7.13 (s, 1H), 6.97 (s, 1H).

### Example 11

### 4H-Thieno[3,2-b]pyrrole-5-carboxylic acid [(1S)-benzyl-3-((3R,4S)-dihydroxypyrrolidin-1-yl)-(2R)-hydroxy-3-oxo-propyl]-amide

4H-Thieno[3,2-b]pyrrole-5-carboxylic acid and (3S)-amino-1-((3R,4S)-dihydroxypyrrolidin-1-yl)-(2R)-hydroxy-4-phenyl-butan-1-one hydrochloride were coupled according to Procedure A (combined organic phases washed with 2 N NaOH, dried over Na₂SO₄); mp 185-190°C; CIMS m/e 430.1 (MH⁺); ¹H NMR (DMSO-*d*₆) δ 11.57 (s, 0.5H), 11.53 (s, 0.5H), 7.80 (d, J = 8.9 Hz, 1H), 7.32 (dd, J = 0.9, 5.3 Hz, 1H), 7.23 (m, 4H), 7.12 (m, 1H), 7.07 (s, 1H), 6.91 (m, 1H), 5.06 (d, J = 7.3 Hz, 0.5H), 4.96 (m, 1H), 4.89 (d, J = 5.2 Hz, 0.5H), 4.82 (d, J = 7.5 Hz, 0.5H), 4.76 (d, J = 4.2 Hz, 0.5H), 4.45-4.38 (m, 1H), 4.21 (m, 1H), 4.01-3.86 (m, 2H), 3.55 (m, 1H), 3.40 (dd, J = 4.9, 12.6 Hz, 0.5H), 3.23 (m, 1.5H), 3.17-3.07 (m, 1H), 2.97-2.83 (m, 2H).

### Example 12

### (±)-2-Bromo-4H-furo[3,2-b]pyrrole-5-carboxylic acid [1-benzyl-2-((3R,4S)-dihydroxypyrrolidin-1-yl)-2-oxo-ethyl]-amide

2-Bromo-4H-furo[3,2-b]pyrrole-5-carboxylic acid and (±)-2-amino-1-((3R,4S)-dihydroxy-pyrrolidin-1-yl)-3-phenyl-propan-1-one hydrochloride were coupled according to Procedure A (1:1 dichloromethane:dimethylformamide; reaction mixture stirred for 3 d; combined organic phases washed with 2 N NaOH, dried over Na₂SO₄); mp 100-101°C (dec.); CIMS m/e 462.2/464.1 (MH⁺); ¹H NMR (DMSO-*d*₆) δ 11.33 (m, 1H), 8.41 (dd, J = 2.8, 8.2 Hz, 1H), 7.27-7.18 (m, 4H), 7.13 (m, 1H), 6.93 (d, J = 5.8 Hz, 1H), 6.69 (d, J = 0.8 Hz, 1H), 4.98-4.75 (m, 3H), 3.99 (m, 0.5H), 3.93 (m, 0.5H), 3.81 (m, 1.5H), 3.41-3.21 (m, 2.5H), 3.12 (m, 1H), 3.00-2.82 (m, 2H).

### Example 12a

### 2-Bromo-4H-furo[3,2-b]pyrrole-5-carboxylic acid

2-Bromo-4H-furo[3,2-b]pyrrole-5-carboxylic acid ethyl ester (Krutosikova, A.; Kovac, J.; Dandarova, M.; Lesko, J.; Ferik, S., Collect. Czech. Chem. Comm., 46: 2564-2573 (1981)) was hydrolyzed according to Procedure E (4 equiv 2 N NaOH, ethanol; reflux 5h, room temperature overnight; after concentration to remove ethanol, residue partitioned between ethyl acetate and 2 N HCI; combined organic phases dried over Na₂SO₄; no purification); ¹H NMR (DMSO-*d*₆) δ 12.47 (br s, 1H), 11.67 (s, 1H), 6.76 (d, J = 0.8 Hz, 1H), 6.67 (t, J = 0.8 Hz, 1H).

### Example 13

### 2-Bromo-4H-furo[3,2-b]pyrrole-5-carboxylic acid [(1S)-benzyl-3-((3R,4S)-dihydroxypyrrolidin-1-yl)-(2R)-hydroxy-3-oxo-propyl]-amide

2-Bromo-4H-furo[3,2-b]pyrrole-5-carboxylic add and (3S)-amino-1-((3R,4S)-dihydroxy-pyrrolidin-1-yl)-(2R)-hydroxy-4-phenyl-butan-1-one hydrochloride were coupled according to Procedure A (2:1 dichloromethane:dimethylformamide; reaction mixture stirred for 3 d; combined organic phases washed with 2 N NaOH, dried over Na₂SO₄); mp 112-123°C (dec.); CIMS m/e 492.1/494.1 (MH⁺); ¹H NMR (DMSO-*d*₆) δ 11.31 (s, 0.5H), 11.27 (s, 0.5H), 7.71 (d, J = 8.7 Hz, 1H), 7.25-7.18 (m, 4H), 7.11 (m, 1H), 6.81 (s, 1H), 6.68 (d, J = 2.9 Hz, 1H), 5.05-4.73 (m, 3H), 4.44-4.34 (m, 1H), 4.17 (br s, 1H), 3.98-3.85 (m, 2H), 3.56-3.48 (m, 1H), 3.40-3.06 (m, 3H), 2.94-2.80 (m, 2H).

### Example 14

### 6H-Thieno[2,3-b]pyrrole-5-carboxylic acid [(1S)-benzyl-2-((3R,4S)-dihydroxypyrrolidin-1-yl)-2-oxo-ethyl]-amide

6H-Thieno[2,3-b]pyrrole-5-carboxylic acid and (2S)-amino-1-((3R,4S)-dihydroxypyrrolidin-1-yl)-3-phenyl-propan-1-one hydrochloride were coupled according to Procedure A (1.5 equiv. 1-hydroxybenzotriazole hydrate, 1.1 equiv. 1-(3-dimethylamino-propyl)-3-ethylcarbodiimide hydrochloride, 15:1 dichloromethane:dimethylformamide; reaction mixture stirred for 3 d; after saturated aqueous NaHCO₃, combined organic phases washed with water, dried over Na₂SO₄); mp 179-184°C, CIMS m/e 400.1 (MH⁺), 398.2 ((M-H)⁺); ¹H NMR (300 MHz, DMSO-*d*₆) δ 11.79 (br s 1H), 8.52 (d, J = 8.3 Hz, 1H), 7.34-7.16 (m, 6H), 7.04 (m, 2H), 5.04 (d, J = 5.1 Hz, 0.5H), 4.96 (d, J = 4.9 Hz, 0.5H), 4.90-4.80 (m, 2H), 4.09-3.98 (m, 1H), 3.89 (m, 1.5H), 3.49-3.29 (m, 2.5H), 3.19 (m, 1H), 3.08-2.91 (m, 2H).

### Example 15

### 2-Bromo-4H-thieno[3,2-b]pyrrole-5-carboxylic acid [(1S)-benzyl-2-((3R,4S)-dihydroxy-pyrrolidin-1-yl)-2-oxo-ethyl]-amide

2-Bromo-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 25:1 dichloromethane:dimethylformamide; reaction mixture stirred for 3 d; combined organic phases washed with water prior to saturated aqueous NaHCO₃, dried over Na₂SO₄); mp 140-143°C; CIMS m/e 476.1 /478.0 ((M-H)⁺); ¹H NMR (300 MHz, DMSO-*d*₆) δ 11.73 (m, 1H), 8.61 (d, J = 8.3 Hz, 1H), 7.34-7.15 (m, 6H), 5.05-4.84 (m, 3H), 4.15-3.85 (m, 2.5H), 3.48-2.95 (m, 5.5H).

### Example 16

### 2-Methyl-4H-thieno[3,2-b]pyrrole-5-carboxylic acid [(1S)-benzyl-2-((3R,4S)-dihydroxy-pyrrolidin-1-yl)-2-oxo-ethyl]-amide

2-Methyl-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 25:1 dichloromethane:dimethylformamide; reaction mixture stirred for 3 d; combined organic phases washed with water prior to saturated aqueous NaHCO₃, dried over Na₂SO₄); mp 128-130°C; CIMS m/e 412.2 ((M-H)⁺), 414.1 (MH⁺); ¹H NMR (DMSO-*d*₆) δ 11.40 (m, 1H), 8.38 (m, 1H), 7.37-7.05 (m, 6H), 6.65 (s, 1H), 4.97 (d, J = 5.2 Hz, 0.5H), 4.90-4.76 (m, 2.5H), 4.07-3.82 (m, 2.5H), 3.42-3.25 (m, 2 H), 3.13 (m, 1.5H), 3.01-2.87 (m, 2H), 2.44 (d, J = 1Hz, 3H).

### Example 16a

### 2-Methyl-4H-thieno[3,2-b]pyrrole-5-carboxylic acid ethyl ester

5-Methyl-2-thiophenecarboxaldehyde was annulated according to Procedure H (acrylate organic phases dried over Na₂SO₄).
mp 129-130°C; CIMS m/e 208.2 ((M-H)⁺), 210.2 (MH⁺); ¹H NMR (CDCl₃) δ 8.90 (br s, 1H), 7.04 (s, 1H), 6.63 (s, 1H), 4.33 (q, J = 7.1 Hz, 1H), 2.54 (s, 3H), 1.36 (t, J = 7.2 Hz, 3H).

### Example 16b

### 2-Methyl-4H-thieno[3,2-b]pyrrole-5-carboxylic acid

2-Methyl-4H-thieno[3,2-b]pyrrole-5-carboxylic acid ethyl ester was hydrolyzed according to Procedure D (after cooling to room temperature, acidification with 2 N HCI, extracted with ethyl acetate; organic phases dried over Na₂SO₄; no purification). CIMS m/e 180.2 ((M-H)⁺); ¹H NMR (300 MHz, DMSO-*d*₆) δ 12.35 (br s, 1H), 11.72 (s, 1H), 6.95 (s, 1H), 6.73 (s, 1H), 2.51 (s, 3H).

### Example 17

### 2,4-Dichloro-6H-thieno[2,3-b]pyrrole-5-carboxylic acid acid [(1S)-benzyl-2-((3R,4S)-dihydroxy-pyrrolidin-1-yl)-2-oxo-ethyl]-amide

2,4-Dichloro-6H-thieno[2,3-b]pyrrole-5-carboxylic acid and (2S)-amino-1-((3R,4S)-dihydroxy-pyrrolidin-1-yl)-3-phenyl-propan-1-one hydrochloride were coupled according to Procedure A (1.5 equiv 1-hydroxybenzotriazole hydrate, 1.1 equiv 1-(3-dimethylamino-propyl)-3-ethylcarbodiimide hydrochloride, 25:1 dichloromethane:dimethylformamide; 2 d reaction time; combined organic phases washed with water prior to saturated aqueous NaHCO₃, dried over Na₂SO₄).
mp 203-204°C; CIMS m/e 468.1/470.1 (MH⁺); ¹H NMR (DMSO-*d*₆) δ 12.20 (s, 1H), 7.65-7.58 (m, 1H), 7.28-7.08 (m, 6H), 5.04 (d, J = 3.3 Hz, 1H), 4.98-4.80 (m, 3H), 4.08-3.95 (m, 1H), 3.91-3.74 (m, 2H), 3.26-3.10 (m, 2H), 3.10-2.88 (m, 2H).

### Example 18

### 2-Cyano-6H-thieno[2,3-b]pyrrole-5-carboxylic acid [(1S)-benzyl-2-(3-hydroxy-azetidin-1-yl)-2-oxo-ethyl]-amide

2-Cyano-6H-thieno[2,3-b]pyrrole-5-carboxylic acid and (2S)-amino-1-(3-hydroxyazetidin-1-yl)-3-phenyl-propan-1-one hydrochloride were coupled according to Procedure B.
CIMS m/e 395.1 (MH⁺); ¹H NMR (DMSO-*d*₆) δ 12.09 (s, 1H), 8.74 (d, J = 8.5 Hz, 1H), 7.99 (s, 1H), 7.29-7.12 (m, 6H), 5.68 (m, 1H), 4.58 (m, 1H), 4.41 (m, 1H), 4.28 (m, 0.5H), 4.11-3.90 (m, 2H), 3.69 (m, 0.5H), 3.57-3.49 (m, 1H), 3.01-2.88 (m, 2H).

### Example 18a

### 2-Cyano-6H-thieno[2,3-b]pyrrole-5-carboxylic acid

2-Formyl-6H-thieno[2,3-b]pyrrole-5-carboxylic acid (Soth, S. et al., Bull. Soc. Chim. Fr., 2511-2515 (1975)) was treated with hydroxylamine hydrochloride according to Procedure G (100°C for 13 h, 125°C for 7 h; after cooling to room temperature, concentration gave crude product).
CIMS m/e 190.9 ((M-H)⁺); ¹H NMR (DMSO-*d*₆) δ 13.03 (br s, 1H), 12.39 (br s, 1H), 7.97 (s, 1H), 7.04 (s, 1H).

### Example 19

### 2-Chloro-6H-thieno[2,3-b]pyrrole-5-carboxylic acid [(1S)-benzyl-2-morpholin-4-yl-2-oxo-ethyl]-amide

2-Chloro-6H-thieno[2,3-b]pyrrole-5-carboxylic acid and (2S)-amino-1-morpholin-4-yl-3-phenyl-propan-1-one hydrochloride (See, for example, Suzuki, K.; Fujita, H.; Sasaki, Y.; Shiratori, M.; Sakurada, S.; Kisara, K,. Chem. Pharm. Bull, 36, 4834-40 (1988)) were coupled according to Procedure C (solution of product in ethyl acetate washed with water, dried over MgSO₄, concentrated).
mp 108-110°C; CIMS m/e 416.3 /418.2 ((M-H)⁺); ¹H NMR (DMSO-*d*₆) δ 11.84 (m, 1H), 8.65 (d, J = 8.2 Hz, 1H) 7.39-7.13 (m, 7H), 5.08 (q, J = 7.6 Hz, 1H), 3.60-3.30 (m, 7H), 3.23 (m, 1H), 3.09-2.95 (m, 2H).

### Example 20

### 2-Chloro-6H-thieno[2,3-b]pyrrole-5-carboxylic acid [(1S)-dimethylcarbamoyl-2-phenyl-ethyl]-amide

2-Chloro-6H-thieno[2,3-b]pyrrole-5-carboxylic acid and (2S)-amino-*N,N*-dimethyl-3-phenyl-propionamide trifluoroacetate (See, for example, Holladay, M. et al., J. Med. Chem., 37: 630-5 (1994)) were coupled according to Procedure C (product washed with ethyl acetate).
mp 234-235°C; CIMS m/e 374.2/376.2 ((M-H)⁺); ¹H NMR (DMSO-*d*₆) δ 11.72 (s, 1H), 8.53 (d, J = 8.1 Hz, 1H), 7.23 (m, 4H), 7.13 (m, 3H), 5.01 (m, 1H), 3.01-2.88 (m, 5H), 2.78 (s, 3H).

### Example 21

### 2-Chloro-6H-thieno[2,3-b]pyrrole-5-carboxylic acid [(1S)-benzyl-2-(1,1-dioxo-1-thiazolidin-3-yl)-2-oxo-ethyl]-amide

2-Chloro-6H-thieno[2,3-b]pyrrole-5-carboxylic acid and (2S)-amino-1-(1,1-dioxo-1-thiazolidin-3-yl)-3-phenyl-propan-1-one hydrochloride (WO96/39384, Example 40a) were coupled according to Procedure C (solution of product in ethyl acetate washed with water, dried over MgSO₄, concentrated).
mp 125-129°C; CIMS m/e 450.2/452.2 ((M-H)⁺); ¹H NMR (300 MHz, DMSO-*d*₆) δ 11.80 (m 1H), 8.75 (dd, J = 8.1, 12.9, 1H), 7.36 (m, 2H), 7.26-7.14 (m, 5H), 5.08-4.97 (m, 1H), 4.81 (m, 0.5H), 4.63 (d, J = 11.4, 0.5H), 4.55 (d, J = 12.5, 0.5H), 4.45 (d, J = 12.4, 0.5H), 4.25 (m, 1H), 3.90-3.75 (m, 1H), 3.55-3.35 (m, 2H), 3.05 (m, 2H).

### Example 22

### 1-{(2S)-[(2-Chloro-6H-thieno[2,3-b]pyrrole-5-carbonyl)-amino]-3-phenyl-propiony}-piperidine-4-carboxylic acid ethyl ester

2-Chloro-6H-thieno[2,3-b]pyrrole-5-carboxylic acid and 1-[(2S)-amino-3-phenylpropionyl]-piperidine-4-carboxylic acid ethyl ester hydrochloride were coupled according to Procedure C (solution of product in ethyl acetate washed with water, dried over MgSO₄, concentrated).
mp 104-105°C; CIMS m/e 486.2/488.2 ((M-H)⁺); ¹H NMR (300 MHz, DMSO-*d*₆) δ 11.84 (m, 1H), 8.64 (t, J = 8.8 Hz, 1H), 7.32-7.14 (m, 7H), 5.10 (m, 1H), 4.30-3.89 (m, 4H), 3.15-2.92 (m, 3H), 2.80-2.50 (m, 2H), 1.83-1.69 (m, 2H), 1.52-0.94 (m, 5H).

### Example 22a

### 1-((2S)-tert-Butoxycarbonylamino-3-phenyl-propionyl)-piperidine-4-carboxylic acid ethyl ester

Boc-L-Phenylalanine (1.1 equiv) and piperidine-4-carboxylic acid ethyl ester were coupled according to Procedure A (1.5 equiv 1-hydroxybenzotriazole hydrate, 1.3 equiv 1-(3-dimethylamino-propyl)-3-ethylcarbodiimide hydrochloride, room temperature, dichloromethane; reaction mixture poured into water, acidified with 1 N HCI; resultant precipitate filtered, filtrate extracted with CHCl₃; organic phase washed sequentially with water and brine, dried over MgSO₄ before concentration).
CIMS m/e 405.2 (MH⁺); ¹H NMR (CDCl₃) δ 7.26-7.14 (m, 5H), 5.40 (dd, J = 8.9, 19.3 Hz, 1H), 4.82 (m, 1H), 4.34-4.24 (m, 1H), 4.09 (dq, J = 2.0, 7.1 Hz, 2H), 3.57 (m, 1H), 2.99-2.88 (m, 2.5H), 2.72 (m, 1H), 2.45-2.32 (m, 1.5H), 1.95-1.79 (m, 1.5H), 1.58 (m, 2H), 1.40 (d, J = 2.1 Hz, 9H), 1.23 (m, 3H), 0.68 (m, 0.5H).

### Example 22b

### 1-((2S)-Amino-3-phenyl-propionyl)-piperidine-4-carboxylic acid ethyl ester hydrochloride

To a solution of 1-((2S)-tert-butoxycarbonylamino-3-phenyl-propionyl)-piperidine-4-carboxylic acid ethyl ester (11 g, 27.20 mmol) in ethyl acetate (150 ml) was bubbled in HCI gas over 10 min. The reaction mixture was stirred overnight, concentrated, redissolved in ethyl acetate and ether, and concentrated. The crude product was precipitated with hexanes, filtered, and dried *in vacuo* to give the title product (9.1 mg, 98%).
CIMS m/e 305.1 (MH⁺); ¹H NMR (CDCl₃) δ 8.56 (br s, 2H), 7.29-7.18 (m, 5H), 4.94-4.82 (m, 1H), 4.22-3.97 (m, 4H), 3.53 (dt, J = 4.5, 12.7 Hz, 1H), 3.41-3.27 (m, 1H), 3.12 (m, 1H), 2.95 (m, 0.5H), 2.76 (t, J = 10.9 Hz, 0.5H), 2.66 (m, 0.5H), 2.27 (m, 1H), 2.07 (m, 0.5H), 1.79-1.51 (m, 2H), 1.38-1.11 (m, 3.5H), 0.41 (m, 0.5H).

### Example 23

### 2-Bromo-6H-thieno[2,3-b]pyrrole-5-carboxylic acid [(1S)-benzyl-2-(3-hydroxy-azetidin-1-yl)-2-oxo-ethyl]-amide

2-Bromo-6H-thieno[2,3-b]pyrrole-5-carboxylic acid and (2S)-amino-1-(3-hydroxyazetidin-1-yl)-3-phenyl-propan-1-one hydrochloride were coupled according to Procedure B.
CIMS m/e 448.1/450.0 (MH⁺); 1H NMR (DMSO-*d*₆) δ 11.77 (s, 1H), 8.55 (d, J = 8.1 Hz, 1H), 7.26-7.10 (m, 7H), 5.00-4.76 (m, 3H), 4.07-3.94 (m, 1.5H), 3.83 (m, 1.5H), 3.40-3.22 (m, 1H), 3.13 (m, 2H), 2.93 (m, 2H).

### Example 24

### 2-Methyl-4H-furo[3,2-b]pyrrole-5-carboxylic acid [(1S)-benzyl-2-((3R,4S)-dihydroxypyrrolidin-1-yl)-2-oxo-ethyl]-amide

2-Methyl-4H-furo[3,2-b]pyrrole-5-carboxylic acid (Krutosikova, A.; Kovac, J.; Dandarova, M.; Lesko, J.; Ferik, S., Collect. Czech. Chem. Comm., 46: 2564-2573 (1981)) and (2S)-amino-1-((3R,4S)-dihydroxy-pyrrolidin-1-yl)-3-phenyl-propan-1-one hydrochloride were coupled according to Procedure B (acidic aqueous phase extracted with ethyl acetate; organic phases combined prior to basic work-up).
CIMS m/e 396.3 ((M-H)⁺); ¹H NMR (DMSO-*d*₆) δ 10.96 (m, 1H), 8.23 (m, 1H), 7.27-7.19 (m, 4H), 7.14 (m, 1H), 6.83 (d, J = 5.4 Hz, 1H), 6.15 (s, 1H), 4.97 (d, J = 5.2 Hz, 0.5H), 4.89 (d, J = 4.6 Hz, 0.5H), 4.80 (m, 2H), 3.99 (m, 0.5H), 3.93 (m, 0.5H), 3.82 (m, 1.5H), 3.38 (m, 1H), 3.25 (m, 1H), 3.13 (m, 1.5H), 3.00-2.85 (m, 2H), 2.31 (s, 3H).

### Example 25

### 2-Trimethylsilanylethynyl-6H-thieno[2,3-b]pyrrole-5-carboxylic acid [(1S)-benzyl-2-(3-hydroxy-azetidin-1-yl)-2-oxo-ethyl]-amide

Using a modified procedure of J. M. Tour et al. (J. Org. Chem., 61: 6906-6921 (1996)), to a degassed solution of 2-bromo-6H-thieno[2,3-b]pyrrole-5-carboxylic acid [(1S)-benzyl-2-(3-hydroxy-azetidin-1-yl)-2-oxo-ethyl]-amide (106 mg, 0.24 mmol) in tetrahydrofuran (5 ml) was sequentially added diisopropylamine (36 µl, 0.26 mmol), a mixture of copper(l) iodide (9 mg, 0.05 mmol) and dichlorobis(triphenylphosphine)palladium(ll) (68 mg, 0.1 mmol), and (trimethylsilyl)acetylene (41 µl, 0.29 mmol). The mixture was stirred overnight, poured into water, and extracted with dichloromethane. The combined organic phases were washed with saturated NaCl, dried over MgSO₄, and concentrated. The product was purified by Chromatotron-chromatography (dichloromethane; 20:1 dichloromethane:methanol) to give the title product (4.5 mg, 4%).
CIMS m/e 464.3 ((M-H)⁺); ¹H NMR (DMSO-*d*₆) δ 11.86 (s, 1H), 8.54 (t, J = 8.9 Hz, 1H), 7.31 (s, 1H), 7.23 (m, 4H), 7.13 (m, 2H), 5.66 (m, 1H), 4.56 (m, 1H), 4.41 (m, 1H), 4.28 (m, 0.5H), 4.11-3.89 (m, 2H), 3.66 (m, 0.5H), 3.57-3.46 (m, 1H), 2.99-2.85 (m, 2H), 0.23 (s, 9H).

### Example 26

### 2-Ethynyl-6H-thieno[2,3-b]pyrrole-5-carboxylic acid [(1S)-benzyl-2-(3-hydroxyazetidin-1-yl)-2-oxo-ethyl]-amide

Using a procedure analogous to that of G. M. Whitesides et al. (J. Org. Chem.,53: 2489-2496 (1988)), to a solution of 2-trimethylsilanylethynyl-6H-thieno[2,3-b]pyrrole-5-carboxylic acid [(1S)-benzyl-2-(3-hydroxy-azetidin-1-yl)-2-oxo-ethyl]-amide (110 mg, 0.02 mmol) in methanol (0.5 ml) was added a 5% aqueous solution of potassium hydroxide (7 µL, 0.06 mmol). The reaction mixture was stirred for 3 h, concentrated to remove the methanol, diluted with water, and extracted with dichloromethane. The organic phase dried over MgSO₄ and concentrated to give the title product (7 mg, 77%).
CIMS m/e 392.1 ((M-H)⁺); ¹H NMR (CDCl₃) δ 7.32-7.16 (m, 7H), 7.03 (m, 2H), 4.66 (m, 1H), 4.47 (m, 1H), 4.17 (m, 1H), 4.00 (m, 1H), 3.75-3.56 (m, 3H), 3.35 (m, 1H), 3.04 (m, 2H).

### Example 27

### 2-Fluoro-4H-thieno[3,2-b]pyrrole-5-carboxylic acid [(1S)-benzyl-2-((3R,4S)-dihydroxypyrrolidin-1-yl)-2-oxo-ethyl]-amide

2-Fluoro-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (4 d reaction time).
mp 128-132°C; CIMS m/e 416.1 ((M-H)⁺), 418.2 (MH⁺); ¹H NMR (DMSO-*d*₆) δ 11.72 (m, 1H), 8.49 (d, J = 8.5 Hz, 1H), 7.28-7.13 (m, 6H), 6.71 (s, 1H), 4.99 (d, J = 5.2 Hz, 0.5H), 4.91 (d, J = 4.8 Hz, 0.5H), 4.86 (d, J = 3.7 Hz, 1H), 4.79 (m, 1H), 4.01 (m, 0.5H), 3.94 (m, 0.5H), 3.83 (m, 1.5H), 3.42-3.24 (m, 2.5H), 3.14 (m, 1H), 3.01-2.84 (m, 2H).

### Example 27a

### 2-Fluoro-4H-thieno[3,2-b]pyrrole-5-carboxylic acid ethyl ester

5-Fluoro-thiophene-2-carbaldehyde (see, for example, Schuetz, R. D. and Nilles, G. P., J. Org. Chem., 36: 2188-90 (1971)) was annulated according to Procedure H (aldehyde and azido-acetic acid ethyl ester added as ethanol solution (0.6 M of ester); acrylate organic phase washed with saturated aqueous NaCI prior to drying; acrylate not purified).
CIMS m/e 212.1 ((M-H)⁺); ¹H NMR (CDCl₃) δ 9.16 (br s, 1H), 7.03 (s, 1H), 6.51 (s, 1H), 4.33 (q, J = 7.2 Hz, 2H), 1.36 (t, J = 7.2 Hz, 3H).

### Example 27b

### 2-Fluoro-4H-thieno[3,2-b]pyrrole-5-carboxylic acid

2-Fluoro-4H-thieno[3,2-b]pyrrole-5-carboxylic acid ethyl ester was hydrolyzed according to Procedure F (acidified aqueous phase extracted with ethyl acetate; combined organic phases dried over MgSO₄, concentrated).
CIMS m/e 184.1 ((M-H)⁺); ¹H NMR (DMSO-*d*₆) δ 12.47 (br s, 1H), 12.03 (s, 1H), 6.96 (s, 1H), 6.73 (s, 1H).

### Example 28

### 2-Cyano-4H-furo[3,2-b]pyrrole-5-carboxylic acid [(1S)-benzyl-2-(3-hydroxy-azetidin-1-yl)-2-oxo-ethyl]-amide

2-Cyano-4H-furo[3,2-b]pyrrole-5-carboxylic acid and (2S)-amino-1-(3-hydroxyazetidin-1-yl)-3-phenyl-propan-1-one hydrochloride were coupled according to Procedure B (reaction mixture partitioned between ethyl acetate and water prior to acidic washing).
CIMS m/e 377.1 ((M-H)⁺), 379.1 (MH⁺); ¹H NMR (DMSO-*d*₆) δ 11.78 (s, 1H), 8.68 (t, J = 8.2 Hz, 1H), 7.67 (s, 1H), 7.22 (m, 4H), 7.15 (m, 1H), 7.01 (d, J = 3.1 Hz, 1H), 5.68 (m, 1H), 4.59 (m, 1H), 4.40 (m, 1H), 4.26 (m, 0.5H), 4.05 (m, 1H), 3.92 (m, 1H), 3.65 (m, 0.5H), 3.53 (m, 1H), 3.00-2.88 (m, 2H).

### Example 28a

### 2-Cyano-4H-furo[3,2-b]pyrrole-5-carboxylic acid

2-Formyl-4H-furo[3,2-b]pyrrole-5-carboxylic acid (see, for example, Krutosikova, A.; Dandarova, M.; Alfoldi, J., Chem. Pap., 48: 268-73 (1994)) was treated with hydroxylamine hydrochloride according to Procedure G.
CIMS m/e 174.9 ((M-H)⁺); ¹H NMR (DMSO-*d*₆) δ 13.10-12.60 (br s, 1H), 12.05 (s, 1H), 7.73 (s, 1H), 6.75 (s, 1H).

### Example 29

### 2-Chloro-4H-furo[3,2-b]pyrrole-5-carboxylic acid [(1S)-benzyl-2-((3R,4S)-dihydroxypyrrolidin-1-yl)-2-oxo-ethyl]-amide

2-Chloro-4H-furo[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (3 d reaction time; reaction mixture partitioned between ethyl acetate and water prior to acidic washing).
CIMS m/e 418.1/420.1 (MH⁺); ¹H NMR (DMSO-*d*₆) δ 11.36 (s, 1H), 8.42 (dd, J = 2.9, 8.3 Hz, 1H), 7.27-7.10 (m, 5H), 6.94 (d, J = 6.0 Hz, 1H), 6.63 (m, 1H), 4.99 (d, J = 5.2 Hz, 0.5H), 4.91 (d, J = 5.0 Hz, 0.5H), 4.86-4.77 (m, 2H), 4.00 (m, 0.5H), 3.94 (m, 0.5H), 3.81 (m, 1.5H), 3.43-3.21 (m, 2.5H), 3.13 (m, 1H), 3.00-2.85 (m, 2H).

### Example 29a

### 2-Chloro-4H-furo[3,2-b]pyrrole-5-carboxylic acid ethyl ester

5-Chloro-furan-2-carbaldehyde (Snyder, H. R., Jr.; Seehausen, P. H., J. Heterocycl. Chem., 10: 385-6 (1973)) was annulated according to Procedure H (8 equiv sodium; aldehyde and azido-acetic acid ethyl ester added as ethanol solution (0.9 M of ester); condensation reaction mixture allowed to warm to room temperature, stirred for 1 h, quenched at -40°C, diluted with water, and extracted with ether; acrylate not purified; crude furanopyrrole filtered before concentration).
CIMS m/e 212.0/214.1 ((M-H)⁺); ¹H NMR (CDCl₃) δ 8.69 (br s, 1H), 6.74 (dd, J = 0.8, 1.7 Hz, 1H), 6.31 (d, J = 0.6 Hz, 1H), 4.33 (q, J = 7.1Hz, 2H), 1.36 (t, J = 7.1 Hz, 3H).

### Example 29b

### 2-Chloro-4H-furo[3,2-b]pyrrole-5-carboxylic acid

2-Chloro-4H-furo[3,2-b]pyrrole-5-carboxylic acid ethyl ester was hydrolyzed according to Procedure F (room temperature overnight, 50°C 4 h; acidified aqueous phase extracted with ethyl acetate; combined organic phases dried over MgSO₄, concentrated).
CIMS m/e 183.8/185.8 ((M-H)⁺); ¹H NMR (DMSO-*d*₆) δ 12.47 (br s, 1H), 11.70 (s, 1H), 6.70 (s, 1H), 6.67 (s, 1H).

### Example 30

### 2-Chloro-4H-furo[3,2-b]pyrrole-5-carboxylic acid [(1S)-benzyl-3-((3R,4S)-dihydroxypyrrolidin-1-yl)-(2R)-hydroxy-3-oxo-propyl]-amide

2-Chloro-4H-furo[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure B (3 d reaction time; reaction mixture partitioned between ethyl acetate and water prior to acidic washing).
CIMS m/e 448.1/450.1 (MH⁺); ¹H NMR (DMSO-*d*₆) δ 11.33 (m, 1H), 7.72 (d, J = 8.7 Hz, 1H), 7.25-7.09 (m, 5H), 6.82 (s, 1H), 6.61 (dd, J = 0.7, 3.0 Hz, 1H), 5.04 (d, J = 7.3 Hz, 0.5H), 4.94 (m, 1H), 4.89 (d, J = 5.0 Hz, 0.5H), 4.80 (d, J = 7.5 Hz, 0.5H), 4.75 (d, J = 4.2 Hz, 0.5H), 4.45-4.35 (m, 1H), 4.18 (m, 1H), 4.00-3.88 (m, 2H), 3.57-3.49 (m, 1H), 3.39 (m, 0.5H), 3.26-3.06 (m, 2.5H), 2.95-2.80 (m, 2H).

### Example 31

### 1-{(2S)-[(2-Chloro-6H-thieno[2,3-b]pyrrole-5-carbonyl)-amino]-3-phenyl-propionyl}-piperidine-4-carboxylic acid

1-{(2S)-[(2-Chloro-6H-thieno[2,3-b]pyrrole-5-carbonyl)-amino]-3-phenyl-propionyl}-piperidine-4-carboxylic acid ethyl ester was hydrolyzed according to Procedure F (room temperature; after concentration, reaction residue partitioned between ethyl acetate and 1-2 N NaOH; aqueous phase acidified with 2 N HCI, extracted with ethyl acetate; combined organic phases dried over MgSO₄, concentrated; crude product washed with ether); mp 145-150°C; ¹H NMR (DMSO-*d*₆) δ 12.21 (s, 1H), 11.83 (s, 0.5H), 11.77 (s, 0.5H), 8.58 (m, 1H), 7.26-7.11 (m, 7H), 5.05 (m, 1H), 4.23 (d, J = 13.3 Hz, 0.5H), 4.10 (d, J = 12.5 Hz, 0.5H), 3.93 (d, J = 12.7 Hz, 0.5H), 3.85 (d, J = 13.5 Hz, 0.5H), 3.11-2.90 (m, 3H), 2.77-2.61 (m, 1H), 2.49-2.39 (m, 1H), 1.75-1.65 (m, 2H), 1.43-1.17 (m, 1.5H), 1.07-0.97 (m, 0.5H).

### Example 32

### 3-Chloro-4H-thieno[3,2-b]pyrrole-5-carboxylic acid [(1S)-benzyl-2-((3R,4S)-dihydroxypyrrolidin-1-yl)-2-oxo-ethyl]-amide

3-Chloro-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (reaction mixture partitioned between ethyl acetate and water prior to acidic washing).
CIMS m/e 434.0/436.0 (MH⁺); ¹H NMR (DMSO-*d*₆) δ 12.09 (m, 1H), 8.57 (d, J = 8.3 Hz, 1H), 7.40 (m, 0.5H), 7.28-7.10 (m, 6.5H), 5.00 (d, J = 5.2 Hz, 0.5H), 4.92 (d, J = 5.0 Hz, 0.5H), 4.84 (m, 2H), 4.10-3.93 (m, 1H), 3.82 (m, 1.5H), 3.44-3.23 (m, 2.5H), 3.13 (m, 1H), 3.03-2.87 (m, 2H).

### Example 32a

### 3-Chloro-4H-thieno[3,2-b]pyrrole-5-carboxylic acid ethyl ester

4-Chloro-thiophene-2-carbaldehyde (Iriarte, J.; Martinez, E.; Muchowski, J. M., J. Heterocycl. Chem., 13: 393-4 (1976)) was annulated according to Procedure H (aldehyde and azido-acetic acid ethyl ester added as ethanol solution (1.2 M of ester) such that reaction temperature maintained at 0°C; reaction mixture allowed to warm to 10°C, stirred for 1.5 h, poured into cold saturated aqueous NH₄Cl; after ether extractions, combined acrylate organic phases washed with water until aqueous phase was neutral; acrylate not purified).
CIMS m/e 228.0 ((M-H)⁺); ¹H NMR (CDCl₃) δ 9.02 (br s, 1H), 7.24 (s, 1H), 7.10 (s, 1H), 4.37 (q, J = 7.1 Hz, 2H), 1.38 (t, J = 7.1 Hz, 3H).

### Example 32b

### 3-Chloro-4H-thieno[3,2-b]pyrrole-5-carboxylic acid

3-Chloro-4H-thieno[3,2-b]pyrrole-5-carboxylic acid ethyl ester was hydrolyzed according to Procedure F (7 equiv LiOH-H₂O; room temperature overnight, then at 50°C overnight again; acidified aqueous phase extracted with ethyl acetate; combined organic phases dried over MgSO₄, concentrated).
CIMS m/e 199.9/201.8 ((M-H)⁺); ¹H NMR (DMSO-*d*₆) δ 12.71 (br s, 1H), 12.40 (s, 1H), 7.48 (s, 1H), 7.06 (d, J = 1.9 Hz, 1H).

### Example 33

### 3-Chloro-4H-thieno[3,2-b]pyrrole-5-carboxylic acid [(1S)-benzyl-3-((3R,4S)-dihydroxypyrrolidin-1-yl)-(2R)-hydroxy-3-oxo-propyl]-amide

3-Chloro-4H-thieno[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure B (reaction mixture partitioned between ethyl acetate and water prior to acidic washing).
CIMS m/e 464.0/466.0 (MH⁺); ¹H NMR (DMSO-*d*₆) δ 12.4 (m, 1H), 7.89 (d, J = 8.9 Hz, 1H), 7.38 (s, 0.5H), 7.26-7.10 (m, 5.5H), 7.05 (d, J = 3.1 Hz, 1H), 5.08 (d, J = 7.1 Hz, 0.5H), 4.97-4.84 (m, 2H), 4.76 (d, J = 4.2 Hz, 0.5H), 4.44 (m, 1H), 4.20 (m, 1H), 4.09-3.88 (m, 2H), 3.53 (m, 1H), 3.38 (m, 0.5H), 3.25-3.06 (m, 2.5H), 2.97-2.82 (m, 2H).

### Example 34

### 3-Bromo-4H-thieno[3,2-b]pyrrole-5-carboxylic acid [(1S)-benzyl-2-((3R,4S)-dihydroxypyrrolidin-1-yl)-2-oxo-ethyl]-amide

3-Bromo-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (reaction mixture partitioned between ethyl acetate and water prior to acidic washing).
CIMS m/e 475.9/478.2 ((M-H)⁺); ¹H NMR (DMSO-*d*₆) δ 11.99 (m, 1H), 8.56 (d, J = 8.3 Hz, 1H), 7.48 (d, J = 1.2 Hz, 0.5H), 7.27-7.12 (m, 6.5H), 4.99 (d, J = 5.2 Hz, 0.5H), 4.91 (d, J = 5.2 Hz, 0.5H), 4.84 (m, 2H), 4.09-3.92 (m, 1.5H), 3.78 (m, 1.5H), 3.43-3.22 (m, 2H), 3.13 (m, 1H), 2.99-2.85 (m, 2H).

### Example 34a

### 3-Bromo-4H-thieno[3,2-b]pyrrole-5-carboxylic acid

4-Bromo-thiophene-2-carbaldehyde was annulated according to Procedure H (aldehyde and azido-acetic acid ethyl ester added as ethanol solution (1.2 M of ester) such that reaction temperature maintained at 0°C; reaction mixture allowed to warm to 10°C, stirred for 1 h, poured into cold saturated aqueous NH₄Cl; after ether extractions, combined acrylate organic phases washed with water until aqueous phase was neutral; acrylate not purified).
CIMS m/e 272.0/273.9 ((M-H)⁺); ¹H NMR (CDCl₃) δ 8.99 (br s, 1H), 7.21 (s, 1H), 7.13 (d, J = 1.9 Hz, 1 H), 4.37 (q, J = 7.2 Hz, 2H), 1.38 (t, J = 7.2 Hz, 3H).

### Example 34b

### 3-Bromo-4H-thieno[3,2-b]pyrrole-5-carboxylic acid

3-Bromo-4H-thieno[3,2-b]pyrrole-5-carboxylic acid ethyl ester was hydrolyzed according to Procedure F (7 equiv LiOH-H₂O; room temperature overnight, then at 50°C overnight again; acidified aqueous phase extracted with ethyl acetate; combined organic phases dried over MgSO₄, concentrated).
CIMS m/e 243.9/245.9 ((M-H)⁺); ¹H NMR (DMSO-*d*₆ δ 12.69 (br s, 1H), 12.33 (s, 1H), 7.56 (s, 1H), 7.08 (s, 1H).

### Example 35

### 3-Bromo-4H-thieno[3,2-b]pyrrole-5-carboxylic acid [(1S)-benzyl-3-((3R,4S)-dihydroxypyrrolidin-1-yl)-(2R)-hydroxy-3-oxo-propyl]-amide

3-Bromo-4H-thieno[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure B (reaction mixture partitioned between ethyl acetate and water prior to acidic washing).
CIMS m/e 508.0/510.0 (MH⁺); ¹H NMR (DMSO-*d*₆) δ 11.96 (s, 0.5H), 11.91 (s, 0.5H), 7.90 (d, J = 9.3 Hz, 1H), 7.46 (s, 0.5H), 7.22 (m, 4.5H), 7.12 (m, 1H), 7.04 (m, 1H), 5.08 (d, J = 6.9 Hz, 0.5H), 4.94 (m, 1H), 4.89 (d, J = 5.0 Hz, 0.5H), 4.85 (d, J = 7.1 Hz, 0.5H), 4.75 (d, J = 4.4 Hz, 0.5H), 4.45 (m, 1H), 4.20 (m, 1H), 4.08-3.87 (m, 2H), 3.53 (m, 1H), 3.40-3.30 (m, 0.5H), 3.22 (m, 1H), 3.15-3.05 (m, 1.5H), 2.98-2.82 (m, 2H).

### Example 36

### 2-Chloro-4H-thieno[3,2-b]pyrrole-5-carboxylic acid [(1S)-benzyl-3-((3R,4S)-dihydroxypyrrolidin-1-yl)-(2R)-hydroxy-3-oxo-propyl]-amide

2-Chloro-4H-thieno[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure B (reaction mixture partitioned between ethyl acetate and water prior to acidic washing).
CIMS m/e 464.0/466.0 (MH⁺); ¹H NMR (DMSO-*d*₆) δ 11.72 (s, 0.5H), 11.67 (s, 0.5H), 7.85 (d, J = 9.1 Hz, 1H), 7.21 (m, 4H), 7.12 (m, 1H), 7.00 (m, 2H), 5.05 (d, J = 7.1 Hz, 0.5H), 4.95 (m, 1H), 4.89 (d, J = 4.8 Hz, 0.5H), 4.81 (d, J = 7.5 Hz, 0.5H), 4.75 (d, J = 3.9 Hz, 0.5H), 4.41 (m, 1H), 4.20 (m, 1H), 4.00-3.87 (m, 2H), 3.54 (m, 1H), 3.40 (m 0.5H), 3.22 (m, 1.5H), 3.15-3.06 (m, 1H), 2.94-2.80 (m, 2H).

### Example 36a

### 2-Chloro-4H-thieno[3,2-b]pyrrole-5-carboxylic acid ethyl ester

5-Chloro-thiophene-2-carbaldehyde was annulated according to Procedure H (aldehyde and azido-acetic acid ethyl ester added as ethanol solution (1.2 M of ester) such that reaction temperature maintained at 0-5°C; reaction mixture allowed to warm to room temperature, stirred for 2 h, poured into cold saturated aqueous NH₄Cl; after ether extractions, combined acrylate organic phases washed with water until aqueous phase was neutral; 0.5 M solution of crude acrylate heated for 1.5 h).
CIMS m/e 228.0/229.9 ((M-H)⁺); ¹H NMR (CDCl₃) δ 9.04 (br s, 1H), 7.02 (m, 1H), 6.88 (m, 1H), 4.34 (q, J = 7.2 Hz, 2H), 1.37 (t, J = 7.2Hz, 3H).

### Example 36b

### 2-Chloro-4H-thieno[3,2-b]pyrrole-5-carboxylic acid

2-Chloro-4H-thieno[3,2-b]pyrrole-5-carboxylic acid ethyl ester was hydrolyzed according to Procedure F (50°C 9 h).
CIMS m/e 199.9/201.8 ((M-H)⁺); ¹H NMR (DMSO-*d*₆) δ 12.62 (s, 1H), 12.04 (s, 1H), 7.05 (s, 1H), 6.97 (s, 1H).

### Example 37

### 2-Chloro-4H-thieno[3,2-b]pyrrole-5-carboxylic acid [(1S)-benzyl-2-((3R,4S)-dihydroxypyrrolidin-1-yl)-2-oxo-ethyl]-amide

2-Chloro-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (reaction mixture partitioned between ethyl acetate and water prior to acidic washing).
CIMS m/e 434.0/436.0 (MH⁺); ¹H NMR (DMSO-*d*₆) δ 11.73 (m, 1H), 8.57 (d, J = 7.9 Hz, 1H), 7.28-7.19 (m, 4H), 7.13 (m, 2H), 7.01 (d, J = 2.7 Hz, 1H), 5.00 (d, J = 5.2 Hz, 0.5H), 4.92 (d, J = 5.2Hz, 0.5H), 4.86 (m, 1H), 4.79 (m, 1H), 4.08-3.94 (m, 1H), 3.82 (m, 1.5H), 3.42-3.24 (m, 2H), 3.14 (m, 1.5H), 3.01-2.87 (m, 2H).

### Example 38

### 3-Methyl-4H-thieno[3,2-b]pyrrole-5-carboxylic acid [(1S)-benzyl-2-((3R,4S)-dihydroxypyrrolidin-1-yl)-2-oxo-ethyl]-amide

3-Methyl-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (reaction mixture partitioned between ethyl acetate and water prior to acidic washing).
CIMS m/e 412.1 ((M-H)⁺), 414.0 (MH⁺); ¹H NMR (DMSO-*d*₆) δ 11.69 (m, 1H), 8.45 (m, 1H), 7.28-7.18 (m, 4H), 7.12 (m, 2H), 6.93 (d, J = 1.0 Hz, 1H), 4.98 (d, J = 5.2 Hz, 0.5H), 4.90 (d, J = 5.0 Hz, 0.5H), 4.83 (m, 2H), 4.03-3.92 (m, 1H), 3.83 (m, 1.5H), 3.42-3.23 (m, 2.5H), 3.14 (m, 1H), 3.03-2.88 (m, 2H), 2.24 (s, 3H).

### Example 38a

### 3-Methyl-4H-thieno[3,2-b]pyrrole-5-carboxylic acid ethyl ester

4-Methyl-thiophene-2-carbaldehyde (Detty, M. R.; Hays, D. S., Heterocycles, 40: 925-37 (1995)) was annulated according to Procedure H (aldehyde and azido-acetic acid ethyl ester added as ethanol solution (1.1 M of ester); reaction poured into cold saturated aqueous NH₄Cl; after ether extractions, acrylate organic phase washed with water until aqueous phase was neutral; acrylate not purified). CIMS m/e 207.9 ((M-H)⁺), 209.9 (MH⁺); ¹H NMR (CDCl₃) δ 9.02 (br s, 1H), 7.09 (d, J = 1.9 Hz, 1H), 6.91 (d, J = 1.0 Hz, 1H), 4.35 (quart, J = 7.3 Hz, 1H), 2.32 (d, J = 1.2 Hz, 3H), 1.38 (t, J = 7.2 Hz, 3H).

### Example 38b

### 3-Methyl-4H-thieno[3,2-b]pyrrole-5-carboxylic acid

3-Methyl-4H-thieno[3,2-b]pyrrole-5-carboxylic acid ethyl ester was hydrolyzed according to Procedure F (50°C 13 h; acidified aqueous phase extracted with ethyl acetate; combined organic phases dried over MgSO₄, concentrated).
CIMS m/e 179.9 ((M-H)⁺), 181.8 (MH⁺); ¹H NMR (DMSO-*d*₆) δ 12.45 (br s, 1H), 11.99 (s, 1H), 7.02 (m, 1H), 6.96 (m, 1H), 2.25 (s, 3H).

### Example 39

### 3-Methyl-4H-thieno[3,2-b]pyrrole-5-carboxylic acid [(1S)-benzyl-3-((3R,4S)-dihydroxypyrrolidin-1-yl)-(2R)-hydroxy-3-oxo-propyl]-amide

3-Methyl-4H-thieno[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure B (reaction mixture partitioned between ethyl acetate and water prior to acidic washing).
CIMS m/e 442.1 ((M-H)⁺), 444.0 (MH⁺); ¹H NMR (DMSO-*d*₆) δ 11.66 (s, 0.5H), 11.62 (s, 0.5H), 7.76 (d, J = 8.9 Hz, 1H), 7.22 (m, 4H), 7.11 (m, 1H), 7.01 (d, J = 1.7 Hz, 1H), 6.91 (s, 1H), 5.06 (d, J = 7.3 Hz, 0.5H), 4.95 (m, 1H), 4.90 (d, J = 5.0 Hz, 0.5H), 4.82 (d, J = 7.5 Hz, 0.5H), 4.77 (d, J = 4.4 Hz, 0.5H), 4.44 (m, 1H), 4.21 (m, 1H), 4.01-3.87 (m, 2H), 3.55 (m, 1H), 3.40 (dd, J = 5.0, 12.6 Hz, 0.5H), 3.24 (m, 1.5H), 3.16-3.06 (m, 1H), 2.97-2.83 (m, 2H), 2.23 (s, 3H).

### Example 40

### 2-Cyano-4H-thieno[3,2-b]pyrrole-5-carboxylic acid [(1S)-benzyl-2-((3R,4S)-dihydroxy-pyrrolidin-1-yl)-2-oxo-ethyl]-amide

2-Cyano-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (reaction mixture partitioned between ethyl acetate and water prior to acidic washing).
CIMS m/e 423.1 ((M-H)⁺), 425.1 (MH⁺); ¹H NMR (DMSO-*d*₆) δ 12.15 (m, 1H), 8.85 (d, J = 8.3 Hz, 1H), 7.79 (m, 1H), 7.29-7.11 (m, 6H), 5.00 (m, 0.5H), 4.93-4.78 (m, 2.5H), 4.04-3.93 (m, 1H), 3.81 (m, 1.5H), 3.43-3.25 (m, 2.5H), 3.15 (m, 1H), 3.03-2.89 (m, 2H).

### Example 40a

### 2-Formyl-4H-thieno[3,2-b]pyrrole-5-carboxylic acid

2-Formyl-4H-thieno[3,2-b]pyrrole-5-carboxylic acid ethyl ester (see, for example, Gale, W. W. et al., J. Org. Chem., 29 2160-2165 (1964)) was hydrolyzed according to Procedure F (50°C overnight; acidified aqueous phase extracted with ethyl acetate; combined organic phases dried over MgSO₄, concentrated).
CIMS m/e 193.9 ((M-H)⁺), 195.8 (MH⁺); ¹H NMR (DMSO-*d*₆) δ 13.38-12.78 (br s, 1H), 12.43 (s, 1H), 9.90 (s, 1H), 7.92 (s, 1H), 7.08 (s, 1H).

### Example 40b

### 2-Cyano-4H-thieno[3,2-b]pyrrole-5-carboxylic acid

2-Formyl-4H-thieno[3,2-b]pyrrole-5-carboxylic acid was treated with hydroxylamine hydrochloride according to Procedure G.
CIMS m/e 190.9 ((M-H)⁺); ¹H NMR (DMSO-*d*₆) δ 13.06 (br s, 1H), 12.45 (s, 1H), 7.84 (s, 1H), 7.09 (s, 1H).

### Example 41

### 2-Cyano-4H-furo[3,2-b]pyrrole-5-carboxylic acid [(1S)-benzyl-3-((3R,4S)-dihydroxypyrrolidin-1-yl)-(2R)-hydroxy-3-oxo-propyl]-amide

2-Cyano-4H-furo[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure A (1 equiv triethylamine, dimethylformamide; 4 d reaction time; reaction mixture partitioned between ethyl acetate and water; organic phase washed with 2 N HCI prior to saturated aqueous NaHCO₃).
mp 137-140°C; CIMS m/e 437.1 ((M-H)⁺), 439.0 (MH⁺); ¹H NMR (DMSO-*d*₆) δ 11.70 (m, 1H), 7.99 (d, J = 8.9 Hz, 1H), 7.65 (m, 1H), 7.22 (m, 4H), 7.12 (m, 1H), 6.91 (s, 1H), 5.09 (d, J = 7.1 Hz, 0.5H), 4.95 (m, 1H), 4.89 (d, J = 5.2 Hz, 0.5H), 4.83 (d, J = 7.3 Hz, 0.5H), 4.76 (d, J = 3.9 Hz, 0.5H), 4.42 (m, 1H), 4.21 (m, 1H), 4.08-3.89 (m, 2H), 3.61-3.50 (m, 1H), 3.40 (m, 0.5H), 3.24 (m, 1.5H), 3.13 (m, 1H), 2.95-2.80 (m, 2H).

### Example 42

### 3-Bromo-4H-furo[3,2-b]pyrrole-5-carboxylic acid [(1S)-benzyl-2-((3R,4S)-dihydroxypyrrolidin-1-yl)-2-oxo-ethyl]-amide

3-Bromo-4H-furo[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure A (dimethylformamide; reaction mixture partitioned between ethyl acetate and water; organic phase washed with 2 N HCI prior to saturated aqueous NaHCO₃).
mp 140°C dec.; CIMS m/e 461.9/463.9 (MH⁺); ¹H NMR (300 MHz, DMSO-*d*₆) δ 11.75 (m, 1H), 8.47 (d, J = 8.6 Hz, 1H), 7.90 (d, J = 0.8 Hz, 1H), 7.33-7.15 (m, 5H), 6.98 (dd, J = 1.5, 3.4 Hz, 1H), 5.03 (d, J = 5.3 Hz, 0.5H), 4.95 (d, J = 5.1 Hz, 0.5H), 4.91-4.83 (m, 2H), 4.13-3.97 (m, 1H), 3.86 (m, 1.5H), 3.48-3.25 (m, 2.5H), 3.18 (m, 1H), 3.08-2.92 (m, 2H).

### Example 42a

### 3-Bromo-4H-furo[3,2-b]pyrrole-5-carboxylic acid ethyl ester

4-Bromo-2-furaldehyde was annulated according to Procedure H (aldehyde and azido-acetic acid ethyl ester added as ethanol solution (1 M of ester) to -20 °C ethoxide solution; -20°C 35 min, -5°C 1.5 h, 5°C 15 min; reaction poured into cold saturated aqueous NH₄Cl; after ether extraction, acrylate organic phase washed with water until aqueous phase was neutral; 0.5 M solution of crude acrylate heated).
CIMS m/e 257.81259.8 (MH⁺); ¹H NMR (300 MHz, CDCl₃) δ 8.81 (br s, 1H), 7.48 (s, 1H), 6.79 (d, J = 1.8 Hz, 1H), 4.35 (q, J = 7.1 Hz, 2H), 1.36 (t, J = 7.1 Hz, 3H).

### Example 42b

### 3-Bromo-4H-furo[3,2-b]pyrrole-5-carboxylic acid

3-Bromo-4H-furo[3,2-b]pyrrole-5-carboxylic acid ethyl ester was hydrolyzed according to Procedure F (50°C 14 h; acidified aqueous phase extracted with ethyl acetate; organic phase dried over MgSO₄, concentrated).
CIMS m/e 228.0/230.0 ((M-H)⁺); ¹H NMR (300 MHz, DMSO-*d*₆) δ 12.60 (br s, 1H), 12.06 (br s, 1H), 7.98 (s, 1H), 6.77 (d, J = 1.8 Hz, 1H).

### Example 43

### 3-Bromo-4H-furo[3,2-b]pyrrole-5-carboxylic acid [(1S)-benzyl-3-((3R,4S)-dihydroxypyrrolidin-1-yl)-(2R)-hydroxy-3-oxo-propyl]-amide

3-Bromo-4H-furo[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure A (1 equiv triethylamine, dimethylformamide; reaction mixture partitioned between ethyl acetate and water; organic phase washed with 2 N HCI prior to saturated aqueous NaHCO₃); mp 140°C dec; CIMS m/e 492.0/494.0 (MH⁺); ¹H NMR (300MHz, DMSO-*d*₆) δ 11.73 (s, 0.5H), 11.68 (s, 0.5H), 7.88 (d, J = 0.7 Hz, 1H), 7.79 (d, J = 8.9 Hz, 1H), 7.27 (m, 4H), 7.17 (m, 1H), 6.87 (s, 1H), 5.18-4.74 (m, 3H), 4.56-4.40 (m, 1H), 4.24 (s, 1H), 4.05-3.94 (m, 1.5H), 3.64-3.11 (m, 4.5H), 3.02-2.85 (m, 2H).

### Example 44

### 4H-1,7-Dithia-4-aza-cyclopenta[a]pentalene-5-carboxylic acid [(1S)-benzyl-3-((3R,4S)-dihydroxy-pyrrolidin-1-yl)-(2R)-hydroxy-3-oxo-propyl]-amide

4H-1,7-Dithia-4-aza-cyclopenta[a]pentalene-5-carboxylic acid and (3S)-amino-1-((3R,4S)-dihydroxy-pyrrolidin-1-yl)-(2R)-hydroxy-4-phenyl-butan-1-one were coupled according to Procedure B (2:1 dichloromethane:dimethylformamide; reaction mixture partitioned between ethyl acetate and water; organic phase washed with 2 N HCI prior to saturated aqueous NaHCO₃).
CIMS m/e 484.0 ((M-H)⁺), 486.0 (MH⁺); ¹H NMR (DMSO-*d*₆) δ 12.00 (s, 0.5H), 11.95 (s, 0.5H), 7.83 (m, 1H), 7.55 (dd, J = 0.8, 5.2 Hz, 1H), 7.35 (dd, J = 1.2, 5.2 Hz, 1H), 7.23 (m, 4H), 7.12 (m, 2H), 5.07 (d, J = 7.3 Hz, 0.5H), 4.96 (m, 1H), 4.90 (d, J = 5.0 Hz, 0.5H), 4.81 (d, J = 7.5 Hz, 0.5H), 4.76 (d, J = 4.2 Hz, 0.5H), 4.44 (m, 1H), 4.20 (m, 1H), 4.08-3.88 (m, 1.5H), 3.57 (m, 1H), 3.40 (m, 0.5H), 3.26 (m, 1.5H), 3.17-3.07 (m, 1.5H), 2.97-2.84 (m, 2H).

### Example 44a

### 4H-1,7-Dithia-4-aza-cyclopenta[a]pentalene-5-carboxylic acid ethyl ester

Thieno[2,3-b]thiophene-2-carbaldehyde (Dopper, J. H. et al., J. Am. Chem. Soc., 95: 3692-8 (1973)) was annulated according to Procedure H (aldehyde and azido-acetic acid ethyl ester added as ethanol solution (1 M of ester) to -20°C ethoxide solution; -20°C 30 min, -20°C to room temperature over 2.5 h; reaction poured into cold saturated aqueous NH₄Cl; after ether extraction, acrylate organic phase washed with water until aqueous phase was neutral; 0.35 M solution of crude acrylate heated).
CIMS m/e 250.1 ((M-H)⁺), 251.9 (MH⁺); ¹H NMR (CDCl₃) δ 9.46 (br s, 1H), 7.35 (d, J = 5.3 Hz, 1H), 7.29 (d, J = 5.3 Hz, 1H), 7.14 (d, J = 2.0 Hz, 1H), 4.38 (q, J = 7.1 Hz, 2H), 1.39 (t, J = 7.1 Hz, 3H).

### Example 44b

### 4H-1,7-Dithia-4-aza-cyclopenta[a]pentalene-5-carboxylic acid

4H-1,7-Dithia-4-aza-cyclopenta[a]pentalene-5-carboxylic acid ethyl ester was hydrolyzed according to Procedure F (50°C 11 h).
CIMS m/e 222.0 ((M-H)⁺); ¹H NMR (DMSO-*d*₆) δ 12.51 (s, 1H), 12.32 (s, 1H), 7.59 (d, J = 5.3 Hz, 1H), 7.38 (d, J = 5.3 Hz, 1H), 7.05 (d, J = 1.9 Hz, 1H).

### Example 45

### 4H-1,7-Dithia-4-aza-cyclopenta[a]pentalene-5-carboxylic acid [(1S)-benzyl-2-((3R,4S)-dihydroxy-pyrrolidin-1-yl)-2-oxo-ethyl]-amide

4H-1,7-Dithia-4-aza-cyclopenta[a]pentalene-5-carboxylic acid and (2S)-amino-1-((3R,4S)-dihydroxy-pyrrolidin-1-yl)-3-phenyl-propan-1-one hydrochloride were coupled according to Procedure B (1:1 dichloromethane:dimethylformamide; reaction mixture partitioned between ethyl acetate and water; organic phase washed with 2 N HCI prior to saturated aqueous NaHCO₃).
CIMS m/e 454.0 ((M-H)⁺), 456.0 (MH⁺); ¹H NMR (DMSO-*d*₆) δ12.03 (m, 1H), 8.53 (d, J = 8.1 Hz, 1H), 7.55 (dd, J = 0.8, 5.2 Hz, 1H), 7.34 (dd, J = 2.7, 5.2 Hz, 1H), 7.29-7.19 (m, 5H), 7.12 (m, 1H), 4.99 (d, J = 5.2 Hz, 0.5H), 4.91 (d, J = 5.0 Hz, 0.5H), 4.84 (m, 2H), 3.98 (m, 1H), 3.83 (m, 2H), 3.41-3.29 (m, 3H), 3.13 (m, 1H), 2.95 (m, 2H).

### Example 46

### 2-Chloro-3-methyl-4H-thieno[3,2-b]pyrrole-5-carboxylic acid [(1S)-benzyl-2-((3R,4S)-dihydroxy-pyrrolidin-1-yl)-2-oxo-ethyl]-amide

2-Chloro-3-methyl-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (1:1 dichloromethane:dimethylformamide; 2 d reaction time; reaction mixture concentrated to remove dichloromethane; partitioned between ethyl acetate and water; organic phase washed with 2 N HCI prior to saturated aqueous NaHCO₃); mp 139-141°C; CIMS mle 448.1/450.1 (MH⁺); ¹H NMR (DMSO-*d*₆) δ 11.95 (m, 1H), 8.54 (d, J = 7.9 Hz, 1H), 7.28-7.18 (m, 4H), 7.13 (m, 2H), 4.98 (d, J = 5.2 Hz, 0.5H), 4.90 (d, J = 4.6 Hz, 0.5H), 4.83 (m, 2H), 4.02-3.92 (m, 1H), 3.82 (m, 1.5H), 3.41-3.22 (m, 2.5H), 3.14 (m, 1H), 3.01-2.90 (m, 2H), 2.20 (d, J = 2.5 Hz, 3H).

### Example 46a

### 5-Chloro-4-methyl-thiophene-2-carbaldehyde

Using a modified procedure of Silverstein et al. (Organic Synthesis Coll. Vol 4, Wiley, NewYork, 1963, N. Rabjohn, ed. p 831), to a 80°C pale yellow solution of 2-chloro-3-methyl-thiophene (Crast, L. B., Jr. U.S. Patent 3290291, Example 2; 70 g, 0.53 mol) in dimethylformamide (48.3 g, 0.66 mol) was added phosphorous oxychloride (101.5 g, 0.66 mol) dropwise over 45 min, while maintaining temperature at 80-97°C. The dark brown solution was stirred at 90°C for 3 h and poured slowly into water (500 mL) at 90°C. The resultant mixture was steam distilled and the distillate was cooled to 0°C, affording white crystals. The first500 ml of distillate was extracted with chloroform and concentrated and the residue was recrystallized from hexane (150 ml) at -50°C. The crude product (8.6 g) was dissolved in hexane (100 ml) and the insoluble material was filtered. The filtrate was diluted with hexane (50 ml), stirred with Norit® (2 g), and concentrated. The product was purified by recrystallization from hexane (50 ml) at -40°C and obtained as white crystals (7.5 g, 13%). The remaining distillate from the steam distillation was extracted with chloroform (2 x 250 ml) and the white crystals dissolved in chloroform (1.5 I). The combined organic phases were dried over MgSO₄, filtered, stirred with Norit® (30 g) for 15 min, and concentrated. The residue was dissolved in hexane (350 ml), the insoluble material was filtered, the filtrate was stirred at -15 °C for 10 min, and the resultant precipitate was filtered. The title product was obtained as pale yellow crystals (48.6 g, 83%); mp 39-40°C.

### Example 46b

### 2-Chloro-3-methyl-4H-thieno[3,2-b]pyrrole-5-carboxylic acid ethyl ester

5-Chloro-4-methyl-thiophene-2-carbaldehyde was annulated according to Procedure H (aldehyde and azido-acetic acid ethyl ester was added as ethanol solution (1 M of ester) to -20°C ethoxide solution; allow to warm to 10°C over 2 h, 10°C 2 h; reaction poured into cold saturated aqueous NH₄Cl; after ether extraction, acrylate organic phase washed with water until aqueous phase was neutral; solution of crude acrylate added to refluxing xylenes over 5 min and then heated at reflux).
CIMS m/e 243.8/245.9 (MH⁺); ¹H NMR (CDCl₃) δ9.25 (br s, 1H), 7.02 (d, J = 1.9 Hz, 1H), 4.36 (q, J = 7.1 Hz, 2H), 2.28 (s, 3H), 1.38 (t, J = 7.1 Hz, 3H).

### Example 46c

### 2-Chloro-3-methyl-4H-thieno[3,2-b]pyrrole-5-carboxylic acid

2-Chloro-3-methyl-4H-thieno[3,2-b]pyrrole-5-carboxylic acid ethyl ester was hydrolyzed according to Procedure F (50 °C 14 h).
CIMS m/e 213.8/215.8 ((M-H)⁺); ¹H NMR (DMSO-*d*₆) δ 12.61 (br s, 1H), 12.25 (s, 1H), 6.96 (dd, J = 0.5, 2.0 Hz, 1H), 2.23 (s, 1H).

### Example 47

### 2-Chloro-3-methyl-4H-thieno[3,2-b]pyrrole-5-carboxylic acid [(1S)-benzyl-3-((3R,4S)-dihydroxy-pyrrolidin-1-yl)-(2R)-hydroxy-3-oxo-propyl]-amide

2-Chloro-3-methyl-4H-thieno[3,2-b]pyrrole-5-carboxylic acid and (3S)-amino-1-((3R,4S)-dihydroxy-pyrrolidin-1-yl)-(2R)-hydroxy-4-phenyl-butan-1-one were coupled according to Procedure B (4:5 dichloromethane:dimethylformamide; 2 d reaction time; reaction mixture concentrated to remove dichloromethane; partitioned between ethyl acetate and water; organic phase washed with 2 N HCI prior to saturated aqueous NaHCO₃).
mp 150-153°C; CIMS m/e 478.1/480.1 (MH⁺); ¹H NMR (DMSO-*d*₆) δ 11.91 (s, 0.5H), 11.86 (s, 0.5H), 7.82 (d, J = 8.7, 1H), 7.22 (m, 4H), 7.11 (m, 1H), 7.01 (m, 1H), 5.07 (d, J = 6.8 Hz, 0.5H), 4.95 (m, 1H), 4.90 (d, J = 5.0 Hz, 0.5H), 4.82 (d, J = 6.8 Hz, 0.5H), 4.77 (d, J = 3.7 Hz, 0.5H), 4.44 (m, 1H), 4.21 (m, 1H), 4.08-3.88 (m, 1.5H), 3.56 (m, 1H), 3.40 (m, 0.5H), 3.24 (m, 1.5H), 3.14 (m, 1H), 3.08 (m, 0.5H), 2.95-2.82 (m, 2H).

### Example 48

### 2-Methylsulfanyl-4H-thieno[3,2-b]pyrrole-5-carboxylic acid [(1S)-benzyl-2-((3R,4S)-dihydroxy-pyrrolidin-1-yl)-2-oxo-ethyl]-amide

2-Methylsulfanyl-4H-thieno[3,2-b]pyrrole-5-carboxylic acid and (2S)-amino-1-((3R,4S)-dihydroxy-pyrrolidin-1-yl)-3-phenyl-propan-1-one hydrochloride were coupled according to Procedure B (1:1 dichloromethane:dimethylformamide; reaction mixture concentrated to remove dichloromethane, partitioned between ethyl acetate and water; organic phase washed with 2 N HCI prior to saturated aqueous NaHCO₃).
mp 104-110°C; 444.0 ((M-H)⁺), 445.9 (MH⁺); ¹H NMR (DMSO-*d*₆)) δ 11.61 (m, 1H), 8.52 (d, J = 8.6 Hz, 1H), 7.28-7.12 (m, 6H), 6.97 (d, J = 3.9 Hz, 1H), 4.99 (d, J = 5.1 Hz, 0.5H), 4.91 (d, J = 5.1 Hz, 0.5H), 4.83 (m, 2H), 4.06-3.94 (m, 1H), 3.80 (m, 2H), 3.43-3.24 (m, 2H), 3.14 (m, 1H), 3.02-2.87 (m, 2H), 2.46 (s, 3H).

### Example 48a

### 2-Methylsulfanyl-4H-thieno[3,2-b]pyrrole-5-carboxylic acid ethyl ester

5-Methylsulfanyl-thiophene-2-carbaldehyde was annulated according to Procedure H (aldehyde and azido-acetic acid ethyl ester added as ethanol solution (1 M of ester) to -20°C ethoxide solution; allow to warm to 10°C over 4 h; reaction poured into cold saturated aqueous NH₄Cl; after ether extraction, acrylate organic phase washed with water until aqueous phase was neutral; crude acrylate solution heated at reflux for 2 h, allowed to cool to room temperature, stirred overnight).
CIMS m/e 240.0 ((M-H)⁺), 242.0 (MH⁺);¹H NMR (CDCl₃) δ 9.05 (br s, 1H), 7.04 (m, 1H), 6.97 (s, 1H), 4.35 (q, J = 7.1 Hz, 2H), 2.53 (s, 3H), 1.37 (t, J = 7.1 Hz, 3H).

### Example 48b

### 2-Methylsulfanyl-4H-thieno[3,2-b]pyrrole-5-carboxylic acid

2-Methylsulfanyl-4H-thieno[3,2-b]pyrrole-5-carboxylic acid ethyl ester was hydrolyzed according to Procedure F (40 °C overnight).
CIMS m/e 211.9 ((M-H)⁺); ¹H NMR (DMSO-*d*₆) δ 12.56 (s, 1H), 11.90 (s, 1H), 6.97 (s, 1H), 6.94 (s, 1H), 2.47 (s, 1H).

A preferred subgroup of formula (IV) compounds are those compounds selected from the group consisting of:
2-chloro-6H-thieno[2,3-b]pyrrole-5-carboxylic acid-[(1S)-benzyl-2-((3R,4S)-dihydroxy-pyrrolidin-1-yl)-2-oxo-ethyl]-amide;
(±)-2-bromo-4H-furo[3,2-b]pyrrole-5-carboxylic acid-[1-benzyl-2-((3R,4S)-dihydroxy-pyrrolidin-1-yl)-2-oxo-ethyl]-amide;
2-bromo-4H-thieno[3,2,-b]pyrrole-5-carboxylic acid-[(1S)-benzyl-2-((3R,4S)-dihydroxy-pyrrolidin-1 -yl)-2-oxo-ethyl]-amide;
2-chloro-6H-thieno[2,3-b]pyrrole-5-carboxylic acid-[(1S)-benzyl-2-morpholin-4-yl-2-oxo-ethyl]-amide;
2-chloro-6H-thieno[2,3-b]pyrrole-5-carboxylic acid-[(1S)-benzyl-2-(1,1dioxo-1-thiazolidin-3-yl)-2-oxo-ethyl]-amide;
2-chloro-4H-furo[3,2-b]pyrrole-5-carboxylic acid-[(1S)-benzyl-2-((3R,4S)-dihydroxy-pyrrolidin-1-yl)-2-oxo-ethyl]-amide;
2-chloro-4H-furo[3,2-b]pyrrole-5-carboxylic acid-[(1S)-benzyl-3-((3R,4S)-dihydroxy-pyrrolidin-1-yl)-(2R)hydroxy-3-oxo-propyl]-amide;
2-chloro-4H-thieno[3,2-b]pyrrole-5-carboxylic acid-[(1S)-benzyl-2-((3R,4S)-dihydroxy-pyrrolidin-1-yl)-2-oxo-ethyl]-amide; and
3-methyl-4H-thieno[3,2-b]pyrrole-5-carboxylic acid-[(1S)-benzyl-2-((3R,4S)-dihydroxy-pyrrolidin-1-yl)-2-oxo-ethyl]-amide; the stereoisomers and prodrugs thereof, and the pharmaceutically acceptable salts of the compounds, stereoisomers and prodrugs.

Another aspect of the invention provides methods of treating prophylactically an individual in whom Type 2 diabetes mellitus has not yet presented, but in whom there is an increased risk of developing such condition, which methods comprise administering to an individual in need thereof effective amounts of a glycogen phosphorylase inhibitor and a non-glycogen phosphorylase inhibiting anti-diabetic agent, or a glycogen phosphorylase inhibitor and an anti-obesity agent, preferably in the form of a pharmaceutical composition.

In this aspect, generally preferred glycogen phosphorylase inhibitors may comprise, for example, the compounds of formulae (I), (II), (III), and (IV), the stereoisomers and prodrugs thereof, and the pharmaceutically acceptable salts of the compounds, stereoisomers, and prodrugs depicted hereinabove.

Generally preferred non-glycogen phosphorylase inhibiting anti-diabetic agents may comprise, for example, D-chiroinositol; insulin and insulin analogs; GLP-1 (7-37)(insulinotropin) and GLP-1 (7-36)-NH₂; α-glucosidase inhibitors; glitazones and/or insulin sensitizers; sulfonylureas and analogs thereof; biguanides; α₂-antagonists and imidazolines; insulin secretagogues; aldose reductase inhibitors; fatty acid oxidation inhibitors; β-agonists; phosphodiesterase inhibitors; lipid-lowering agents; vanadate and vanadium complexes; amylin antagonists; glucagon antagonists; growth hormone secretagogues; gluconeogenesis inhibitors; somatostatin analogs; antilipolytic agents; lipoxygenase inhibitors; insulin signaling agonists; insulin mimetics; PTP1B inhibitors; insulin degrading enzyme inhibitors; glycogen synthase kinase inhibitors; and the like. Other non-glycogen phosphorylase inhibiting anti-diabetic agents, including the preferred agents set forth hereinbelow, are well known, or will be readily apparent in light of the instant disclosure, to one of ordinary skill in the art.

Preferred forms of insulin useful in the methods of the invention may comprise, for example, inhaled insulin, or insulin analogs, for example, LysPro insulin. Preferred α-glucosidase inhibitors useful in the methods of the invention may comprise those agents such as acarbose, voglibose, miglitol, emiglitate, camiglibose, MDL-25637, and MDL-73,945. Preferred glitazones and/or insulin sensitizers useful in the methods of the invention may comprise, for example, ciglitazone, pioglitazone, englitazone, troglitazone, darglitazone, rosiglitazone, JTT-501, MCC-555, and MX 6054. Preferred sulfonylureas and analogs thereof useful in the methods of the invention may comprise, for example, chlorpropamide, glibenclamide, tolbutamide, tolazamide, acetohexamide, glipizide, glimepiride, repaglinide, and meglitinide. Preferred biguanides useful in the methods of the invention may comprise, for example, metformin, phenformin, and buformin. Preferred α₂-antagonists and imidazolines useful in the methods of the invention may comprise, for example, midaglizole, isaglidole, deriglidole, idazoxan, efaroxan, and fluparoxan. Preferred insulin secretagogues useful in the methods of the invention may comprise, for example, linogliride, A-4166, exendin-4, and BTS-67582. Preferred aldose reductase inhibitors useful in the methods of the invention may comprise, for example, epalrestat, sorbinil, tolrestat, zenarestat, and zopolrestat. Preferred fatty acid oxidation inhibitors useful in the methods of the invention may comprise, for example, clomoxir and etomoxir. Preferred β-agonists useful in the methods of the invention may comprise, for example, BRL-35135, BRL-37344, TAK-667, AZ 40140, and CL 316,243. Preferred phosphodiesterase inhibitors useful in the methods of the invention may comprise, for example, L-386,398. Preferred lipid-lowering agents useful in the methods of the invention may comprise, for example, benfluorex. Preferred vanadate and vanadium complexes useful in the methods of the invention may comprise, for example, naglivan and peroxovandium complexes. Preferred gluconeogenesis inhibitors useful in the methods of the invention may comprise, for example, glucose-6-phosphatase inhibitors, or GP 3034. Preferred antilipolytic agents useful in the methods of the invention may comprise, for example, nicotinic acid, acipimox, and WAG 994. Preferred amylin antagonists useful in the methods of the invention may comprise, for example, pramlintide and AC-137. Preferred glucagon antagonists useful in the methods of the invention may comprise, for example, BAY 27-9955. Preferred lipoxygenase inhibitors useful in the methods of the invention may comprise, for example, masoprocol. Preferred insulin signaling agonists useful in the methods of the invention may comprise, for example, L-783281.

Generally preferred anti-obesity agents may comprise, for example, β-adrenergic receptor agonists, apolipoprotein-B secretion/microsomal triglyceride transfer protein (apo-B/MTP) inhibitors, MCR-4 agonists, cholecystokinin-A (CCK-A) agonists, monoamine reuptake inhibitors (such as sibutramine), sympathiomimetic agents, serotoninergic agents (such as dexfenfluramine or fenfluramine), dopamine agonists (such as bromocriptine), melanocyte-stimulating hormone receptor agonists or mimetics, melanocyte-stimulating hormone analogs, melanin concentrating hormone antagonists, cannabinoid receptor antagonists, the OB protein (leptin), a leptin analog, galanin antagonists, lipase inhibitors (such as orlistat), anorectic agents, for example, bombesin agonists, Neuropeptide-Y antagonists, thyromimetic agents, dehydroepiandrosterones or analogs thereof, glucocorticoid receptor agonists or antagonists, orexin receptor antagonists, urocortin binding protein antagonists, glucagon-like peptide-1 receptor agonists, ciliary neurotrophic factors (such as Axokine), or human agouti-related protein (referred to hereinafter as AGRP) antagonists. Other anti-obesity agents, including the preferred agents set forth hereinbelow, are well known, or will be readily apparent in light of the instant disclosure, to one of ordinary skill in the art.

Particularly preferred anti-obesity agents useful in the practice of this invention comprise β-adrenergic receptor agonists, sibutramine, orlistat, fenfluramine, dexfenfluramine, bromocriptine, phentermine, ephedrine, leptin, phenylpropanolamine, and pseudoephedrine. Particularly preferred β-adrenergic receptor agonists include those substituted aminopyridines disclosed in commonly assigned PCT International Application Publication No. WO 96/35671, the disclosure of which is hereby incorporated by reference. Especially preferred β-adrenergic receptor agonists disclosed therein are selected from the group consisting of {4-[2-(2-[6-aminopyridin-3-yl]-2-(R)-hydroxyethylamino)ethoxy]phenyl}acetic acid, {4-[2-(2-[6-aminopyridin-3-yl]-2-(R)-hydroxyethylamino)ethoxy]phenyl}benzoic acid, {4-[2-(2-[6-aminopyridin-3-yl]-2-(R)-hydroxyethylamino)ethoxy]phenyl}propionic acid, and {4-[2-(2-[6-aminopyridin-3-yl]-2-(R)-hydroxyethylamino)ethoxy]phenoxy}acetic acid.

The dosage of the glycogen phosphorylase inhibitor to be administered in accordance with the methods of the invention will generally be dependent upon a number of factors including the health of the subject being treated, the extent of treatment desired, the nature and kind of concurrent therapy, if any, and the frequency of treatment and the nature of the effect desired. In general, glycogen phosphorylase inhibitors have been reported with representative dosage ranges being from about 0.005 to about 50 mg/kg body weight of the individual per day. Generally, preferable dosages range from about 0.01 to about 25 mg/kg body weight of the individual per day, and, most preferably, from about 0.1 to about 15 mg/kg body weight of the individual per day. However, some variability in the general dosage range may be required depending upon the age and weight of the subject being treated, the intended route of administration, and the like.

The dosage of the non-glycogen phosphorylase inhibiting anti-diabetic agent will also be generally dependent upon a number of factors including the health of the subject being treated, the extent of treatment desired, the nature and kind of concurrent therapy, if any, and the frequency of treatment and the nature of the effect desired. In general, the dosage range of the non-glycogen phosphorylase inhibiting anti-diabetic agent is generally from about 0.001 to about 50 mg/kg body weight of the individual per day, preferably from about 0.01 to about 20 mg/kg body weight of the individual per day, administered as a single or divided dose. However, some variability in the general dosage range may be required depending upon the age and weight of the subject being treated, the intended route of administration, the particular non-glycogen phosphorylase inhibiting anti-diabetic agent being administered, and the like.

The dosage of the anti-obesity agent will also be generally dependent upon a number of factors including the health of the subject being treated, the extent of treatment desired, the nature and kind of concurrent therapy, if any, and the frequency of treatment and the nature of the effect desired. In general, the dosage range of the anti-obesity agent is generally in the range of from about 0.001 to about 100 mg/kg body weight of the individual per day, preferably from about 0.1 to about 10 mg/kg body weight of the individual per day, administered as a single or divided dose. However, some variability in the general dosage range may be required depending upon the age and weight of the subject being treated, the intended route of administration, the particular anti-obesity agent being administered, and the like.

According to the methods of the invention, a glycogen phosphorylase inhibitor, a stereoisomer or prodrug thereof, or a pharmaceutically acceptable salt of the inhibitor, stereoisomer, or prodrug; or a glycogen phosphorylase inhibitor, a stereoisomer, or prodrug thereof, or a pharmaceutically acceptable salt of the inhibitor, stereoisomer, or prodrug, and a non-glycogen phosphorylase inhibiting anti-diabetic agent or anti-obesity agent is administered to the subject in need of treatment therewith, preferably in the form of a pharmaceutical composition. The glycogen phosphorylase inhibitor, the stereoisomer or prodrug thereof, or the pharmaceutically acceptable salt of the inhibitor, stereoisomer, or prodrug, and the non-glycogen phosphorylase inhibiting anti-diabetic agent or anti-obesity agent may be administered either separately or in the pharmaceutical composition comprising both. It is generally preferred that such administration be oral. However, if the subject being treated is unable to swallow, or oral administration is otherwise impaired or undesirable, parenteral or transdermal administration will be appropriate.

According to the methods of the invention, when the glycogen phosphorylase inhibitor, a stereoisomer, or prodrug thereof, or a pharmaceutically acceptable salt of the inhibitor, stereoisomer, or prodrug; or the glycogen phosphorylase inhibitor, a stereoisomer, or prodrug thereof, or a pharmaceutically acceptable salt of the inhibitor, stereoisomer, or prodrug, and the non-glycogen phosphorylase inhibiting anti-diabetic agent or anti-obesity agent are administered together, such administration can be sequential in time or simultaneous with the simultaneous method being generally preferred. For sequential admininstration, the glycogen phosphorylase inhibitor, a stereoisomer, or prodrug thereof, or a pharmaceutically acceptable salt of the inhibitor, stereoisomer, or prodrug; or the glycogen phosphorylase inhibitor, a stereoisomer, or prodrug thereof, or a pharmaceutically acceptable salt of the inhibitor, stereoisomer, or prodrug, and the non-glycogen phosphorylase inhibiting anti-diabetic agent or anti-obesity agent can be administered in any order. It is generally preferred that such administration be oral. It is especially preferred that such administration be oral and simultaneous. However, if the subject being treated is unable to swallow, or oral absorption is otherwise impaired or undesirable, parenteral or transdermal administration will be appropriate. When the glycogen phosphorylase inhibitor, a stereoisomer, or prodrug thereof, or a pharmaceutically acceptable salt of the inhibitor, stereoisomer, or prodrug; or the glycogen phosphorylase inhibitor, a stereoisomer, or prodrug thereof, or a pharmaceutically acceptable salt of the inhibitor, stereoisomer, or prodrug, and the non-glycogen phosphorylase inhibiting anti-diabetic agent or anti-obesity agent are administered sequentially, the administration of each can be by the same or by different methods.

According to the methods of the invention, the glycogen phosphorylase inhibitor, a stereoisomer, or prodrug thereof, or a pharmaceutically acceptable salt of the inhibitor, stereoisomer, or prodrug; or the the glycogen phosphorylase inhibitor, a stereoisomer, or prodrug thereof, or a pharmaceutically acceptable salt of the inhibitor, stereoisomer, or prodrug, and the non-glycogen phosphorylase inhibiting anti-diabetic agent or anti-obesity agent is preferably administered in the form of a pharmaceutical composition comprising a pharmaceutically acceptable carrier, vehicle, or diluent. Accordingly, the glycogen phosphorylase inhibitor, a stereoisomer, or prodrug thereof, or a pharmaceutically acceptable salt of the inhibitor, stereoisomer, or prodrug; or the the glycogen phosphorylase inhibitor, a stereoisomer, or prodrug thereof, or a pharmaceutically acceptable salt of the inhibitor, stereoisomer, or prodrug, and the non-glycogen phosphorylase inhibiting anti-diabetic agent or anti-obesity agent can be administered separately or together in any conventional oral, parenteral, or transdermal dosage form.

Suitable pharmaceutically acceptable carriers include inert solid fillers or diluents and sterile aqueous or organic solutions. According to the methods of the invention, the glycogen phosphorylase inhibitor, a stereoisomer, or prodrug thereof, or a pharmaceutically acceptable salt of the inhibitor, stereoisomer, or prodrug; or the glycogen phosphorylase inhibitor, a stereoisomer, or prodrug thereof, or a pharmaceutically acceptable salt of the inhibitor, stereoisomer, or prodrug, and the non-glycogen phosphorylase inhibiting anti-diabetic agent or anti-obesity agent will be present in such pharmaceutical compositions in amounts sufficient to provide the desired dosage amount in the ranges described hereinabove. Thus, for oral administration, the compounds can be combined with a suitable solid or liquid carrier, vehicle or diluent to form capsules, tablets, pills, powders, syrups, solutions, suspensions, and the like. The pharmaceutical compositions may contain, if desired, additional components such as flavorants, sweeteners, excipients, and the like.

The tablets, pills, capsules, and the like may also contain a binder such as gum tragacanth, acacia, com starch or gelatin; excipients such as dicalcium phosphate; a disintegrating agent such as com starch, potato starch, or alginic acid; a lubricant such as magnesium stearate; a sweetening agent such as sucrose, lactose, or saccharin; and adjuvants including coloring agents, preservants, and antioxidants. When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar, or both. A syrup or elixir may contain, in addition to the active ingredient, sucrose as a sweetening agent, methyl or propylparabens as preservatives, a dye and a flavoring such as cherry or orange flavor.

The pharmaceutical compositions of the invention may also be administered parenterally. For parenteral administration, the pharmaceutical compositions can be combined with sterile aqueous or organic media to form injectable solutions or suspensions. Solutions or suspensions of these pharmaceutical compositions can be prepared in water suitably mixed with a surfactant such as hydroxypropylcellulose. Dispersions can also be prepared in sesame or peanut oil, ethanol, water, polyol (e.g., glycerol, propylene glycol, and liquid polyethylene glycol), suitable mixtures thereof, vegetable oils, N-methyl glucamine, polyvinylpyrrolidone, and mixtures thereof in oils as well as aqueous solutions of water-soluble pharmaceutically acceptable salts of the compounds or prodrugs of the compounds. Under ordinary conditions of storage and use, these preparations contain a preservative to prevent the growth of microorganisms. The injectable solutions prepared in this manner can then be administered intravenously, intraperitoneally, subcutaneously, or intramuscularly, with intramuscular administration being the preferred parenteral route in humans. Solutions prepared for intravenous administration are preferably rendered isotonic prior to usage.

The pharmaceutical forms suitable for injectable use include sterile aqueous solutions or dispersions and sterile powders for the preparation of sterile injectable solutions or dispersions. In all cases, however, the form must be sterile and must be fluid to the extent that facile syringability exists. It must be stable under the conditions of manufacture and storage and must be preserved against contamination by microorganisms such as bacteria and fungi.

The glycogen phosphorylase inhibitor, a stereoisomer, or prodrug thereof, or a pharmaceutically acceptable salt of the inhibitor, stereoisomer, or prodrug; or the glycogen phosphorylase inhibitor, a stereoisomer, or prodrug thereof, or a pharmaceutically acceptable salt of the inhibitor, stereoisomer, or prodrug, and the non-glycogen phosphorylase inhibiting anti-diabetic agent or anti-obesity agent may also be encapsulated in liposomes to permit intravenous administration thereof. The liposomes suitable for use in this intention may include lipid vesicles and comprise plurilamellar lipid vesicles, small sonicated multilamellar vesicles, reverse phase evaporation vesicles, large multilamellar vesicles, and the like, wherein the lipid vesicles are formed by one or more phospholipids such as phosphotidylcholine, phosphatidylcholine, sphingomyelin, phospholactic acid, and the like. In addition, the vesicles may also comprise a sterol component such as cholesterol.

The pharmaceutical compositions may also be administered transdermally. Suitable formulations for transdermal application include an amount of the glycogen phosphorylase inhibitor, a stereoisomer, or prodrug thereof, or a pharmaceutically acceptable salt of the inhibitor, stereoisomer, or prodrug; or the glycogen phosphorylase inhibitor, a stereoisomer, or prodrug thereof, or a pharmaceutically acceptable salt of the inhibitor, stereoisomer, prodrug, and the non-glycogen phosphorylase inhibiting anti-diabetic agent or anti-obesity agent with a suitable transdermal carrier. Preferred transdermal carriers include absorbable pharmacologically acceptable solvents to promote and assist passage through the skin of the subject being treated. Characteristically, transdermal devices comprise the form of a bandage having a backing member, a reservoir containing the compound, optionally with carriers, optionally a rate-controlling barrier to deliver the compound to the skin of the subject being treated at a controlled and predetermined rate over a prolonged period of time, and means to secure the device to the skin of the subject being treated.

Methods of preparing the various pharmaceutical compositions with a desired amount of an active ingredient are known, or will be apparent in light of the instant disclosure, to one of ordinary skill in the art. See, for example, Remington's Pharmaceutical Sciences, Mack Publishing Co., Easton, PA, 18th Edition, (1990).

### Experimental

The ability of a glycogen phosphorylase inhibitor, a combination of a glycogen phosphorylase inhibitor and a non-glycogen phosphorylase inhibiting anti-diabetic agent, or a combination of a glycogen phosphorylase inhibitor and an anti-obesity agent, to treat prophylactically an individual in whom Type 2 diabetes mellitus has not yet presented, but in whom there is an increased risk of developing such condition, may be demonstrated according to the following exemplary, non-limiting protocols.

The experimetal protocol described by Sreenan, et al., Am. J. Physiol., 271, E742-747 (1996), may be employed to evaluate the glycogen phosphorylase inhibitor, a combination of a glycogen phosphorylase inhibitor and a non-glycogen phosphorylase inhibiting anti-diabetic agent, or a combination of a glycogen phosphorylase inhibitor and an anti-obesity agent for the ability to prevent or delay the onset of diabetes in the prone obese Zucker diabetic fatty rat (Charles River Labs, Wilmington, MA and Genetic Models Inc.; Indianapolis, IN), or for the delay or prevention of the onset of insulin resistance or impaired glucose toleration in the prone obese Zucker fatty rat.

Rats six weeks of age may be initiated on a daily regimen of treatment employing a glycogen phosphorylase inhibitor, a combination of a glycogen phosphorylase inhibitor and a non-glycogen phosphorylase inhibiting anti-diabetic agent, or a combination of a glycogen phosphorylase inhibitor and an anti-obesity agent (p.o. by gavage or in the chow), while being maintained on a standard rodent diet (Purina 5008; W.F. Fisher & Son, Inc., Bound Brook, NJ). After six weeks, the rats are fasted overnight, and blood samples are taken for determination of serum glucose, insulin, triglyceride, and free fatty acid concentrations. The results for the treated rats are compared against the untreated rats, and also against the lean littermates, which are considered normal. A reduction in serum glucose, insulin, triglyceride, and/or free fatty acid levels in the treated group compared to the untreated group indicates delay or prevention of the onset of diabetes or insulin resistance attributable to the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent. The animals can also be administered a glucose tolerance test after the six week treatment period. A reduction in serum glucose or insulin levels during the glucose tolerance test period by the treated group compared to the untreated group also indicates that the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent delayed or prevented the onset of diabetes, insulin resistance, and/or impaired glucose tolerance.

According to the methods described by Thomas, et al., Biochem. Pharm., 56, 1145-1150 (1998), the glycogen phosphorylase inhibitor, a combination of a glycogen phosphorylase inhibitor and a non-glycogen phosphorylase inhibiting anti-diabetic agent, or a combination of a glycogen phosphorylase inhibitor and an anti-obesity agent can also be tested for delay or prevention of the onset of insulin resistance in dexamethasone-induced hyperglycemic and insulin resistant mice.

C57BL6 (+/+) mice (Jackson Laboratory; Bar Harbor, ME) 15 weeks of age may be treated with dexamethasone at 2.5 mg/kg/day plus the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent (p.o. by gavage) or vehicle (untreated) for ten days. At the end of the ten-day period, blood samples are taken for plasma glucose and insulin determination in the fed state. The animals are fasted for 12 hr., and subjected to an insulin tolerance test, comprising blood sampling at 10, 20, and 40 min. after i.p. administration of 0.5 U/kg insulin, for calculation of plasma glucose disappearance. Alternatively, a glucose tolerance test may be administered to the fasted animals after the ten-day treatment period.A reduction in plasma glucose or insulin levels in the fed state, a greater plasma glucose disappearance during the insulin tolerance test, and/or lower glucose or insulin levels during the glucose tolerance test by the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent plus dexamethasone-treated group compared to the dexamethasone control group indicates that the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent prevented or delayed the onset of insulin resistance, hyperglycemia, and impaired glucose tolerance by the dexamethasone treatment.

Further, according to the method described by Davidson, et al., Am. J. Physiol., 264, E18-23, (1993), the glycogen phosphorylase inhibitor, a combination of a glycogen phosphorylase inhibitor and a non-glycogen phosphorylase inhibiting anti-diabetic agent, or a combination of a glycogen phosphorylase inhibitor and an anti-obesity agent may also be tested for the ability to delay or prevent the onset of insulin resistance induced by a cafeteria diet treatment of rats.

Male Sprague-Dawley rats (Charles Rivers Labs; Wilmington, MA) weighing 200 g are fed a cafeteria diet consisting of braunschweiger liver sausage, assorted candy bars, cheeses, cookies, com chips, granola bars, marshmallows, peanut butter, Twinkies, and sweetened condensed milk plus the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent (p.o. by gavage or in the diet) or vehicle (untreated) for seven to forty-two days. At the end of the seven to forty-two day period, blood samples are taken for plasma glucose and insulin determination in the fed state. Body weight and adipose depot weight are also determined.A reduction in plasma glucose or insulin levels in the fed state, and/or lower glucose or insulin levels during the glucose tolerance test,- and/or reduced weight gain or obesity (adipose depot weight) by the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent plus cafeteria diet-treated group compared to the cafeteria-fed control group will indicate that the glycogen phosphorylase inhibitor, the combination of the glycogen phosphorylase inhibitor and the non-glycogen phosphorylase inhibiting anti-diabetic agent, or the combination of the glycogen phosphorylase inhibitor and the anti-obesity agent delayed or prevented the onset of insulin resistance, hyperglycemia, and impaired glucose tolerance induced by the cafeteria diet treatment.

## Claims

1. Use of a glycogen phosphorylase inhibitor in the manufacture of a medicament for treating prophylactically an individual in whom Type 2 diabetes mellitus has not yet presented, but in whom there is an increased risk of developing such condition.

2. The use according to claim 1 wherein said increased risk comprises a risk factor associated with a Type 2 diabetes pre-disposing disease state or condition.

3. The use according to claim 2 wherein said risk factor is selected from the group consisting of:
(i) risk factors associated with classification as an individual having insulin resistance and/or hyperinsulinemia;
(ii) risk factors based on environmental or genetic Type 2 diabetes pre-disposing disease states or conditions;
(iii) risk factors predicated on race and/or ethnicity;
(iv) risk factors based on genetic mutations affecting (3-cell function;
(v) risk factors based on genetic defects in insulin action;
(vi) risk factors based on presence of excess adipose tissue or clinically diagnosed obesity;
(vii) risk factors identified through clinical chemistries or diagnostic testing signifying a pre-diabetic state;
(viii) risk factors related to physiologic and endocrine changes associated with growth, development, or aging;
(ix) risk factors related to diet or eating behaviors;
(x) risk factors based on abnormal cardiovascular or blood lipid parameters;
(xi) risk factors based on reproductive status;
(xii) risk factors attributable to muscle wasting;
(xiii) risk factors associated with polycystic ovary syndrome;
(xiv) risk factors due to organ disease or dysfunction;
(xv) risk factors due to conditions resulting in metabolic disturbances;
(xvi) risk factors due to endocrine disorders or endocrinopathies;
(xvii) risk factors due to pathophysiologic states;
(xviii) risk factors factors due to immune-mediated disease;
(xix) risk factors incurred due to drug or chemical exposure;
(xx) risk factors associated with having a genetic syndrome associated with diabetes; and
(xxi) risk factors associated with the detrimental effects caused by the administration of prolonged, elevated doses of insulin and/or the presence of ketoacidosis.

4. The use according to claim 3 wherein said risk factor based on an environmental or genetic Type 2 diabetes pre-disposing disease state or condition comprises a family history of diabetes; said risk factor predicated on race and/or ethnicity comprises individual membership in an African-American, Hispanic, Native American, Asian, or Pacific Islander population; said risk factor based on genetic mutations affecting (3-cell function comprises a defect on chromosome 12, gene HNF-1α (MODY3), a defect on chromosome 7, gene glucokinase (MODY2), a defect on chromosome 20, gene HNF-4α (MODY1), or a defect in mitochondrial DNA; said risk factor based on a genetic defect in insulin action comprises a genetic mutation leading to Type A insulin resistance, acanthosis nigricans, leprechaunism, Rabson-Mendenhall syndrome, lipoatrophic diabetes or condition, or a genetic mutation or mutations in the insulin receptor, IRS proteins, glucose transporters, PC-1, glucokinase, UCP-1, β3 adrenergic receptor gene; said risk factor based on the presence of excess adipose tissue or diagnosed obesity comprises central obesity; said risk factor identified through clinical chemistries or diagnostic testing signifying a pre-diabetic state comprises impaired glucose tolerance, impaired fasting glucose, or hyperglycemia relative to normoglycemia; said risk factor related to physiologic or endocrine changes associated with growth, development, comprises classification as a menopausal, pubescent, or aged individual; said risk factor related to diet or eating behaviors comprises consumption of high fat or high carbohydrate diets, experiencing prolonged fasting or starvation, or having anorexia nervosa or bulemia; said risk factor based on abnormal cardiovascular or blood lipid parameters comprises hypertension, HDL cholesterol levels ≤ 35 mg/dl and/or TG levels ≥ 250 mg/dl, or classification as having metabolic syndrome; said risk factor based on reproductive status comprises pregnancy, a history of gestational diabetes, or macrosomia; said risk factor attributable to muscle wasting comprises risk due to aging, starvation, exposure to anti-gravity environments, or paralysis resulting from spinal cord injury; said risk factor due to organ disease or dysfunction comprises liver cirrhosis or renal disease; said risk factor due to conditions resulting in metabolic disturbances comprises ketoacidosis; said risk factor due to endocrine disorders or endocrinopathies comprises hyperandrogenism, thyrotoxicosis, hyperthyroidism, insulinoma, glucagonoma, somatostatinoma, aldosteroma, Cushing's Syndrome, pheochromocytoma, acromegaly, hypercortisolemia; said risk factor due to a pathophysiologic state comprises infection, congenital rubella, cytomegalovirus, toxemia, uremia, sepsis, or trauma; said risk factor due to immune-mediated disease comprises "stiff man" syndrome or the production of anti-insulin receptor antibodies; said risk factor incurred due to drug or chemical exposure comprises treatment with insulin-resistance-inducing or hyperglycemia-inducing agents comprising glucocorticoids, cytokines, α-interferon, thyroid hormone, TNFα, thiazides, estrogen-containing products, β-blockers, nicotinic acid, serotonin receptor-targeted antipsychotics or antidepressants, vacor, diazoxide, dilantin, and HIV protease inhibitors; and said risk factor associated with having a genetic syndrome associated with diabetes comprises Down's Syndrome, Klinefelter's Syndrome, Wolfram's Syndrome, Freidreich's Syndrome, Huntington's chorea, Laurence-Moon-Biedl Syndrome, myotonic dystrophy, porphyria, Prader-Willi Syndrome, and Alzheimer's Disease.

5. The use according to claim 1 wherein said glycogen phosphorylase inhibitor is selected from the group consisting of:
(i) a compound of formula (I) 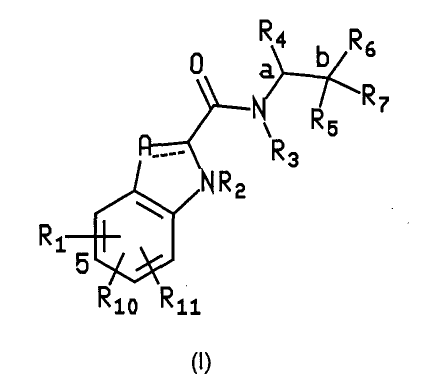 the stereoisomers and prodrugs thereof, and the pharmaceutically acceptable salts of said compounds, stereoisomers, and prodrugs, wherein:
the dotted line (---) is an optional bond;
A is -C(H)=, -C((C₁-C₄)alkyl)= or -C(halo)= when the dotted line (-) is a bond, or A is methylene or -CH((C₁-C₄)alkyl)- when the dotted line (-) is not a bond;
R₁, R₁₀ or R₁₁ are each independently H, halo, 4-, 6- or 7-nitro, cyano, (C₁-C₄)alkyl, (C₁-C₄)alkoxy, fluoromethyl, difluoromethyl or trifluoromethyl;
R₂ is H;
R₃ is H or (C₁-C₅)alkyl;
R₄ is H, methyl, ethyl, n-propyl, hydroxy(C₁-C₃)alkyl, (C₁-C₃)alkoxy(C₁-C₃)alkyl, phenyl(C₁-C₄)alkyl, phenylhydroxy(C₁-C₄)alkyl, phenyl(C₁-C₄)alkoxy(C₁-C₄)alkyl, thien-2- or -3-yl(C₁-C₄)alkyl or fur-2- or -3-yl(C₁-C₄)alkyl wherein said R₄ rings are mono-, di- or tri-substituted independently on carbon with H, halo, (C₁-C₄)alkyl, (C₁-C₄)alkoxy, trifluoromethyl, hydroxy, amino or cyano; or
R₄ is pyrid-2-, -3- or -4-yl(C₁-C₄)alkyl, thiazol-2-, -4- or -5-yl(C₁-C₄)alkyl, imidazol -1-,-2-, -4- or -5-yl(C₁-C₄)alkyl, pyrrol-2- or -3-yl(C₁-C₄)alkyl, oxazol-2-, -4- or - 5-yl-(C₁-C₄)alkyl, pyrazol-3-, -4- or -5-yl(C₁-C₄)alkyl, isoxazol-3-, -4- or -5-yl(C₁-C₄)alkyl, isothiazol-3-, -4- or -5-yl(C₁-C₄)alkyl, pyridazin-3- or -4-yl-(C₁-C₄)alkyl, pyrimidin-2-, -4-, -5- or -6-yl(C₁-C₄)alkyl, pyrazin-2- or -3-yl(C₁-C₄)alkyl or 1,3,5-triazin-2-yl(C₁-C₄)alkyl, wherein said preceding R₄ heterocycles are optionally mono- or di-substituted independently with halo, trifluoromethyl, (C₁-C₄)alkyl, (C₁-C₄)alkoxy, amino or hydroxy and said mono-or di-substituents are bonded to carbon;
R₅ is H, hydroxy, fluoro, (C₁-C₅)alkyl, (C₁-C₅)alkoxy, (C₁-C₆)alkanoyl, amino(C₁-C₄)alkoxy, mono-N- or di-N,N-(C₁-C₄)alkylamino(C₁-C₄)alkoxy, carboxy(C₁-C₄)alkoxy, (C₁-C₅)alkoxy-carbonyl(C₁-C₄)alkoxy, benzyloxycarbonyl(C₁-C₄)alkoxy, or carbonyloxy wherein said carbonyloxy is carbon-carbon linked with phenyl, thiazolyl, imidazolyl, 1H-indolyl, furyl, pyrrolyl, oxazolyl, pyrazolyl, isoxazolyl, isothiazolyl, pyridazinyl, pyrimidinyl, pyrazinyl or 1,3,5-triazinyl and wherein said preceding R₅ rings are optionally mono-substituted with halo, (C₁-C₄)alkyl, (C₁-C₄)alkoxy, hydroxy, amino or trifluoromethyl and said mono-substituents are bonded to carbon;
R₇ is H, fluoro or (C₁-C₅)alkyl; or
R₅ and R₇ can be taken together to be oxo;
R₆ is carboxy, (C₁-C₈)alkoxycarbonyl, C(O)NR₈R₉ or C(O)R₁₂,
wherein
R₈ is H, (C₁-C₃)alkyl, hydroxy or (C₁-C₃)alkoxy; and
R₉ is H, (C₁-C₈)alkyl, hydroxy, (C₁-C₈)alkoxy, methylene-perfluorinated(C₁-C₈)alkyl, phenyl, pyridyl, thienyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, pyridazinyl, pyrimidinyl, pyrazinyl, piperazinyl or 1,3,5-triazinyl wherein said preceding R₉ rings are carbon-nitrogen linked; or
R₉ is mono-, di- or tri-substituted (C₁-C₅)alkyl, wherein said substituents are independently H, hydroxy, amino, mono-N- or di-N,N-(C₁-C₅)alkylamino; or
R₉ is mono- or di-substituted (C₁-C₅)alkyl, wherein said substituents are independently phenyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl or 1,3,5-triazinyl
wherein the nonaromatic nitrogen-containing R₉ rings are optionally mono-substituted on nitrogen with (C₁-C₆)alkyl, benzyl, benzoyl or (C₁-C₆)alkoxycarbonyl and wherein the R₉ rings are optionally mono-substituted on carbon with halo, (C₁-C₄)alkyl, (C₁-C₄)alkoxy, hydroxy, amino, or mono-N- and di-N,N (C₁-C₅)alkylamino provided that no quaternized nitrogen is included and there are no nitrogen-oxygen, nitrogen-nitrogen or nitrogen-halo bonds;
R₁₂ is piperazin-1-yl, 4-(C₁-C₄)alkylpiperazin-1-yl, 4-formylpiperazin-1-yl, morpholino, thiomorpholino, 1-oxothiomorpholino, 1,1-dioxo-thiomorpholino, thiazolidin-3-yl, 1-oxo-thiazolidin-3-yl, 1,1-dioxo-thiazolidin-3-yl, 2-(C₁-C₆)alkoxycarbonylpyrrolidin-1-yl, oxazolidin-3-yl or 2(R)-hydroxymethylpyrrolidin-1-yl; or
R₁₂ is 3- and/or 4-mono-or di-substituted oxazetidin-2-yl, 2-, 4-, and/or 5- mono- or di-substituted oxazolidin-3-yl, 2-, 4-, and/or 5- mono- or di- substituted thiazolidin-3-yl, 2-, 4-, and/or 5- mono- or di- substituted 1-oxothiazolidin-3-yl, 2-, 4-, and/or 5- mono- or di- substituted 1,1-dioxothiazolidin-3-yl, 3- and/or 4-, mono- or di-substituted pyrrolidin-1-yl, 3-, 4- and/or 5-, mono-, di- or tri-substituted piperidin-1-yl, 3-, 4-, and/or 5- mono-, di-, or tri-substituted piperazin-1-yl, 3-substituted azetidin-1-yl, 4- and/or 5-, mono- or di-substituted 1,2-oxazinan-2-yl, 3-and/or 4-mono- or di-substituted pyrazolidin-1-yl, 4- and/or 5-, mono- or di-substituted isoxazolidin-2-yl, 4-and/or 5-, mono- and/or di-substituted isothiazolidin-2-yl wherein said R₁₂ substituents are independently H, halo, (C₁-C₅)-alkyl, hydroxy, amino, mono-N- or di-N,N-(C₁-C₅)alkylamino, formyl, oxo, hydroxyimino, (C₁-C₅)alkoxy, carboxy, carbamoyl, mono- N-or di-N,N-(C₁-C₄)alkylcarbamoyl, (C₁-C₄)alkoxyimino, (C₁-C₄)alkoxymethoxy, (C₁-C₆)alkoxycarbonyl, carboxy(C₁-C₅)alkyl or hydroxy(C₁-C₅)alkyl;
(ii) a compound of formula (II) 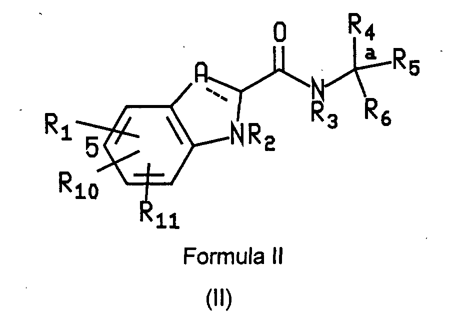 the stereoisomers and prodrugs thereof, and the pharmaceutically acceptable salts of said compounds, stereoisomers, and prodrugs,
wherein:
the dotted line (---) is an optional bond;
A is -C(H)=, -C((C₁-C₄)alkyl)=, -C(halo)= or -N=, when the dotted line (---) is a bond, or A is methylene or -CH((C₁-C₄)alkyl)-, when the dotted line (---) is not a bond;
R₁, R₁₀ or R₁₁ are each independently H, halo, cyano, 4-, 6-, or 7-nitro, (C₁-C₄)alkyl, (C₁-C₄)alkoxy, fluoromethyl, difluoromethyl or trifluoromethyl;
R₂ is H;
R₃ is H or (C₁-C₅)alkyl;
R₄ is H, methyl, ethyl, n-propyl, hydroxy(C₁-C₃)alkyl, (C₁-C₃)alkoxy(C₁-C₃)alkyl, phenyl(C₁-C₄)alkyl, phenylhydroxy(C₁-C₄)alkyl, (phenyl)((C₁-C₄)-alkoxy)(C₁-C₄)alkyl, thien-2- or -3-yl(C₁-C₄)alkyl or fur-2- or -3-yl(C₁-C₄)alkyl wherein said R₄ rings are mono-, di- or tri-substituted independently on carbon with H, halo, (C₁-C₄)alkyl, (C₁-C₄)alkoxy, trifluoromethyl, hydroxy, amino, cyano or 4,5-dihydro-1H-imidazol-2-yl; or
R₄ is pyrid-2-, -3- or -4-yl(C₁-C₄)alkyl, thiazol-2-, -4- or -5-yl(C₁-C₄)alkyl, imidazol-2-, -4- or -5-yl(C₁-C₄)alkyl, pyrrol-2- or -3-yl(C₁-C₄)alkyl, oxazol-2-, -4- or -5- yl(C₁-C₄)alkyl, pyrazol-3-, -4- or -5-yl(C₁-C₄)alkyl, isoxazol-3-, -4- or -5-yl(C₁-C₄)alkyl, isothiazol-3-, -4- or -5-yl(C₁-C₄)alkyl, pyridazin-3- or -4-yl(C₁-C₄)alkyl, pyrimidin-2-, -4-, -5- or -6-yl(C₁-C₄)alkyl, pyrazin-2- or -3-yl(C₁-C₄)alkyl, 1,3,5-triazin-2-yl(C₁-C₄)alkyl or indol-2-(C₁-C₄)alkyl, wherein said preceding R₄ heterocycles are optionally mono- or di-substituted independently with halo, trifluoromethyl, (C₁-C₄)alkyl, (C₁-C₄)alkoxy, amino, hydroxy or cyano and said substituents are bonded to carbon; or
R₄ is R₁₅-carbonyloxymethyl, wherein said R₁₅ is phenyl, thiazolyl, imidazolyl, 1H-indolyl, furyl, pyrrolyl, oxazolyl, pyrazolyl, isoxazolyl, isothiazolyl, pyridyl, pyridazinyl, pyrimidinyl, pyrazinyl or 1,3,5-triazinyl and wherein said preceding R₁₅ rings are optionally mono- or di-substituted independently with halo, amino, hydroxy, (C₁-C₄)alkyl, (C₁-C₄)alkoxy or trifluoromethyl and said mono- or di-substituents are bonded to carbon;
R₅ is H, methyl, ethyl, n-propyl, hydroxymethyl or hydroxyethyl;
R₆ is carboxy, (C₁-C₈)alkoxycarbonyl, benzyloxycarbonyl, C(O)NR₈R₉ or C(O)R₁₂
wherein
R₈ is H, (C₁-C₆)alkyl, cyclo(C₃-C₆)alkyl, cyclo(C₃-C₆)alkyl(C₁-C₅)alkyl, hydroxy or (C₁-C₈)alkoxy; and
R₉ is H, cyclo(C₃-C₈)alkyl, cyclo(C₃-C₈)alkyl(C₁-C₅)alkyl, cyclo(C₄-C₇)alkenyl, cyclo(C₃-C₇)alkyl(C₁-C₅)alkoxy, cyclo(C₃-C₇)alkyloxy, hydroxy, methyleneperfluorinated(C₁-C₈)alkyl, phenyl, or a heterocycle wherein said heterocycle is pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, pyridinyl, piperidinyl, morpholinyl, pyridazinyl, pyrimidinyl, pyrazinyl, piperazinyl, 1,3,5-triazinyl, benzothiazolyl, benzoxazolyl, benzimidazolyl, thiochromanyl or tetrahydrobenzothiazolyl wherein said heterocycle rings are carbon-nitrogen linked; or
R₉ is (C₁-C₆)alkyl or (C₁-C₈)alkoxy wherein said (C₁-C₆)alkyl or (C₁-C₈)alkoxy is optionally monosubstituted with cyclo(C₄-C₇)alken-1-yl, phenyl, thienyl, pyridyl, furyl, pyrrolyl, pyrrolidinyl, oxazolyl, thiazolyl, imidazolyl, pyrazolyl, pyrazolinyl, pyrazolidinyl, isoxazolyl, isothiazolyl, pyranyl, piperidinyl, morpholinyl, thiomorpholinyl, 1-oxothiomorpholinyl, 1,1-dioxothiomorpholinyl, pyridazinyl, pyrimidinyl, pyrazinyl, piperazinyl, 1,3,5-triazinyl or indolyl and wherein said (C₁-C₆)alkyl or (C₁-C₈)alkoxy are optionally additionally independently mono- or di-substituted with halo, hydroxy, (C₁-C₅)alkoxy, amino, mono-N- or di-N,N-(C₁-C₅)alkylamino, cyano, carboxy, or (C₁-C₄)alkoxycarbonyl; and
wherein the R₉ rings are optionally mono- or di-substituted independently on carbon with halo, (C₁-C₄)alkyl, (C₁-C₄)alkoxy, hydroxy, hydroxy(C₁-C₄)alkyl, amino(C₁-C₄)alkyl, mono-N- or di-N,N-(C₁-C₄)alkylamino(C₁-C₄)alkyl, (C₁-C₄)alkoxy(C₁-C₄)alkyl, amino, mono-N- or di-N,N-(C₁-C₄)alkylamino, cyano, carboxy, (C₁-C₅)alkoxycarbonyl, carbamoyl, formyl or trifluoromethyl and said R₉ rings may optionally be additionally mono- or di-substituted independently with (C₁-C₅)alkyl or halo;
R₁₂ is morpholino, thiomorpholino, 1-oxothiomorpholino, 1,1-dioxothiomorpholino, thiazolidin-3-yl, 1-oxothiazolidin-3-yl, 1,1-dioxothiazolidin-3-yl, pyrrolidin-1-yl, piperidin-1-yl, piperazin-1-yl, piperazin-4-yl, azetidin-1-yl, 1,2-oxazinan-2-yl, pyrazolidin-1-yl, isoxazolidin-2-yl, isothiazolidin-2-yl, 1,2-oxazetidin-2-yl, oxazolidin-3-yl, 3,4-dihydroisoquinolin-2-yl, 1,3-dihydroisoindol-2-yl, 3,4-dihydro-2H-quinol-1-yl, 2,3-dihydro-benzo[1,4]oxazin-4-yl, 2,3-dihydro-benzo[1,4]-thiazine-4-yl, 3,4-dihydro-2H-quinoxalin-1-yl, 3,4-dihydro-benzo[c][1,2]oxazin-1-yl, 1,4-dihydrobenzo[d][1,2]oxazin-3-yl, 3,4-dihydro-benzo[e][1,2]-oxazin-2-yl, 3H-benzo[d]isoxazol-2-yl, 3H-benzo[c]isoxazol-1-yl or azepan-1-yl,
wherein said R₁₂ rings are optionally mono-, di- or tri-substituted independently with halo, (C₁-C₅)alkyl, (C₁-C₅)alkoxy, hydroxy, amino, mono-N- or di- N,N-(C₁-C₅)alkylamino, formyl, carboxy, carbamoyl, mono-N- or di-N,N-(C₁-C₅)alkylcarbamoyl, (C₁-C₆)alkoxy(C₁-C₃)alkoxy, (C₁-C₅)alkoxycarbonyl, benzyloxycarbonyl, (C₁-C₅)alkoxycarbonyl(C₁-C₅)alkyl, (C₁-C₄)alkoxycarbonylamino, carboxy(C₁-C₅)alkyl, carbamoyl(C₁-C₅)alkyl, mono-N- or di-N,N-(C₁-C₅)alkylcarbamoyl(C₁-C₅)alkyl, hydroxy(C₁-C₅)alkyl, (C₁-C₄)alkoxy(C₁-C₄)alkyl, amino(C₁-C₄)alkyl, mono-N- or di-N,N-(C₁-C₄)alkylamino(C₁-C₄)alkyl, oxo, hydroxyimino or (C₁-C₆)alkoxyimino and wherein no more than two substituents are selected from oxo, hydroxyimino or (C₁-C₆)alkoxyimino and oxo, hydroxyimino or (C₁-C₆)alkoxyimino are on nonaromatic carbon; and
wherein said R₁₂ rings are optionally additionally mono- or di-substituted independently with (C₁-C₅)alkyl or halo;
(iii) a compound of formula (III) 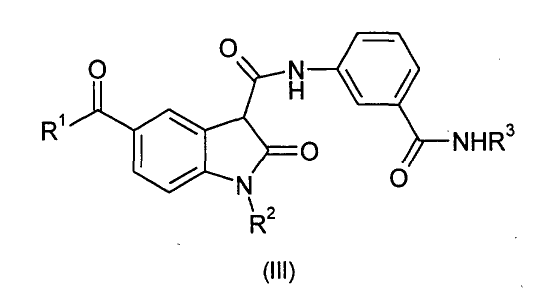 the stereoisomers and prodrugs thereof, and the pharmaceutically acceptable salts of said compounds, stereoisomers, and prodrugs,
wherein:
R¹ is (C₁-C₄)alkyl, (C₃-C₇)cycloalkyl, phenyl or phenyl independently substituted with up to three (C₁-C₄)alkyl, (C₁-C₄)alkoxy or halogen;
R² is (C₁-C₄)alkyl optionally substituted with up to three fluoro atoms; and
R³ is (C₃-C₇)cycloalkyl; phenyl; phenyl substituted at the para position with (C₁-C₄)alkyl, halo or trifluoromethyl; phenyl substituted at the meta position with fluoro; or phenyl substituted at the ortho position with fluoro; and
(iv) a compound of formula (IV) 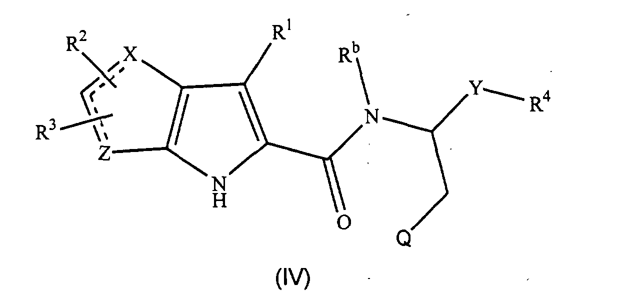 the stereoisomers and prodrugs thereof, and the pharmaceutically acceptable salts of said compounds, stereoisomers, and prodrugs,
wherein:
Q is aryl, substitued aryl, heteroaryl, or substitued heteroaryl;
each Z and X are independently (C, CH or CH₂), N, O or S;
X¹ is NR^{a}, -CH₂-, O or S;
each - - - - is independently a bond or is absent, provided that both - - - - are not simlutaneously bonds;
R¹ is hydrogen, halogen, -OC₁-C₈alkyl, -SC₁₋-C₈alkyl,
-C₁-C₈alkyl, -CF₃, -NH₂, -NHC₁-C₈alkyl, -N(C₁-C₈alkyl)₂, -NO₂, -CN,
-CO₂H, -CO₂C₁-C₈alkyl, -C₂-C₈alkenyl, or -C₂-C₈alkynyl;
each R^{a} and R^{b} is independently hydrogen or -C₁-C₈alkyl;
Y is 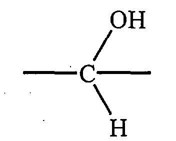 or absent;
R² and R³ are independently hydrogen, halogen, -C₁-C₈alkyl, -CN, -C≡C-Si(CH₃)₃, -OC₁-C₈alkyl, -SC₁-C₈alkyl, -CF₃, -NH₂, -NHC₁-C₈alkyl, -N(C₁-C₈alkyl)₂, -NO₂, -CO₂H, -CO₂C₁-C₈alkyl, -C₂-C₈alkenyl, or -C₂-C₈alkynyl, or R² and R³ together with the atoms on the ring to which they are attached form a five or six membered ring containing from 0 to 3 heteroatoms and from 0 to 2 double bonds;
R⁴ is -C(=O)-A;
A is -NR^{d}R^{d}, -NR^{a}CH₂CH₂OR^{a}, 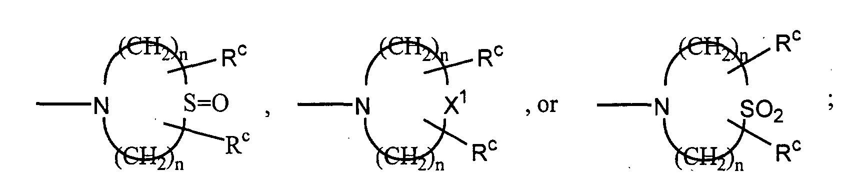
each R^{d} is independently hydrogen, C₁-C₈alkyl, C₁-C₈alkoxy, aryl, substituted aryl, heteroaryl, or substituted heteroaryl;
each R^{c} is independently hydrogen, -C(=O)OR^{a}, -OR^{a}, -SR^{a}, or -NR^{a}R^{a;} and
each n is independently 1-3.

6. The use according to claim 5 wherein said glycogen phosphorylase inhibitor is a compound of formula (I), a stereoisomer or prodrug thereof, or a pharmaceutically acceptable salt of said compound, stereoisomer, or prodrug.

7. The use according to claim 6 wherein said compound of formula (I) is selected from the group consisting of:
5-chloro-1H-indole-2-carboxylic acid-[(1S)-((R)-hydroxy-dimethylcarbamoylmethyl)-2-phenyl-ethyl]-amide;
5,6-dichloro-1H-indole-2-carboxylic acid-{(1S)-[(R)-hydroxy-(methoxy-methylcarbamoyl)-methyl]-2-phenyl-ethyl}-amide;
5-chloro-1H-indole-2-carboxylic acid-{(1S)-[(R)-hydroxy-(methoxy-methylcarbamoyl)-methyl]-2-phenyl-ethyl}-amide;
5-chloro-1H-indole-2-carboxylic acid-((1S)-{(R)-hydroxy-[(2-hydroxy-ethyl)-methyl-carbamoyl]-methyl}2-phenyl-ethyl}-amide;
5-chloro-1H-indole-2-carboxylic acid-((1S)-{(R)-hydroxy-[methyl-(2-pyridin-2-yl-ethyl)-carbamoyl]-methyl}-2-phenyl-ethyl)-amide;
5-chloro-1H-indole-2-carboxylic acid-[(1S)-benzyl-(2R)-hydroxy-3-(4-methylpiperazin-1-yl)-3-oxo-propyl]-amide;
5-chloro-1H-indole-2-carboxylic acid-[(1S)-benzyl-(2R)-hydroxy-3-(3-hydroxyazetidin-1-yl)-3-oxo-propyl]-amide;
5-chloro-1H-indole-2-carboxylic acid-((1S)-benzyl-(2R)-hydroxy-3-isoxazolidin-2-yl-3-oxo-propyl)-amide;
5-chloro-1H-indole-2-carboxylic acid-((1S)-benzyl-(2R)-hydroxy-3-[1,2]oxazinan-2-yl-3-oxo-propyl)-amide;
5-chloro-1H-indole-2-carboxylic acid-[(1S)-benzyl-(2R)-hydroxy-3-((3S)-hydroxy-pyrrolidin-1-yl)-3-oxo-propyl]-amide;
5-chloro-1H-indole-2-carboxylic acid-[(1S)-benzyl-3-((3S,4S)-dihydroxypyrrolidin-1-yl)-(2R)-hydroxy-3-oxo-propyl]-amide;
5-chloro-1H-indole-2-carboxylic acid-[(1S)-benzyl-3-((3R,4S)-dihydroxypyrrolidin-1-yl)-(2R)-hydroxy-3-oxo-propyl]-amide; and
5-chloro-1H-indole-2-carboxylic acid-((1S)-benzyl-(2R)-hydroxy-3-morpholin-4-yl-3-oxo-propyl)-amide;
the stereoisomers and prodrugs thereof, and the pharmaceutically acceptable salts of said compounds, stereoisomers, and prodrugs.

8. The use according to claim 5 wherein said glycogen phosphorylase inhibitor is a compound of formula (II), a stereoisomer or prodrug thereof, or a pharmaceutically acceptable salt of said compound, stereoisomer, or prodrug.

9. The use according to claim 8 wherein said compound of formula (II) is selected from the group consisting of:
5-chloro-1H-indole-2-carboxylic acid-[(1S)-benzyl-2-(3-hydroxyiminopyrrolidin-1-yl)-2-oxo-ethyl]-amide;
5-chloro-1H-indole-2-carboxylic acid-[2-(cis-3,4-dihydroxy-pyrrolidin-1 -yl)-2-oxo-ethyl]-amide;
5-chloro-1H-indole-2-carboxylic acid-[(1S)-benzyl-2-(cis-3,4-dihydroxypyrrolidin-1-yl)-2-oxo-ethyl]-amide;
5-chloro-1H-indole-2-carboxylic acid-[2-(1,1-dioxo-thiazolidin-3-yl)-2-oxo-ethyl]-amide;
5-chloro-1H-indole-2-carboxylic acid-(2-oxo-2-thiazolidin-3-yl-ethyl)-amide;
5-chloro-1H-indole-2-carboxylic acid-(1S)-(4-fluoro-benzyl)-2-(4-hydroxypiperidin-1-yl)-2-oxo-ethyl]-amide;
5-chloro-1H-indole-2-carboxylic acid-[(1S)-benzyl-2-((3RS)-hydroxy-piperidin-1-yl)-2-oxo-ethyl]-amide;
5-chloro-1H-indole-2-carboxylic acid-2-oxo-2-((1RS)-oxo-1-thiazolidin-3-yl)-ethyl]-amide;
5-chloro-1H-indole-2-carboxylic acid-[(1S)-(2-fluoro-benzyl)-2-(4-hydroxypiperidin-1-yl)-2-oxo-ethyl]-amide;
5-chloro-1H-indole-2-carboxylic acid-(1S)-benzyl-2-(3-hydroxy-azetidin-1-yl)-2-oxo-ethyl]-amide;
5-chloro-1H-indole-2-carboxylic acid-[(1S)-benzyl-2-(3-hydroxyimino-azetidin-1-yl)-2-oxo-ethyl]-amide; and
5-chloro-1H-indole-2-carboxylic acid-[(1S)-benzyl-2-(4-hydroxyimino-piperidin-1-yl)-2-oxo-ethyl]-amide;
the stereoisomers and prodrugs thereof, and the pharmaceutically acceptable salts of said compounds, stereoisomers, and prodrugs.

10. The use according to claim 5 wherein said glycogen phosphorylase inhibitor is a compound of formula (III), a stereoisomer or prodrug thereof, or a pharmaceutically acceptable salt of said compound, stereoisomer, or prodrug.

11. The use according to claim 10 wherein said compound of formula (III) is selected from the group consisting of:
5-acetyl-1-ethyl-2-oxo-2,3-dihydro-1H-indole-3-carboxylic acid (3-p-tolylcarbamoyl-phenyl)-amide;
5-acetyl-1-ethyl-2-oxo-2,3-dihydro-1H-indole-3-carboxylic acid (3-(4-bromophenylcarbamoyl-phenyl)-amide; and
5-acetyl-1-ethyl-2-oxo-2,3-dihydro-1H-indole-3-carboxylic acid (3-phenylcarbamoyl-phenyl)-amide;
the stereoisomers and prodrugs thereof, and the pharmaceutically acceptable salts of said compounds, stereoisomers, and prodrugs.

12. The use according to claim 5 wherein said glycogen phosphorylase inhibitor is a compound of formula (IV), a stereoisomer or prodrug thereof, or a pharmaceutically acceptable salt of said compound, stereoisomer, or prodrug.

13. The use according to claim 12 wherein said compound of formula (IV) is selected from the group consisting of:
2-chloro-6H-thieno[2,3-b]pyrrole-5-carboxylic acid-[(1S)-benzyl-2-((3R,4S)-dihydroxy-pyrrolidin-1-yl)-2-oxo-ethyl]-amide;
(±)-2-bromo-4H-furo[3,2-b]pyrrole-5-carboxylic acid-[1-benzyl-2-((3R,4S)-dihydroxy-pyrrolidin-1-yl)-2-oxo-ethyl]-amide;
2-bromo-4H-thieno[3,2-b]pyrrole-5-carboxylic acid-[(1S)-benzyl-2-((3R,4S)-dihydroxy-pyrrolidin-1-yl)-2-oxo-ethyl]-amide;
2-chloro-6H-thieno[2,3-b]pyrrole-5-carboxylic acid-[(1S)-benzyl-2-morpholin-4-yl-2-oxo-ethyl]-amide;
2-chloro-6H-thieno[2,3-b]pyrrole-5-carboxylic acid-[(1S)-benzyl-2-(1,1dioxo-1-thiazolidin-3-yl)-2-oxo-ethyl]-amide;
2-chloro-4H-furo[3,2-b]pyrrole-5-carboxylic acid-[(1S)-benzyl-2-((3R,4S)-dihydroxy-pyrrolidin-1-yl)-2-oxo-ethyl]-amide;
2-chloro-4H-furo[3,2-b]pyrrole-5-carboxylic acid-[(1S)-benzyl-3-((3R,4S)-dihydroxy-pyrrolidin-1-yl)-(2R)hydroxy-3-oxo-propyl]-amide;
2-chloro-4H-thieno[3,2-b]pyrrole-5-carboxylic acid-[(1S)-benzyl-2-((3R,4S)-dihydroxy-pyrrolidin-1-yl)-2-oxo-ethyl]-amide; and
3-methyl-4H-thieno[3,2-b]pyrrole-5-carboxylic acid-[(1S)-benzyl-2-((3R,4S)-dihydroxy-pyrrolidin-1-yl)-2-oxo-ethyl]-amide;
the stereoisomers and prodrugs thereof, and the pharmaceutically acceptable salts of said compounds, stereoisomers, and prodrugs.

14. Use of a glycogen phosphorylase inhibitor and a non-glycogen phosphorylase inhibiting anti-diabetic agent in the manufacture of a medicament for treating prophylactically an individual in whom Type 2 diabetes mellitus has not yet presented, but in whom there is an increased risk of developing such condition.

15. The use according to claim 14 wherein said glycogen phosphorylase inhibitor is selected from the group consisting of:
5-chloro-1H-indole-2-carboxylic acid-[(1S)-((R)-hydroxy-dimethylcarbamoylmethyl)-2-phenyl-ethyl]-amide;
5,6-dichloro-1H-indole-2-carboxylic acid-{(1S)-[(R)-hydroxy-(methoxy-methylcarbamoyl)-methyl]-2-phenyl-ethyl}-amide;
5-chloro-1H-indole-2-carboxylic acid-{(1S)-[(R)-hydroxy-(methoxy-methylcarbamoyl)-methyl]-2-phenyl-ethyl}amide;
5-chloro-1H-indole-2-carboxylic acid-((1S)-{(R)-hydroxy-[(2-hydroxy-ethyl)-methyl-carbamoyl]-methyl}-2-phenyl-ethyl}amide;
5-chloro-1H-indole-2-carboxylic acid-((1S)-{(R)-hydroxy-[methyl-(2-pyridin-2-yl-ethyl)-carbamoyl]-methyl}-2-phenyl-ethyl)-amide;
5-chloro-1H-indole-2-carboxylic acid-[(1S)-benzyl-(2R)-hydroxy-3-(4-methylpiperazin-1-yl)-3-oxo-propyl]-amide;
5-chloro-1H-indole-2-carboxylic acid-[(1S)-benzyl-(2R)-hydroxy-3-(3-hydroxyazetidin-1 -yl)-3-oxo-propyl]-amide;
5-chloro-1H-indole-2-carboxylic acid-((1S)-benzyl-(2R)-hydroxy-3-isoxazoiidin-2-yl-3-oxo-propyl)-amide;
5-chloro-1H-indole-2-carboxylic acid-((1S)-benzyl-(2R)-hydroxy-3-[1,2]oxazinan-2-yl-3-oxo-propyl)-amide;
5-chloro-1H-indole-2-carboxylic acid-[(1S)-benzyl-(2R)-hydroxy-3-((3S)-hydroxy-pyrrolidin-1-yl)-3-oxo-propyl]-amide;
5-chloro-1H-indole-2-carboxylic acid-[(1S)-benzyl-3-((3S,4S)-dihydroxypyrrolidin-1-yl)-(2R)-hydroxy-3-oxo-propyl]-amide;
5-chloro-1H-indole-2-carboxylic acid-[(1S)-benzyl-3-((3R,4S)-dihydroxypyrrolidin-1-yl)-(2R)-hydroxy-3-oxo-propyl]-amide;
5-chloro-1H-indole-2-carboxylic acid-((1S)-benzyl-(2R)-hydroxy-3-morpholin-4-yl-3-oxo-propyl)-amide;
5-chloro-1H-indole-2-carboxylic acid-[(1 S)-benzyl-2-(3-hydroxyiminopyrrolidin-1-yl)-2-oxo-ethyl]-amide;
5-chloro-1H-indole-2-carboxylic acid-[2-(cis-3,4-dihydroxy-pyrrolidin-1-yl)-2-oxo-ethyl]-amide;
5-chloro-1H-indole-2-carboxylic acid-[(1S)-benzyl-2-(cis-3,4-dihydroxypyrrolidin-1-yl)-2-oxo-ethyl]-amide;
5-chloro- 1H-indole-2-carboxylic acid-[2-(1,1-dioxo-thiazolidin-3-yl)-2-oxoethyl]-amide;
5-chloro-1H-indole-2-carboxylic acid-(2-oxo-2-thiazolidin-3-yl-ethyl)-amide;
5-chloro-1H-indole-2-carboxylic acid-(1S)-(4-fluoro-benzyl)-2-(4-hydroxypiperidin-1-yl)-2-oxo-ethyl]-amide;
5-chloro-1H-indole-2-carboxylic acid-[(1S)-benzyl-2-((3RS)-hydroxy-piperidin-1-yl)-2-oxo-ethyl]-amide;
5-chloro-1H-indole-2-carboxylic acid-2-oxo-2-((1RS)-oxo-1-thiazolidin-3-yl)-ethyl]-amide;
5-chloro-1H-indole-2-carboxylic acid-[(1S)-(2-fluoro-benzyl)-2-(4-hydroxypiperidin-1-yl)-2-oxo-ethyl]-amide;
5-chloro-1H-indole-2-carboxylic acid-(1S)-benzyl-2-(3-hydroxy-azetidin-1-yl)-2-oxo-ethyl]-amide;
5-chloro-1H-indole-2-carboxylic acid-[(1S)-benzyl-2-(3-hydroxyimino-azetidin-1 -yl)-2-oxo-ethyl]-amide;
5-chloro-1H-indole-2-carboxylic acid-[(1S)-benzyl-2-(4-hydroxyimino-piperidin-1-yl)-2-oxo-ethyl]-amide;
5-acetyl-1-ethyl-2-oxo-2,3-dihydro-1H-indole-3-carboxylic acid (3-p-tolylcarbamoyl-phenyl)-amide;
5-acetyl-1-ethyl-2-oxo-2,3-dihydro-1H-indole-3-carboxylic acid (3-(4-bromophenylcarbamoyl-phenyl)-amide;
5-acetyl-1-ethyl-2-oxo-2,3-dihydro-1H-indole-3-carboxylic acid (3-phenylcarbamoyl-phenyl)-amide;
2-chloro-6H-thieno[2,3-b]pyrrole-5-carboxylic acid-[(1S)-benzyl-2-((3R,4S)-dihydroxy-pyrrolidin-1-yl)-2-oxo-ethyl]-amide;
(±)-2-bromo-4H-furo[3,2-b]pyrrole-5-carboxylic acid-[1-benzyl-2-((3R,4S)-dihydroxy-pyrrolidin-1-yl)-2-oxo-ethyl]-amide;
2-bromo-4H-thieno[3,2-b]pyrrole-5-carboxylic acid-[(1S)-benzyl-2-((3R,4S)-dihydroxy-pyrrolidin-1-yl)-2-oxo-ethyl]-amide;
2-chloro-6H-thieno[2,3-b]pyrrole-5-carboxylic acid-[(1S)-benzyl-2-morpholin-4-yl-2-oxo-ethyl]-amide;
2-chloro-6H-thieno[2,3-b]pyrrole-5-carboxylic acid-[(1S)-benzyl-2-(1,1dioxo-1-thiazolidin-3-yl)-2-oxo-ethyl]-amide;
2-chloro-4H-furo[3,2-b]pyrrole-5-carboxylic acid-[(1S)-benzyl-2-((3R,4S)-dihydroxy-pyrrolidin-1-yl)-2-oxo-ethyl]-amide;
2-chloro-4H-furo[3,2-b]pyrrole-5-carboxylic acid-[(1S)-benzyl-3-((3R,4S)-dihydroxy-pyrrolidin-1-yl)-(2R)hydroxy-3-oxo-propyl]-amide;
2-chloro-4H-thieno[3,2-b]pyrrole-5-carboxylic acid-[(1S)-benzyl-2-((3R,4S)-dihydroxy-pyrrolidin-1-yl)-2-oxo-ethyl]-amide; and
3-methyl-4H-thieno[3,2-b]pyrrole-5-carboxylic acid-[(1S)-benzyl-2-((3R,4S)-dihydroxy-pyrrolidin-1-yl)-2-oxo-ethyl]-amide; the stereoisomers and prodrugs thereof, and the pharmaceutically acceptable salts of said compounds, stereoisomers, and prodrugs; and
said non-glycogen phosphorylase inhibiting anti-diabetic agent is selected from the group consisting of D-chiroinositol; insulin or an insulin analog, GLP-1 (7-37) (insulinotropin) or GLP-1 (7-36)-NH₂, an α-glucosidase inhibitor, a glitazone and/or an insulin sensitizer, a sulfonylurea or an analog thereof, a biguanide, an α₂-antagonist or imidazoline, an insulin secretagogue, an aldose reductase inhibitor, a fatty acid oxidation inhibitor, a β-agonist, a phosphodiesterase inhibitor, a lipid-lowering agent, a vanadate or vanadium complex, an amylin antagonist, a glucagon antagonist, a growth hormone secretagogue, a gluconeogenesis inhibitor, a somatostatin analog, an antilipolytic agent; a lipoxygenase inhibitor; an insulin signaling agonist; an insulin mimetic; a PTP1B inhibitor; an insulin degrading enzyme inhibitor; and a glycogen synthase kinase inhibitor.

16. The use according to claim 15 wherein said insulin analog is LysPro insulin; said α-glucosidase inhibitor is selected from the group consisting of acarbose, voglibose, miglitol, emiglitate, camiglibose, MDL-25,637, and MDL-73,945; said glitazone and/or insulin sensitizer is selected from the group consisting of ciglitazone, pioglitazone, englitazone, troglitazone, darglitazone, rosiglitazone, JTT-501, MCC-555, and MX 6054; said sulfonylurea or analog thereof is selected from the group consisting of chlorpropamide, glibenclamide, tolbutamide, tolazamide, acetohexamide, glipizide, glimepiride, repaglinide, and meglitinide; said biguanide is selected from the group consisting of metformin, phenformin, and buformin; said α₂-antagonist or imidazoline is selected from the group consisting of midaglazole, isaglidole, deriglidole, idazoxan, efaroxan, and fluparoxan; said insulin secretagogue is selected from the group consisting of linogliride, A-4166, exendin-4, and BTS-67582; said aldose reductase inhibitor is selected from the group consisting of epalrestat, sorbinil, tolrestat, zenarestat, and zopolrestat; said fatty acid oxidation inhibitor is selected from the group consisting of clomoxir and etomoxir; said β-agonist is selected from the group consisting of BRL-35135, BRL-37344, TAK-37344, AZ 40140, and CL 316,243; said phosphodiesterase inhibitor is L-386,398; said lipid-lowering agent is benfluorex; said vanadate or vanadium complex is selected from the group consisting of naglivan and peroxovanadium complexes; said gluconeogenesis inhibitor is a glucose-6-phosphatase inhibitor or GP 3034; said antilipolytic agent is selected from the group consisting of nicotinic acid, acipimox, and WAG 994; said amylin antagonist is pramlintide or AC-137; said glucagon antagonist is BAY 27-9955; said lipoxygenase inhibitor is masoprocol; and said insulin signaling agonist is L-783281.

17. Use of a glycogen phosphorylase inhibitor and an anti-obesity agent in the manufacture of a medicament for treating prophylactically an individual in whom Type 2 diabetes mellitus has not yet presented, but in whom there is an increased risk of developing such condition.

18. The use according to claim 17 wherein said glycogen phosphorylase inhibitor is selected from the group consisting of:
5-chloro-1H-indole-2-carboxylic acid-[(1S)-((R)-hydroxy-dimethylcarbamoylmethyl)-2-phenyl-ethyl]-amide;
5,6-dichloro-1H-indole-2-carboxylic acid-{(1S)-[(R)-hydroxy-(methoxy-methylcarbamoyl)-methyl]-2-phenyl-ethyl}-amide;
5-chloro-1H-indole-2-carboxylic acid-{(1S)-[(R)-hydroxy-(methoxy-methylcarbamoyl)-methyl]-2-phenyl-ethyl}-amide;
5-chloro-1H-indole-2-carboxylic acid-((1S)-{(R)-hydroxy-[(2-hydroxy-ethyl)-methyl-carbamoyl]-methy}-2-phenyl-ethyl}-amide;
5-chloro-1H-indole-2-carboxylic acid-((1S)-{(R)-hydroxy-[methyl-(2-pyridin-2-yl-ethyl)-carbamoyl]-methyl}2-phenyl-ethyl)-amide;
5-chloro-1H-indole-2-carboxylic acid-[(1S)-benzyl-(2R)-hydroxy-3-(4-methylpiperazin-1-yl)-3-oxo-propyl]-amide;
5-chloro-1H-indole-2-carboxylic acid-[(1S)-benzyl-(2R)-hydroxy-3-(3-hydroxyazetidin-1-yl)-3-oxo-propyl]-amide;
5-chloro-1H-indole-2-carboxylic acid-((1S)-benzyl-(2R)-hydroxy-3-isoxazolidin-2-yl-3-oxo-propyl)-amide;
5-chloro-1H-indole-2-carboxylic acid-((1S)-benzyl-(2R)-hydroxy-3-[1,2]oxazinan-2-yl-3-oxo-propyl)-amide;
5-chloro-1H-indole-2-carboxylic acid-[(1S)-benzyl-(2R)-hydroxy-3-((3S)-hydroxy-pyrrolidin-1-yl)-3-oxo-propyl]-amide;
5-chloro-1H-indole-2-carboxylic acid-[(1S)-benzyl-3-((3S,4S)-dihydroxypyrrolidin-1-yl)-(2R)-hydroxy-3-oxo-propyl]-amide;
5-chloro-1 H-indole-2-carboxylic acid-[(1S)-benzyl-3-((3R,4S)-dihydroxypyrrolidin-1-yl)-(2R)-hydroxy-3-oxo-propyl]-amide;
5-chloro-1H-indole-2-carboxylic acid-((1S)-benzyl-(2R)-hydroxy-3-morpholin-4-yl-3-oxo-propyl)-amide;
5-chloro-1H-indole-2-carboxylic acid-[(1S)-benzyl-2-(3-hydroxyiminopyrrolidin-1-yl)-2-oxo-ethyl]-amide;
5-chloro-1H-indole-2-carboxylic acid-[2-(cis-3,4-dihydroxy-pyrrolidin-1-yl)-2-oxo-ethyl]-amide;
5-chloro-1H-indole-2-carboxylic acid-[(1S)-benzyl-2-(cis-3,4-dihydroxypyrrolidin-1-yl)-2-oxo-ethyl]-amide;
5-chloro-1H-indole-2-carboxylic acid-[2-(1,1-dioxo-thiazolidin-3-yl)-2-oxo-ethyl]-amide;
5-chloro-1H-indole-2-carboxylic acid-(2-oxo-2-thiazolidin-3-yl-ethyl)-amide;
5-chloro-1 H-indole-2-carboxylic acid-(1S)-(4-fluoro-benzyl)-2-(4-hydroxypiperidin-1-yl)-2-oxo-ethyl]-amide;
5-chloro-1H-indole-2-carboxylic acid-[(1S)-benzyl-2-((3RS)-hydroxy-piperidin-1-yl)-2-oxo-ethyl]-amide;
5-chloro-1H-indole-2-carboxylic acid-2-oxo-2-((1RS)-oxo-1-thiazolidin-3-yl)-ethyl]-amide;
5-chloro-1H-indole-2-carboxylic acid-[(1S)-(2-fluoro-benzyl)-2-(4-hydroxypiperidin-1-yl)-2-oxo-ethyl]-amide;
5-chloro-1H-indole-2-carboxylic acid-(1S)-benzyl-2-(3-hydroxy-azetidin-1-yl)-2-oxo-ethyl]-amide;
5-chloro-1H-indole-2-carboxylic acid-[(1S)-benzyl-2-(3-hydroxyimino-azetidin-1-yl)-2-oxo-ethyl]-amide;
5-chloro-1H-indole-2-carboxylic acid-[(1S)-benzyl-2-(4-hydroxyimino-piperidin-1-yl)-2-oxo-ethyl]-amide;
5-acetyl-1-ethyl-2-oxo-2,3-dihydro-1H-indole-3-carboxylic acid (3-p-tolylcarbamoyl-phenyl)-amide;
5-acetyl-1-ethyl-2-oxo-2,3-dihydro-1 H-indole-3-carboxylic acid (3-(4-bromophenylcarbamoyl-phenyl)-amide;
5-acetyl-1-ethyl-2-oxo-2,3-dihydro-1H-indole-3-carboxylic acid (3-phenylcarbamoyl-phenyl)-amide;
2-chloro-6H-thieno[2,3-b]pyrrole-5-carboxylic acid-[(1S)-benzyl-2-((3R,4S)-dihydroxy-pyrrolidin-1 -yl)-2-oxo-ethyl]-amide;
(±)-2-bromo-4H-furo[3,2-b]pyrrole-5-carboxylic acid-[1-benzyl-2-((3R,4S)-dihydroxy-pyrrolidin-1-yl)-2-oxo-ethyl]-amide;
2-bromo-4H-thieno[3,2-b]pyrrole-5-carboxylic acid-[(1S)-benzyl-2-((3R,4S)-dihydroxy-pyrrolidin-1-yl)-2-oxo-ethyl]-amide;
2-chloro-6H-thieno[2,3-b]pyrrole-5-carboxylic acid-[(1S)-benzyl-2-morpholin-4-yl-2-oxo-ethyl]-amide;
2-chloro-6H-thieno[2,3-b]pyrrole-5-carboxylic acid-[(1S)-benzyl-2-(1,1dioxo-1-thiazolidin-3-yl)-2-oxo-ethyl]-amide;
2-chloro-4H-furo[3,2-b]pyrrole-5-carboxylic acid-[(1S)-benzyl-2-((3R,4S)-dihydroxy-pyrrolidin-1-yl)-2-oxo-ethyl]-amide;
2-chloro-4H-furo[3,2-b]pyrrole-5-carboxylic acid-[(1S)-benzyl-3-((3R,4S)-dihydroxy-pyrrolidin-1-yl)-(2R)hydroxy-3-oxo-propyl]-amide;
2-chloro-4H-thieno[3,2-b]pyrrole-5-carboxylic acid-[(1S)-benzyl-2-((3R,4S)-dihydroxy-pyrrolidin-1-yl)-2-oxo-ethyl]-amide; and
3-methyl-4H-thieno[3,2-b]pyrrole-5-carboxylic acid-[(1S)-benzyl-2-((3R,4S)-dihydroxy-pyrrolidin-1-yl)-2-oxo-ethyl]-amide; the stereoisomers and prodrugs thereof, and the pharmaceutically acceptable salts of said compounds, stereoisomers, and prodrugs; and
said anti-obesity agent is selected from the group consisting of a β-adrenergic receptor agonist, an apolipoprotein-B secretion/microsomal triglyceride transfer protein inhibitor, an MCR-4 agonist, a cholecystokinin-A agonist, a monoamine reuptake inhibitor, a sympathiomimetic agent, a serotoninergic agent, a dopamine agonist, a melanocyte-stimulating hormone receptor agonist or mimetic, a melanocyte-stimulating hormone analog, a melanin concentrating hormone antagonist, a cannabinoid receptor antagonist, leptin or an analog thereof, a galanin antagonist, a lipase inhibitor, an anorectic agent, a Neuropeptide-Y antagonist, a thyromimetic agent, a dehydroepiandrosterone or an analog thereof, a glucocorticoid receptor agonist or antagonist, an orexin receptor antagonist, a urocortin binding protein antagonist, a glucagon-like peptide-1 receptor agonist, a ciliary neurotrophic factor, and an AGRP antagonist.

19. The use according to claim 18 wherein said anti-obesity agent is selected from the group consisting of phentermine, ephedrine, leptin, phenylpropanolamine, and pseudoephedrine; said (3-adrenergic receptor agonist is selected from the group consisting of {4-[2-(2-[6-aminopyridin-3-yl]-2-(R)-hydroxyethylamino)ethoxy]phenyl}acetic acid, {4-[2-(2-[6-aminopyridin-3-yl]-2-(R)-hydroxyethylamino)ethoxy]phenyl}benzoic acid, {4-[2-(2-[6-aminopyridin-3-yl]-2-(R)-hydroxyethylamino)ethoxy]phenyl}propionic acid, and {4-[2-(2-[6-aminopyridin-3-yl]-2-(R)-hydroxyethylamino)ethoxy]phenoxy}acetic acid; said monoamine reuptake inhibitor is sibutramine; said serotoninergic agent is fenfluramine or dexfenfluramine; said dopamine agonist is bromocriptine; said lipase inhibitor is orlistat; and said anorectic agent is a bombesin agonist.

20. A pharmaceutical composition comprising a glycogen phosphorylase inhibitor and a non-glycogen phosphorylase inhibiting anti-diabetic agent.

21. A pharmaceutical composition comprising a glycogen phosphorylase inhibitor and an anti-obesity agent.

22. A pharmaceutical product containing a glycogen phosphorylase inhibitor and a non-glycogen phosphorylase inhibiting anti-diabetic agent as a combined prepraration for simultaneous, separate or sequential use in treating prophylactically an individual in whom Type 2 diabetes mellitus has not yet presented, but in whom there is an increased risk of developing such condition.

23. A pharmaceutical product containing a glycogen phosphorylase inhibitor and an anti-obesity agent as a combined prepraration for simultaneous, separate or sequential use in treating prophylactically an individual in whom Type 2 diabetes mellitus has not yet presented, but in whom there is an increased risk of developing such condition.
